(19) Europäisches Patentamt / European Patent Office / Office européen des brevets

(11) **EP 4 343 054 A1**

(12) **EUROPEAN PATENT APPLICATION**
published in accordance with Art. 153(4) EPC

(43) Date of publication:
**27.03.2024 Bulletin 2024/13**

(21) Application number: **22804789.0**

(22) Date of filing: **20.05.2022**

(51) International Patent Classification (IPC):
**D06N 3/00** *(2006.01)*

(52) Cooperative Patent Classification (CPC):
**D06N 3/00**

(86) International application number:
**PCT/JP2022/021013**

(87) International publication number:
**WO 2022/244882 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 21.05.2021 JP 2021086464
21.05.2021 JP 2021086467
21.05.2021 JP 2021086501
21.05.2021 JP 2021086495
21.05.2021 JP 2021086520

(71) Applicant: **Asahi Kasei Kabushiki Kaisha
Tokyo 1000006 (JP)**

(72) Inventors:
• **YAMAGUCHI, Hidehiro**
**Tokyo 100-0006 (JP)**
• **MATSUMOTO, Mitsuaki**
**Tokyo 100-0006 (JP)**
• **RIKITAKE, Masato**
**Tokyo 100-0006 (JP)**
• **WATANABE, Yusuke**
**Tokyo 100-0006 (JP)**
• **HIRONAKA, Daisuke**
**Tokyo 100-0006 (JP)**
• **TADOKORO, Yoshiyuki**
**Tokyo 100-0006 (JP)**
• **YAMAMOTO, Aguru**
**Tokyo 100-0006 (JP)**
• **FUJIMOTO, Yoko**
**Tokyo 100-0006 (JP)**
• **ITO, Hidemi**
**Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **ARTIFICIAL LEATHER AND MANUFACTURING METHOD THEREFOR**

(57) Provided is artificial leather that combines good texture, high wear resistance, and a high-quality appearance without the addition of polyurethane resin. Artificial leather that includes at least a surface fiber layer constituting a first surface, the artificial leather being characterized by the following: the surface fiber layer includes main fibers and a thermoplastic resin; the main fibers have a fineness of 0.01 dtex to 0.5 dtex, inclusive; at least a part of the thermoplastic resin adheres between the main fibers; the thermoplastic resin in the surface fiber layer is such that the distance to which a probe is pushed thereinto under prescribed conditions using an atomic force microscope is 20 nm to 200 nm, inclusive; the number average volume of the thermoplastic resin in the surface fiber layer is 3,500 $\mu m^3$ to 24,000 $\mu m^3$, inclusive; the number average volume of the thermoplastic resin in a first surface fiber layer when the first surface is measured by X-ray CT is 5,000 $\mu m^3$ to 14,000 $\mu m^3$, inclusive; the volume number density of the thermoplastic resin in the first surface fiber layer is $1.1 \times 10^{12}/m^3$ to $3.0 \times 10^{12}/m^3$, inclusive; etc.

FIG. 1

EP 4 343 054 A1

**Description**

FIELD

The present invention relates to artificial leather and to a method for producing it.

BACKGROUND

(Aspect 1)

Artificial leather is widely accepted in the market as a substitute material for natural leather and has allowed the development of a variety of products with silver tones or suede tones, while also being dyeable to exhibit various colors not found in natural leather, for which reasons it is widely used as a highly functional design material. Artificial leather having a suede tone with a raised outer surface is suitable for use in the fields of seat skin materials and interior materials for clothing, shoes, bags, furniture, automobiles, railway vehicles, aircraft and ships. Such fields have requirements for a satisfactory appearance quality, a pliable feel, and resistance against physical load, such as abrasion resistance, during long-term use.

In the definition according to JIS-6601, artificial leather is classified as being "smooth", having the silver surface appearance of leather, or "napped" having a leather nubuck, suede or velour outer appearance, and the present artificial leather is classified as being "napped" (that is, "brushed" artificial leather having a brushed-style outer appearance). A brushed appearance can be formed by buffing of the outer surface of the main fiber layer (the front side) using sandpaper or the like. For the purpose of the present specification, the outer surface of artificial leather, the outer surface of the main fiber layer, the outer surface of the fiber sheet and the outer surface of the laminated sheet, each refer to the surface that is externally exposed when the artificial leather is used (the surface that contacts with the human body, in the case of a chair, for example). According to one aspect, for brushed artificial leather, the outer surface of the main fiber layer has raised or standing fibers produced by buffing, for example.

The material used for artificial leather commonly comprises polymer elastomer resins such as polyurethane adhered to a nonwoven fabric structure with entangled ultrafine fibers composed of a material such as polyethylene terephthalate or nylon. If fibers are simply physically entangled by needle punching or hydroentangling without polymer elastomer resins, then problems may occur such as failure to exhibit sufficient abrasion resistance for practical use, lack of the pliable feel of artificial leather, more shedding of yarn during the dyeing step, and problems during production.

In the production steps for artificial leather, it is common to employ methods of adhering polymer elastomers such as polyurethane in order to impart a satisfactory hand quality and abrasion resistance. Various types of artificial leather with impregnated adhesion of polyurethane are available on the market under the names Lamousse™, ECSAINE™ and Alcantara™, for example. However, polymer elastomers such as polyurethane have high bleed out of dyes. Artificial leather with polymer elastomers have poor washing fastness, unless a sufficient amount of reduction cleaning is used. And polymer elastomers such as polyurethane are exposed to ultraviolet rays , resulting in changes in color shade or progressive degradation with prolonged use. In addition, since they cannot be depolymerized under reaction conditions in which polyesters depolymerize, their recycling is difficult when used in composite systems together with polyester fibers, which are the most widely used type of fiber for artificial leather.

In PTL 1, artificial leather is produced by a process in which thermal bonding stable fibers are mixed in a specific proportion with at least the front side fiber layer of a nonwoven fabric having a multilayer structure of two or more layers consisting of a front side fiber layer and a scrim layer (a woven or knitted fabric), and then heat fused, in order to provide artificial leather exhibiting a satisfactory hand quality and high abrasion resistance as well as cuttability and shape stability, without impregnation of a polymer elastomer such as a polyurethane resin. Since the obtained artificial leather contains no polyurethane resin it has increased resistance against artificial sebum, and yet it is still in need of improvement from the standpoint of use and deployment for special purposes such as car seats that must exhibit abrasion resistance of 20,000 times or more in a abrasion resistance test by the Martindale method, and hand quality value of less than 26 cm in flexibility test, as well as both high abrasion resistance and a pliable texture.

PTL 2 provides an improvement over the invention described in PTL 1, with improved abrasion resistance (40,000 times or more in a abrasion resistance test by the Martindale method) and hand quality (a bending value of less than 24 cm in KES pure bending measurement), by exposure of a portion of the thermoplastic resin formed by melting of the thermal bonding stable fibers on the surface of the front side fiber layer in the form of masses of predetermined sizes, but as with PTL 1, there is still room for improvement in terms of use and deployment for specific purposes such as car seats which require both high abrasion resistance and a pliable texture.

The invention of PTL 3 describes providing a novel fiber laminated sheet having a soft surface hand quality and exhibiting suitable firmness, resilience and air permeability, without elastic polymers. And further PTL 3 describes fiber laminated sheet that, with elastic polymers, can serve as artificial leather exhibiting a hand quality approximating natural leather, as well as artificial leather using it, and a low-cost synthetic fiber sheet used in it, wherein the single filaments are easily dispersed and easily entangled by hydroentangling, and therefore the artificial leather described in PTL 3 is limited to artificial leather that includes a polymer elastomer such as water-dispersed polyurethane. PTL 3 also teaches that the synthetic fiber sheet of the invention exhibits the following functions (1) to (3): (1) as a synthetic fiber sheet in which the fibers are weakly bonded together by agglutination, (2) as a synthetic fiber sheet in which agglutination produced by the pressure of mechanical entangling is dispersed to that the fibers become re-dispersed in the thickness direction to form three-dimensional entangling, and (3) as a synthetic fiber sheet in which the binder staple fibers undergo softened shrinkage deformation by heating after entangling, producing bonds between the entanglement points and thereby resulting in interlayer peel strength that is improved compared to the strength during entangling, and that it is therefore a synthetic fiber sheet obtained by wet method (papermaking method), that includes polyester staple fibers as the main component fibers, and also includes binder staple fibers that cause further softening and shrinkage and have a bond-exhibiting effect, due to partial bonding by weak agglutination with the main component fibers at the drying temperature during sheet formation (100 to 120°C), followed by subsequent heat treatment at a temperature of 150 to 180°C higher than the drying temperature during sheet formation.

Since it is further explained that it is the binder staple fibers that cause softening and shrinkage, PTL 3 does not teach that a portion of the thermoplastic resin formed by melting of the thermal bonding stable fibers is exposed on the surface of the front side fiber layer as masses of specified sizes, nor does it mention improvement in the abrasion resistance or hand quality.

(Aspect 2)

Artificial leather is widely accepted in the market as a substitute material for natural leather and has allowed the development of a variety of products with silver tones or suede tones, while also being dyeable to exhibit various colors not found in natural leather, for which reasons it is widely used as a highly functional design material. Artificial leather having a suede tone with a raised outer surface is suitably used in the fields of seat skin materials and interior materials for clothing, shoes, bags, furniture, automobiles, railway vehicles, aircraft and ships. Such fields require a satisfactory appearance quality, a pliable feel, and resistance against physical load, such as abrasion resistance, during long-term use.

In the definition according to JIS-6601, artificial leather is classified as "smooth" having the silver surface appearance of leather, or "napped" having a leather nubuck, suede or velour outer appearance, and the artificial leather of this embodiment is classified as "napped" (that is, "brushed" artificial leather having a brushed-style outer appearance). A brushed appearance can be formed by buffing of the outer surface of the main fiber layer (the front side) using sandpaper or the like. For the purpose of the present specification, the outer surface of artificial leather, the outer surface of the main fiber layer, the outer surface of the fiber sheet and the outer surface of the laminated sheet, each refer to the surface that is externally exposed when the artificial leather is used (the surface that contacts with the human body, in the case of a chair, for example). According to one aspect, for brushed artificial leather, the outer surface of the main fiber layer has raised or standing fibers produced by buffing, for example.

The material used for artificial leather commonly comprises a polymer elastomer resin such as polyurethane adhered to a nonwoven fabric structure with entangled ultrafine fibers composed of a material such as polyethylene terephthalate or nylon. If fibers are simply physically entangled by needle punching or hydroentangling without impregnation with a polymer elastomer resin, then problems will occur such as failure to exhibit sufficient abrasion resistance for practical use, lack of the pliable feel of artificial leather, more shedding of yarn during the dyeing step, and problems during production.

In the production steps for artificial leather, it is common to employ methods of adhering polymer elastomers such as polyurethane in order to impart a satisfactory hand quality and abrasion resistance. For example, types of artificial leather with impregnated adhesion of polyurethane are marketed, under the names Lamousse™, Exeine™ and Alcantara™. However, polymer elastomers such as polyurethane have high bleed out of dyes and poor washing fastness unless a sufficient amount of reduction cleaning treatment is used, while also being susceptible to ultraviolet rays and tending to undergo deterioration with prolonged use, resulting in changes in color shade or progressive degradation with prolonged use. In addition, since they cannot be decomposed under reaction conditions in which polyesters depolymerize, their recycling properties cannot be exhibited when used in composite systems with polyester fibers, which are the most widely used type of fiber for artificial leather.

PTL 4 discloses the use of polyurethane having a porous structure, as an example of a binder for artificial leather. In this method, satisfactory quality can be obtained at the start of production but the polyurethane degrades with prolonged use, and therefore abrasion resistance is not adequately exhibited. In addition, the material composition is a composite system of polyester fibers and polyurethane binder, which are different types of chemical materials, rather than a single material composition. In order to be applied in recycling steps such as chemical recycling and material recycling, therefore, it becomes necessary to develop removal techniques to separate the polyester and polyurethane, which creates difficulties for recycling.

In addition to impregnating a polymer elastomer such as polyurethane, another means that has been examined in the past for obtaining a binder effect, in order to increase physical strength of artificial leather, has been a method of mixing thermal bonding fibers during production of a nonwoven fabric for artificial leather, and melting them to adhere the main fibers together. For example, in the Examples of PTL 5, fibers with thick fiber diameters and a sheath/core structure are used as thermal bonding fibers. One drawback has been that linking the bonding points of the main fibers by the core fibers produces a hard hand quality for the nonwoven fabric, and inferior quality.

The method of PTL 6 includes a step of heat shrinkage of a nonwoven fabric, and consequently the molten masses of the thermal bonding fibers increase in size, tending to result in inferior surface quality. Moreover, since the melted fibers are produced by ultrafine fiber-generating composite fibers, which are typically sea-island fibers, the molten masses tend to concentrate at the original composite fiber locations. This aspect also tends to result in the same drawbacks as production methods that tend to produce larger molten masses.

PTL 7 proposes a leather-like material and a method for its production, wherein a polyester ultrafine fiber layer, and fusible fibers composed of a copolymerized polyester having a melting point of at least 10°C lower, are layered and subjected to high-speed fluid treatment followed by molding bonding treatment. The leather-like material produced by this production method is layered with the thermal bonding fiber layer and polyester fiber layer as separate layers, and because this method not only employs thermal bonding but also uses ultrafine fibers formed from sea-island fibers, pilling of the ultrafine fibers are tended to produce during abrasion when the abrasiveness is evaluated, due to voids inside the fiber bundles produced from the sea portions of the sea-island fibers, making it impossible to exhibit sufficient abrasion resistance and increasing deterioration of the outer appearance during use. Therefore it has not been able to exhibit abrasion resistance to withstand practical use as artificial leather while maintaining a luxurious outer appearance.

In PTL 1, artificial leather is produced by a process in which thermal bonding stable fibers are mixed in a specific proportion with at least the front side fiber layer of a nonwoven fabric having a multilayer structure of two or more layers consisting of a front side fiber layer and a scrim layer (a woven or knitted fabric), and then melted thermal bonding fibers in order to provide artificial leather exhibiting a satisfactory hand quality and high abrasion resistance as well as cuttability and shape stability, without i polymer elastomers such as polyurethane resin. Since the obtained artificial leather contains no polyurethane resin it has increased resistance against artificial sebum, and yet it is still in need of improvement from the standpoint of use and deployment for special purposes such as car seats that must exhibit abrasion resistance of 20,000 times or more in a abrasion resistance test by the Martindale method and a hand quality value of less than 26 cm in a flexibility test, as well as both a high abrasion resistance property and a pliable texture. Since in the production process entangling is followed by heat treatment at 200°C using a pin tenter dryer, simultaneously with melting the thermal bonding stable fibers, despite satisfactory shape stability it is still in need of improvement in terms of both abrasion resistance and texture.

PTL 2 provides an improvement over the invention described in PTL 5, with improved abrasion resistance (40,000 times or more in a abrasion resistance test by the Martindale method) and hand quality (a bending value of less than 24 cm in KES pure bending measurement), by exposure of a portion of the thermoplastic resin formed by melting of the thermal bonding stable fibers on the surface of the front side fiber layer as masses of predetermined sizes, but as with PTL 5, entangling by spraying of a high-speed water stream is followed by the use of a pin tenter dryer for heat treatment at 190°C, simultaneously with melting the thermal bonding stable fibers, and consequently there is still room for improvement in terms of both abrasion resistance and texture.

The invention of PTL 3 describes providing a novel fiber laminated sheet having a soft surface hand quality and exhibiting suitable firmness, resilience and air permeability, without elastic polymers, and further describes providing a fiber laminated sheet that, when impregnated with an elastic polymer, can serve as artificial leather exhibiting a hand quality approximating natural leather, as well as artificial leather using it, and a low-cost synthetic fiber sheet used in it, wherein the single filaments are easily dispersed and easily entangled by hydroentangling, while the artificial leather described in PTL 7 is limited to artificial leather that includes a polymer elastomer such as water-dispersed polyurethane. PTL 7 also teaches that the synthetic fiber sheet of the invention exhibits the following functions (1) to (3): (1) as a synthetic fiber sheet in which the fibers are weakly bonded together by agglutination, (2) as a synthetic fiber sheet in which agglutination produced by the pressure of mechanical entangling is dispersed to that the fibers become re-dispersed in the thickness direction to form three-dimensional entangling, and (3) as a synthetic fiber sheet in which the binder staple fibers undergo softened shrinkage deformation by heat treatment after entangling, producing bonds between the entanglement points and thereby resulting in interlayer peel strength that is improved compared to the strength during entangling, and that it is therefore a synthetic fiber sheet obtained by wet method (papermaking method), that includes polyester staple fibers as the main component fibers, and also includes binder staple fibers that cause further softening and shrinkage and have a bond-exhibiting effect, due to partial bonding by weak agglutination with the main component fibers at the drying temperature during sheet formation (100 to 120°C), followed by subsequent heat treatment at a temperature of 150 to 180°C higher than the drying temperature during sheet formation.

Since it is further explained that it is the binder staple fibers that cause softening and shrinkage, PTL 3 does not teach that a portion of the thermoplastic resin formed by melting of the thermal bonding stable fibers is exposed on the surface of the front side fiber layer as masses of specified sizes, as described in PTL 2, nor does it mention improvement in the abrasion resistance or hand quality.

[CITATION LIST]

[PATENT LITERATURE]

[PTL 1] Japanese Patent Publication No. 5685003
[PTL 2] Japanese Patent Publication No. 6118174
[PTL 3] Japanese Patent Publication No. 470494
[PTL 4] Japanese Patent Publication No. 5919627
[PTL 5] Japanese Unexamined Patent Publication HEI No. 07-216756
[PTL 6] Japanese Examined Patent Publication HEI No. 03-016427
[PTL 7] Japanese Patent Publication No. 4835181

SUMMARY

[TECHNICAL PROBLEM]

(Aspect 1)

In light of the current level of the prior art, one problem to be solved by the invention is to provide artificial leather exhibiting both a satisfactory hand quality and high abrasion resistance without polyurethane resins, as well as a method for its production.

(Aspect 2)

In light of the current level of the prior art, another problem to be solved by the invention is to provide recyclable artificial leather exhibiting a satisfactory hand quality, high abrasion resistance and a luxurious outer appearance, as well as a method for its production.

[SOLUTION TO PROBLEM]

As a result of diligent experimentation with the aim of solving this problem, the present inventors have completed this invention upon finding, unexpectedly, that the problem can be solved by artificial leather having the following features.
Specifically, the present invention provides the following.

(Aspect 1)

[1] Artificial leather that includes at least a front side fiber layer constituting a first surface, and having the following features:

(1) the front side fiber layer includes main fibers and thermoplastic resins;
(2) the main fibers have a fineness of 0.01 dtex to 0.5 dtex;
(3) at least part of the thermoplastic resin adheres together the main fibers;
(4) the thermoplastic resin in the front side fiber layer is such that the distance to which a probe is pushed, during maximum load from the contact starting point when the probe is pushed with a weight of 200 nN using a cantilever in measurement with an atomic force microscope, is 20 nm to 200 nm, and
(5) the number average volume of the thermoplastic resin in the front side fiber layer is 3500 $\mu m^3$ to 24,000 $\mu m^3$.

[2] The artificial leather according to [1] above, wherein
the thermoplastic resin satisfies the following (i), (ii) and (iii) in measurement by Time-Of-Flight Secondary Ion Mass Spectrometry(TOF-SIMS):

(i) the ion intensity ratio (71/27) of the positive ion for m/z = 71 (fragment ion: $C_4H_7O$) with respect to m/z = 27 (fragment ion: $C_2H_3$) is 0.2 to 2.3;

(ii) the ion intensity ratio (42/25) of the negative ion for m/z = 42 (fragment ion: CNO) with respect to m/z = 25 (fragment ion: $C_2H$) is 0 to 0.5; and

(iii) the ion intensity ratio (104/27) of the positive ion for m/z = 104 (fragment ion: $C_7H_4O$) with respect to m/z = 27 (fragment ion: $C_2H_3$) is 0.2 to 1.0.

[3] The artificial leather according to [1] or [2] above, wherein the thermoplastic resin is a copolymer of a polyester and an aliphatic polyether.

[4] The artificial leather according to [3] above, wherein the thermoplastic resin is a copolymer of polybutylene phthalate and an aliphatic polyether.

[5] The artificial leather according to [4] above, wherein the thermoplastic resin is a copolymer of polybutylene phthalate and polytetramethylene ether glycol.

[6] The artificial leather according to any one of [1] to [5] above, wherein the main fibers are polyester fibers.

[7] The artificial leather according to any one of [1] to [6] above, wherein the volume number density of the thermoplastic resin per unit volume in the front side fiber layer is $0.5 \times 10^{12}/m^3$ to $5.0 \times 10^{12}/m^3$.

[8] The artificial leather according to any one of [1] to [7] above, wherein the average quotient of the long axis divided by the short axis is 100 or lower, where the thermoplastic resin in the front side fiber layer is approximated as elliptical.

[9] The artificial leather according to any one of [1] to [8] above, wherein the front side fiber layer is entangled with a woven scrim layer.

[10] The artificial leather according to [9] above, wherein the scrim layer is composed of polyester resin fibers.

[11] The artificial leather according to any one of [1] to [11] above, wherein the scrim layer is not exposed after less than 50,000 abrasion cycles, with abrasion of the surface under a pressing load of 12 kPa according to JIS-L-1096 E (Martindale method).

[12] The artificial leather according to any one of [1] to [11] above, wherein the abrasion loss is 21 mg or less after 50,000 abrasion cycles, with abrasion of the surface under a pressing load of 12 kPa according to JIS-L-1096 E (Martindale method).

[13] A method for producing artificial leather according to any one of [1] to [12] above, wherein the method includes the following steps:

(1) a step in which the main fibers are mixed with thermal bonding fibers produced using the thermoplastic resin such that the distance to which a probe is pushed, during maximum load from the contact starting point when the probe is pushed with a weight of 200 nN using a cantilever in measurement with an atomic force microscope, is 20 nm to 200 nm, so that the weight ratio of the thermal bonding fibers is 3% to 25% with respect to the front side fiber layer, and the fibers are entangled by wet method (papermaking method), after which a front side fiber web is formed by hydroenentangling or needle punching, and

(2) a step in which the entangled structure of the obtained front side fiber web is subjected to thermal annealing at a temperature at or above the melting point of the thermal bonding fibers and below the melting point of the main fibers, to form a front side fiber layer;

and wherein in step (2), the front side fiber web has a shrinkage factor in the MD of 0.5 to 5% and a shrinkage factor in the CD of 1 to 8%.

[14] The method according to [13] above, wherein the fiber lengths of the thermal bonding fibers composed of the thermoplastic resin are 2 mm to 90 mm.

[15] The method according to [13] or [14] above, wherein:

the single fiber fineness of the thermal bonding fibers produced using the thermoplastic resin are 0.5 dtex to 2.2 dtex, and

the single fiber fineness of the main fibers are 0.01 dtex to 0.5 dtex.

[16] The method according to any one of [13] to [15] above, wherein the thermal bonding fibers composed of the thermoplastic resin are produced by melt spinning.

[17] The method according to any one of [13] to [16] above, wherein at least 95 wt% of the resin in the thermal bonding fibers is a copolymer of a polyester and an aliphatic polyether.

[18] The method according to any one of [13] to [17] above, wherein the melting point of the thermoplastic resin is 130°C or higher, and at least 20°C lower than the melting point of the main fibers.

(Aspect 2)

[19] Artificial leather that includes at least a front side fiber layer constituting a first surface, and having the following features:

(1) the front side fiber layer includes at least one type of main fiber and thermoplastic resins having a melting point of 20°C to 170°C lower than the melting point of the main fibers;
(2) the main fibers have a fineness of 0.01 dtex to 0.5 dtex;
(3) at least part of the thermoplastic resin adheres together the main fibers;
(4) the number average volume of the thermoplastic resin in the first surface fiber layer is 5000 $\mu m^3$ to 14,000 $\mu m^3$ as measured by X-ray CT; and
(5) the volume number density of the thermoplastic resin in the first surface fiber layer is $1.1 \times 10^{12}/m^3$ to $3.0 \times 10^{12}/m^3$.

[20] The artificial leather according to [19] above, which has the following features:

(6) at least part of the thermoplastic resin is exposed on the surface of the front side fiber layer in the form of resin masses, at a projected area mean value of $0.66 \times 10^{-9}$ m$^2$ to $5.0 \times 10^{-9}$ m$^2$ per resin mass;
(7) the front side fiber layer is integrally entangled with the woven scrim layer; and
(8) the artificial leather satisfies the following inequality:

$$220,000 \leq (A) \times (B) \times (C) \leq 600,000$$

where (A) is the sum of the elongation under 500 gf/cm constant load in the MD (%) and the elongation under 500 gf/cm constant load in the CD (%), (B) is the sum of the scrim layer woven density in the MD (number/2.54 cm) and the woven density in the CD (number/2.54 cm), and (C) is the fineness (denier) of the weaving yarn forming the scrim layer,
and which has an elongation of 3% or greater under 500 gf/cm constant load in the MD.

[21] The artificial leather according to [19] or [20] above, wherein the main fibers are polyester fibers.
[22] The artificial leather according to any one of [19] to [21] above, wherein the thermoplastic resin is a polyester resin.
[23] The artificial leather according to any one of [19] to [22] above, wherein the scrim layer is composed of polyester resin fibers.
[24] The artificial leather according to any one of [19] to [23] above, wherein the scrim layer is not exposed after less than 50,000 abrasion cycles, with abrasion of the surface under a pressing load of 12 kPa according to JIS-L-1096 E (Martindale method).
[25] The artificial leather according to any one of [19] to [24] above, wherein the abrasion loss is 21 mg or less after 50,000 abrasion cycles, with abrasion of the surface under a pressing load of 12 kPa according to JIS-L-1096 E (Martindale method).
[26] The artificial leather according to any one of [19] to [25] above, wherein the flexural rigidity per unit width in KES pure bending measurement is 1.0 gfcm$^2$/cm or lower.
[27] A method for producing artificial leather according to any one of [19] to [26] above, wherein the method has the following steps:

(1) a step in which the main fibers are mixed with thermal bonding fibers composed of the thermoplastic resin having a melting point of 20°C to 170°C lower than the melting point of the main fibers, so that the weight ratio of the thermal bonding fibers is 3% to 25% with respect to the front side fiber layer, and then the fibers are entangled by wet method (papermaking method) on a scrim layer, after which hydroenentangling or needle punching is carried out to form a front side fiber web entangled with the scrim layer, and
(2) a step in which the entangled structure of the obtained front side fiber web is subjected to thermal annealing shrinkage at a temperature at or above the melting point of the thermal bonding fibers and below the melting point of the main fibers, to form a front side fiber layer;

and wherein in step (2), the front side fiber web has a shrinkage factor in the MD of 0.5 to 5% and a shrinkage factor in the CD of 1 to 8%.
[28] The method according to [27] above, wherein the lengths of the main fibers in step (1) are 2.5 mm to 90 mm.

[29] The method according to [27] or [28] above, wherein the fineness of the thermal bonding fibers in step (1) is 0.5 dtex to 2.2 dtex and the fineness of the main fibers is 0.01 dtex to 0.5 dtex.

[ADVANTAGEOUS EFFECTS OF INVENTION]

(Aspect 1)

The artificial leather of the invention improves the flexibility of thermoplastic resins adhering between main fibers with a fineness of 0.01 dtex to 0.5 dtex, to provide excellent hand quality, while also limiting the sizes of the heat bonding points to within a predetermined range so as to provide both texture and abrasion resistance while maintaining mechanical strength.

(Aspect 2)

The artificial leather of the invention reduces the number of fibers to which one thermoplastic resin (bonding point) adheres, thereby providing excellent hand quality. Since the density of the bonding points per unit volume can be maintained at or above a constant value, it is possible to maintain entanglement between fibers and to simultaneously exhibit abrasion resistance. Moreover, since the artificial leather of the invention uses polyester fibers as the main fibers and the thermoplastic resin, which do not include an elastic polymer such as water-dispersed polyurethane, it exhibits excellent recycling properties and excellent flame resistance to help reduce gas generated during combustion.

BRIEF DESCRIPTION OF DRAWINGS

(Aspect 1)

Fig. 1 is a diagram showing an example of the state of a resin mass in a front side fiber layer.
Fig. 2 is a diagram showing another example of the state of a resin mass in a front side fiber layer.
Fig. 3 is a diagram showing another example of the state of a resin mass in a front side fiber layer.
Fig. 4 is a diagram showing another example of the state of a resin mass in a front side fiber layer.
Fig. 5 is a diagram showing another example of the state of a resin mass in a front side fiber layer.
Fig. 6 is a diagram showing another example of the state of a resin mass in a front side fiber layer.
Fig. 7 is a diagram showing another example of the state of a resin mass in a front side fiber layer.
Fig. 8 is a diagram showing another example of the state of a resin mass in a front side fiber layer.
Fig. 9 is a schematic diagram showing a force curve for an atomic force microscope.

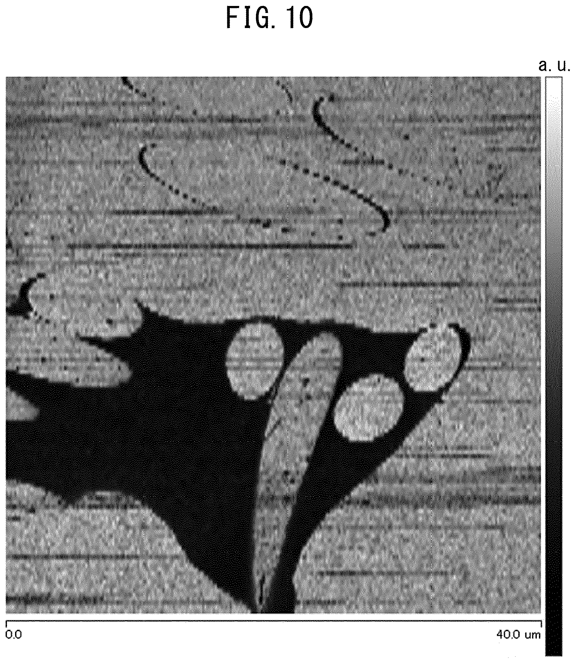

Fig. 10 is a diagram showing an example of an image obtained by cutting artificial leather according to one embodiment and measuring it with an atomic force microscope.

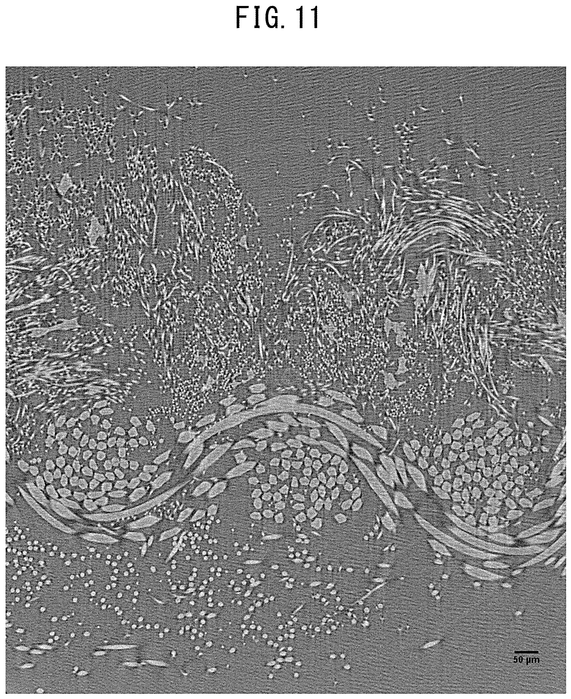

Fig. 11 is a diagram showing an example of a cross-sectional image obtained by cutting artificial leather according to one embodiment and evaluating it by X-ray CT.

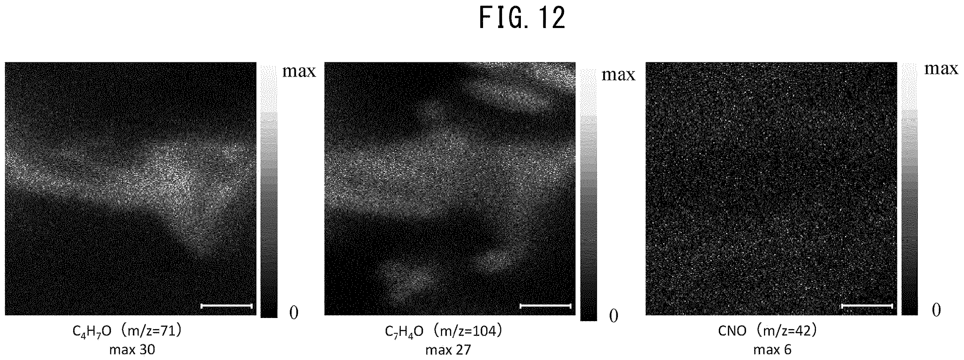

Fig. 12 is a set of photographs in lieu of a diagram, showing TOF-SIMS analysis results obtained by cutting artificial leather according to one embodiment.

(Aspect 2)

Fig. 13 is a conceptual drawing of a thermal annealing shrinkage method.

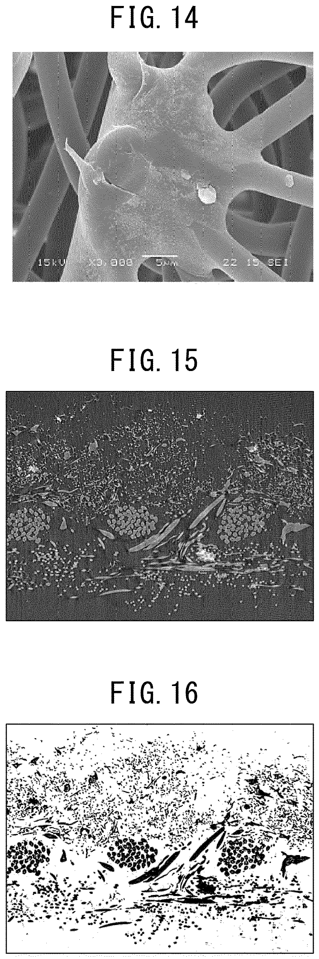

Fig. 14 is a photograph in lieu of a drawing, showing the state in which thermoplastic resins is adhering between main fibers, with the thermoplastic resin present on the surface of the front side fiber layer in the form of resin masses.
Fig. 15 is a cross-sectional view obtained by cutting artificial leather actually fabricated by the method described in Example 1 and evaluating the cross-section by X-ray CT, representing the image data after noise removal with a 3D median filter: 2 pix.
Fig. 16 shows image data obtained by binarizing the cross-section obtained in Fig. 15 under the described conditions and performing black-white inversion.

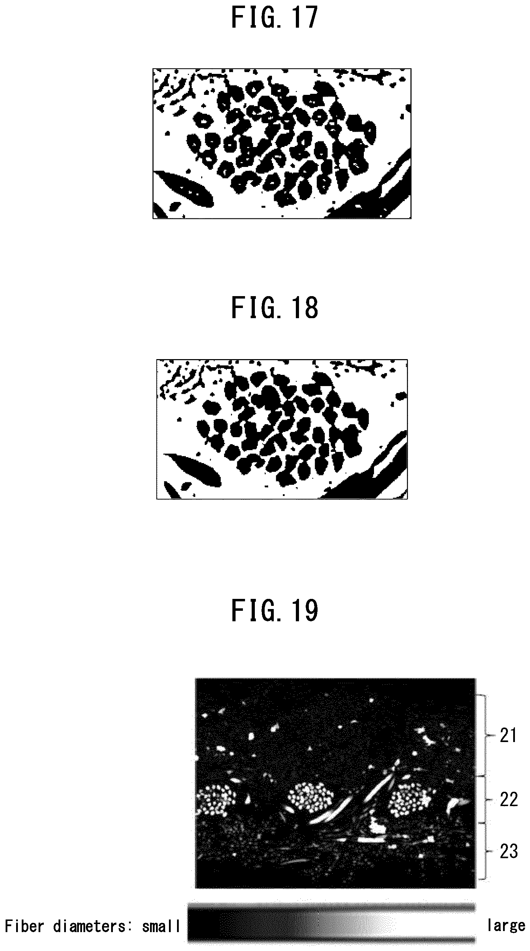

Fig. 17 shows image data as a partial enlarged view of Fig. 16 (scrim section).
Fig. 18 shows image data obtained by fill holes treatment of the image data of Fig. 17.
Fig. 19 shows image data obtained by color mapping the image data of Fig. 18 by fiber diameter distribution. In the image, 21 represents the front layer, 22 represents the scrim layer and 23 represents the back layer. The bright points seen in the front layer and back layer are the thermal bonding resin.

Fig. 20 shows the fiber sheet 11 including a woven or knitted fabric scrim 12, a front side fiber layer (A) 13 forming the front side and a fiber layer (B) 14 forming the back side. However, the fiber layer (B) 14 is optional and is not an essential element.

Fig. 21 shows the fiber sheet 11 including a woven or knitted fabric scrim 12 and a front side fiber layer (A) 13.

Fig. 22 is a diagram showing an example of a roof panel test piece for a vehicle.

DESCRIPTION OF EMBODIMENTS

(Aspect 1)

The invention will now be explained in detail using an embodiment.

One embodiment of the invention is artificial leather that includes at least a front side fiber layer constituting a first surface, and having the following features:

(1) the front side fiber layer includes main fibers and thermoplastic resins;
(2) the main fibers have a fineness of 0.01 dtex to 0.5 dtex;
(3) at least part of the thermoplastic resin adheres between the main fibers;
(4) the thermoplastic resin in the front side fiber layer is such that the distance to which a probe is pushed, during maximum load from the contact starting point when the probe is pushed with a weight of 200 nN using a cantilever in measurement with an atomic force microscope, is 20 nm to 200 nm, and
(5) the number average volume of the thermoplastic resin in the front side fiber layer is 3500 $\mu m^3$ to 24,000 $\mu m^3$.

The main fibers in the front side fiber layer are fibers included at 60 mass% or greater with respect to 100 mass% of the front side fiber layer. The percentage is more preferably 70 mass% or greater and even more preferably 80 mass% or greater. The upper limit is not particularly restricted but may be 99 mass% or lower.

From the viewpoint of strength, easier production of ultrafine fibers and general-purpose market distribution, the main fibers in the front side fiber layer are preferably polyester fibers, polyamide fibers, acrylic fibers or polyolefin fibers, but considering uses that require durability, such as in the field of car seats as mentioned above, polyethylene terephthalate is preferred from the standpoint of obtaining excellent color fastness without yellowing of the fibers themselves even after prolonged exposure to direct sunlight. More preferred, from the viewpoint of reducing environmental impact, are chemical-recycled or material-recycled polyethylene terephthalate, or polyethylene terephthalate obtained using a plant-derived starting material.

The polyester fibers suitable for use as the main fibers composing the front side fiber layer may be polyethylene terephthalate (PET), polytrimethylene terephthalate, polybutylene terephthalate, polyethylene naphthalate, polylactate, or copolymers of these. The polyamide fibers used may also be nylon, meta-aramid, para-aramid, or copolymers of these. Acrylic fibers may be an acrylic acid ester or methacrylic acid ester polymer, or a copolymer of these. Polyolefin fibers may be polyethylene, polypropylene, polybutene, polystyrene, or a copolymer of these. These fibers may be used alone or in mixtures of different polymer fibers in arbitrary proportions.

If a polyester resin is used to form not only the main fibers but also the thermoplastic resin in the front side fiber layer described below, as well as the scrim, then it will be possible to recover used PET from clothing or beverage bottles and to process it as recycled PET resin for use in production of artificial leather, and to produce recyclable artificial leather suited for a circular economy in which used artificial leather (with expired lifetime) is subjected to recycling treatment and used to form a heat-insulating material or filter material, for example, and the PET is further recovered from the used heat-insulating material or filter material.

From the viewpoint of helping to obtain a hand quality similar to natural leather or a suede-like or nubuck-like surface texture, the main fibers preferably have a fineness of 0.5 dtex or lower, more preferably a fineness of 0.35 dtex or lower and even more preferably a fineness of 0.2 dtex or lower. From the viewpoint of production efficiency, production stability and abrasion resistance during production of the fibers, the fineness is also preferably 0.01 dtex or higher and more preferably 0.03 dtex or higher.

The main fibers used may be fibers directly spun by a melt spinning method, fibers obtained by a wet spinning method, or ultrafine fibers obtained by removing the sea component from sea-island fibers comprising copolymerized polyester as the sea component and a regular polyester as the island component.

The main fibers do not necessarily need to consist entirely of fibers composed of a single polymer, and they may be mixed with fibers composed of different types of polymers. Moreover the main fibers do not necessarily need to consist entirely of fibers of a single fineness, and they may be mixed with fibers having different finenesses.

The main fibers may also have infiltrating or adhering additive types, so long as the desired effect is exhibited.

The types of additives may be titanium oxide, antioxidants, light fastness agents, antistatic agents, flame retardants, flexibilizers, fastness improvers, pigments such as carbon black, or dyes.

When a method using staple fibers is selected as the method for producing the main fibers, the staple fiber lengths are preferably 13 mm to 102 mm, more preferably 25 mm to 76 mm and even more preferably 38 mm to 76 mm for a dry method (carding method or airlaid method), and preferably 1 mm to 30 mm, more preferably 2 mm to 25 mm and even more preferably 3 mm to 20 mm for a wet method (sheet formation method). For staple fibers used in a wet method (sheet forming method), for example, the aspect ratio (L/D), as the ratio of the length (L) and diameter (D), is preferably 500 to 2000 and more preferably 700 to 1500. This aspect ratio range is preferred because the dispersibility of the staple fibers in the slurry of the staple fibers dispersed in water will be satisfactory during preparation of the slurry, the fiber layer strength will be satisfactory, and fiber balls known as "pilling" caused by abrasion will be less likely to be outwardly apparent since the fiber lengths are shorter than by a dry method, allowing the single filaments to more easily disperse. The fiber lengths of staple fibers with diameters of 4 $\mu$m, for example, are preferably 2 mm to 8 mm and more preferably 3 mm to 6 mm.

Higher flexibility for the thermoplastic resin adhering between the main fibers will increase the flexibility of the artificial leather and enhance the texture. In order to obtain artificial leather with satisfactory texture, the thermoplastic resin must be such that the distance at which the probe is pressed between the contact start point and the maximum load is between 20 nm and 200 nm, when the probe is pressed into the thermoplastic resin in the surface fiber layer using a cantilever with a load of 200 nN in atomic force microscopy, If the distance to which a probe is pushed during maximum load from the contact starting point is 20 nm or greater, the flexibility of the thermoplastic resin will increase and the texture of the artificial leather will be enhanced. The lower limit is preferably 30 nm or greater and more preferably 40 nm or greater in order to obtain artificial leather with a more excellent texture. If the distance to which a probe is pushed during maximum load from the contact starting point is greater than 200 nm, on the other hand, the flexibility of the thermoplastic resin will be too high, mechanical strength will not be obtained and the resin will lack spinnability, and therefore from the viewpoint of spinnability it is preferably 170 nm or lower and more preferably 150 nm or lower.

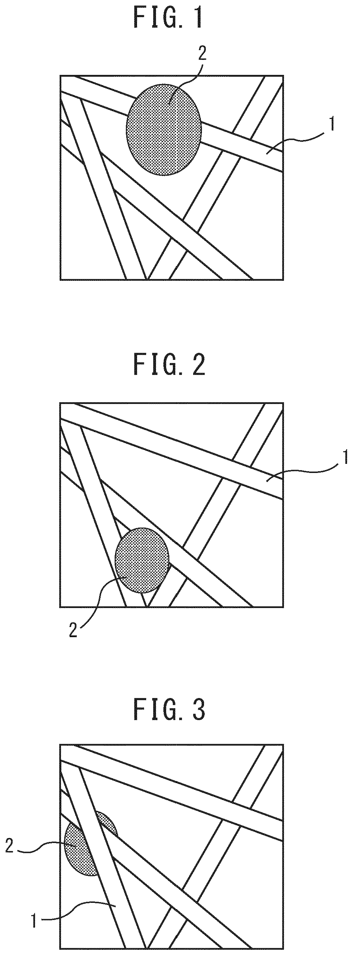

The thermoplastic resin binding between the main fibers provides a function of maintaining a three-dimensional entangled state, with at least a portion thereof adhering between the main fibers. Since the thermoplastic resin adheres between the main fibers, the main fibers are held together and shedding or loss of the fibers is reduced, thereby allowing satisfactory abrasion resistance and strength to be obtained for the artificial leather. The adhesion between the main fibers is in the state illustrated in Fig. 2, Fig. 3 and Fig. 4. The thermoplastic resin forming the front side fiber layer is preferably a polyester resin, polyamide resin, acrylic resin or polyolefin resin from the viewpoint of availability. Suitable polyester resins for use include polyethylene terephthalate, polytrimethylene terephthalate, polybutylene terephthalate, polyethylene naphthalate and polylactate, as well as their copolymers. Suitable polyamide resins for use include nylon, and its copolymers. Suitable acrylic resins for use include acrylic acid ester and methacrylic acid ester polymers, as well as their copolymers. Suitable polyolefin resins for use include polyethylene, polypropylene, polybutene and polystyrene, as well as their copolymers.

Preferred among these are copolymers of polyesters and aliphatic polyethers, from the viewpoint of excellent chemical resistance against acids, alkalis and artificial sebum during actual use of the artificial leather. Suitable polyesters are polybutylene phthalate-based resins, with polybutylene phthalate and aliphatic polyether copolymers being preferred, polybutylene phthalate and polytetramethylene ether glycol copolymers being more preferred, and polybutylene phthalate and polytetramethylene ether glycol blocked copolymers being especially preferred.

The term "phthalate" generally includes benzenedicarboxylic acid compounds. Specifically, isomers such as 1,2-benzenedicarboxylic acid, 1,3-benzenedicarboxylic acid and 1,4-benzenedicarboxylic acid may be used in any desired proportions. The form in which the desired proportion is used is not particularly restricted, and it may be a simple isomer alone or a mixture of homopolymers in a desired proportion, or the isomers may be used in a copolymer.

When a copolymer is used, the block structure or randomness of the comonomers is not particularly restricted, but most preferably soft segments and hard segments are used together in proper amounts, and a blocked copolymer is preferred for convenience of production.

The CASVEN 8000, MELTY 4080 and CASVEN 8080 used in the Comparative Examples do not contain aliphatic polyethers and are not polyester and aliphatic polyether copolymers. PEBAX 6333SP01 is a nylon-based elastomer.

Suitable acrylic resins for use include acrylic acid ester and methacrylic acid ester polymers, as well as their copolymers. Suitable polyolefin resins for use include polyethylene, polypropylene, polybutene and polystyrene, as well as their copolymers.

The thermoplastic resin in the artificial leather can be examined by compositional analysis of the thermoplastic resin using a TOF-SIMS with sputter cleaning. TOF-SIMS allows analysis of the elemental composition and chemical structure of the extreme surface of a sample. When a primary ion beam is irradiated onto a sample in an ultra high vacuum, secondary ions are emitted from the extreme surface of the sample (1 to 3 nm). The secondary ions are introduced into a time-of-flight (TOF) mass spectrometer, to obtain a mass spectrum for the extreme surface of the sample. By limiting the primary ion exposure dose during this time, the surface components can be detected as molecular ions and partially cleaved fragments retaining their chemical structure, to obtain information regarding the elemental composition and chemical structure of the extreme surface in the planar direction. By evaluating the fragment intensity for the positive ion with respect to m/z = 27 (fragment ion: $C_2H_3$) detected from a common organic materials, it is possible to make a relative comparison of the intensity of the positive ion fragment. For the negative ion, the intensity of the negative ion fragment can be compared relatively to m/z = 25 (fragment ion: $C_2H$) detected from common organic materials.

For the desired effect of the invention to be adequately exhibited, the fragment intensity ratio of the thermoplastic resin measured by TOF-SIMS, as the ion intensity ratio (71/27) of the positive ion for m/z = 71 (fragment ion: $C_4H_7O$) from a polyol, and especially tetramethylene glycol, with respect to m/z = 27 (fragment ion: $C_2H_3$), is preferably 0.2 to 2.3. With this ion intensity ratio, the content of the polyol, or tetramethylene glycol in particular, will be suitable, and the thermoplastic resin will have excellent flexibility and dynamic strength as a binder for artificial leather, and will be able to yield artificial leather with high quality and abrasion resistance. From this viewpoint, the fragment intensity ratio of the thermoplastic resin measured by TOF-SIMS, as the ion intensity ratio (71/27) of the positive ion for m/z = 71 (fragment ion: $C_4H_7O$) from a polyol, and especially tetramethylene glycol, with respect to m/z = 27 (fragment ion: $C_2H_3$), is preferably 0.8 to 2.4 and more preferably 1.2 to 2.2.

The fragment intensity ratio of the thermoplastic resin measured by TOF-SIMS, as the ion intensity ratio (104/27) of the positive ion for m/z = 104 (fragment ion: $C_7H_4O$) from an aromatic compound, with respect to m/z = 27 (fragment ion: $C_2H_3$), is preferably 0.2 to 1.0. If the ion intensity ratio is 0.2 or greater the thermoplastic resin will include adequate hard segments, resulting in dynamic strength as a binder for artificial leather, and excellent abrasion resistance. If the ion intensity ratio is 1.0 or lower, on the other hand, the amount of hard segments will not be excessive, resulting in excellent flexibility as a binder for artificial leather, and excellent surface quality. From this viewpoint, the fragment intensity ratio of the thermoplastic resin measured by TOF-SIMS, as the ion intensity ratio (104/27) of the positive ion for m/z = 104 (fragment ion: $C_7H_4O$) from an aromatic compound, with respect to m/z = 27 (fragment ion: $C_2H_3$), is preferably 0.4 to 0.9 and more preferably 0.5 to 0.8.

The fragment intensity ratio of the thermoplastic resin measured by TOF-SIMS, as the ion intensity ratio (42/25) of the negative ion for m/z = 42 (fragment ion: CNO) from an isocyanate, with respect to m/z = 25 (fragment ion: $C_2H$), is preferably 0 to 0.5. Within this range there will be substantially no urethane present, resulting in excellent recycling properties and flame retardance for artificial leather.

The thermoplastic resin, as well, does not necessarily need to consist entirely of a single polymer, and several different polymers may be used in admixture.

The thermoplastic resin may also include different types of infiltrating or adhering additives, so long as the desired effect is exhibited. The types of additives may be titanium oxide, antioxidants, light fastness agents, antistatic agents, flame retardants, flexibilizers, fastness improvers, pigments such as carbon black, or dyes.

The thermoplastic resin has a melting point of 130°C or higher, and preferably a temperature of at least 20°C lower than the melting point of the main fibers. From the viewpoint of shedding of the thermoplastic resin from the artificial leather during dyeing, the melting point of the thermoplastic resin is preferably 130°C or higher, more preferably 140°C or higher and even more preferably 150°C or higher. When the main fibers are to be adhered by thermoplastic resins, the melting point of the thermoplastic resin must be lower than the main fibers. When the thermal bonding is with thermoplastic resins comprising different types of polymers mixed together, the thermoplastic resin with the highest melting point preferably has a melting point of at least 20°C lower than the melting point of the main fibers with the lowest melting point. A melting point within this range allows the abrasion resistance and appearance quality described herein to be achieved, but it is preferably a melting point of no more than 30°C lower and more preferably no more than 40°C lower, from the viewpoint of satisfactorily maintaining appearance quality.

The composition of the thermoplastic resin is not particularly restricted so long as the distance to which a probe is pushed, during maximum load from the contact starting point when the probe is pushed with a weight of 200 nN using a cantilever, is 20 nm to 200 nm. From the viewpoint of providing both texture and high abrasion resistance for artificial leather, an elastomer composed of a copolymer of a polyester and an aliphatic polyether, or a nylon-based elastomer, may be used. From the viewpoint of abrasion resistance, it is preferred to use a polyester-based resin, or a copolymer of polybutylene phthalate and an aliphatic polyether, or a copolymer of polybutylene phthalate and polytetramethylene ether glycol, and especially a blocked copolymer of polybutylene phthalate and polytetramethylene ether glycol. The form of the copolymer is not particularly restricted, but most preferably soft segments and hard segments are used together in proper amounts, and a blocked copolymer is preferred for convenience of production. The abundance ratio of the soft segment can be calculated by the following formula.

$$\text{Abundance ratio (\%) of soft segment} = \text{Number of monomer units of soft segment} \times 100 \ /$$

$$\text{(total number of monomer units of soft segment and hard segment)}$$

The abundance ratio (%) of the soft segment can be calculated using 1H-NMR, and it is preferably from 40% to 85%, and more preferably 50% to 75%, as the proportion of the soft segment.

For a copolymer of polybutylene phthalate and polytetramethylene ether glycol, the polybutylene phthalate is the hard segment and the polytetramethylene ether glycol is the soft segment.

The thermoplastic resin is preferably exposed on the surface of the front side fiber layer in the form of masses. When exposed on the surface, the main fibers on the surface are adequately held by fusion of the resin making it unlikely for the fibers to be shed or lost, and thus improving the abrasion resistance of the artificial leather. Some or all of the thermoplastic resin preferably adheres between the main fibers. When the thermoplastic resin adheres between the main fibers, it becomes possible to hold the main fibers together, making it unlikely for the fibers to be shed or lost and providing satisfactory abrasion resistance and strength for the artificial leather.

The thermoplastic resin is preferably also exposed in the form of masses on the cross-sections of the front side fiber layer. When it is present only on the front side, and also when present in the form of masses on the cross-section, the main fibers will be adequately held by fusion of the resin, improving the abrasion resistance of the artificial leather and increasing the density of the front side fiber layer, and also helping to provide a luxurious, moist texture characteristic of artificial leather.

For this embodiment, it is necessary for the thermoplastic resin to adhere between the main fibers and for the number average volume of the thermoplastic resin to be 3500 $\mu$m$^3$ to 24,000 $\mu$m$^3$. If the number average volume of the thermoplastic resin exceeds 24,000 $\mu$m$^3$, the resin masses present on the surfaces will be too large with respect to the main fibers, resulting in impaired appearance quality and tactile hand quality and also lower density of bonding points between main fibers, which will fail to sufficiently hold together the main fibers and will create inferior abrasion resistance on the surface layer. If the volume is less than an average of 3500 $\mu$m$^3$, on the other hand, the sizes will be insufficient for adhering between the main fibers, making it impossible to exhibit abrasion resistance. The number average volume is preferably 4000 $\mu$m$^3$ to 22,000 $\mu$m$^3$ and more preferably 6000 $\mu$m$^3$ to 20,000 $\mu$m$^3$.

The volume number density per unit volume in the front side fiber layer is preferably $0.5 \times 10^{12}/\text{m}^3$ to $5.0 \times 10^{12}/\text{m}^3$. If the number per unit volume of the thermoplastic resin is $0.5 \times 10^{12}/\text{m}^3$ or greater, multiple main fibers will be able to be bonded by multiple resin thermal bonding points, thus reducing shedding of fiber bundles when abraded and helping to exhibit abrasion resistance. If the number per unit volume of the thermoplastic resin is $5.0 \times 10^{12}/\text{m}^3$ or less, the front side fiber layer will not become too hard and it will be possible to obtain a pliable luxurious quality as required for artificial leather. The volume number density per unit volume of the front side fiber layer is preferably $0.6 \times 10^{12}/\text{m}^3$ to $3.0 \times 10^{12}/\text{m}^3$ and more preferably $0.8 \times 10^{12}/\text{m}^3$ to $1.5 \times 10^{12}/\text{m}^3$.

The thermoplastic resin preferably adheres the main fibers in an isotropic manner from any angle in three dimensions. Specifically, the average value for the quotient of the long axis divided by the short axis, where the thermoplastic resin in the front side fiber layer is approximated as elliptical, is preferably 100 or smaller. If the average quotient of the long axis divided by the short axis when the thermoplastic resin is approximated as elliptical is 100 or less, then the thermal bonding resin will adequately melt when caused to adhere the main fibers together, resulting in higher bonding strength and high abrasion resistance. The average quotient of the long axis divided by the short axis when the thermoplastic resin is approximated as elliptical is more preferably 30 or smaller and even more preferably 10 or smaller. Since it is preferred for the thermoplastic resin to be nearly spherical (quotient of 1), the average value of the quotient of the long axis divided by the short axis when the thermoplastic resin is approximated as elliptical may be 1 or greater, although this is not limitative.

The flexural modulus of the thermoplastic resin is preferably 40 MPa to 200 MPa. By limiting the flexural modulus to this range it is possible to control the sizes and distribution of the bonding points of the thermal bonding resin during thermal annealing, allowing both satisfactory abrasion resistance and texture to be obtained. By limiting the flexural modulus to the range of 40 MPa to 200 MPa based on this action mechanism, it is possible to produce sufficient adhesion between the main fibers while imparting a suitable flowing feel to the raised fibers when touched by the finger, to exhibit a luxurious tactile sensation as required for artificial leather. The range for the flexural modulus is preferably 41 MPa to 150 MPa and more preferably 45 MPa to 120 MPa from the viewpoint of more satisfactorily ensuring the appearance quality.

Another embodiment of the invention is a method for producing artificial leather which includes the following steps:

> (1) a step in which the main fibers are mixed with thermal bonding fibers produced using the thermoplastic resin such that the distance to which a probe is pushed, during maximum load from the contact starting point when the probe is pushed with a weight of 200 nN using a cantilever in measurement with an atomic force microscope, is 20 nm to 200 nm, so that the weight ratio of the thermal bonding fibers is 3% to 25% with respect to the front side fiber layer, and the fibers are entangled by wet method (papermaking method), after which a front side fiber web is formed by hydroentangling or needle punching, and

(2) a step in which the entangled structure of the obtained front side fiber web is subjected to thermal annealing at a temperature at or above the melting point of the thermal bonding fibers and below the melting point of the main fibers, to form a front side fiber layer.

The artificial leather of this embodiment may include a scrim layer. The scrim layer when used functions as a core material, and it is preferably a woven or knitted fabric from the viewpoint of stabilizing fabrication of a sheet in the sheet-forming process, or helping to increase mechanical strength of the obtained artificial leather. From the viewpoint of color uniformity in dyeing, the material of the scrim layer is preferably the same polymer system as the main fibers, while for a knitted fabric, a single knit at 22-gauge to 28-gauge is preferred. A woven fabric is more preferred as it can exhibit higher dimensional stability and strength than a knitted fabric. The yarn composing the woven fabric may be monofilament or multifilament yarn. The single fiber fineness of the yarn is preferably 5.5 dtex or smaller to more easily obtain flexible artificial leather using an entangled sheet. The form of the yarn composing the woven fabric may be multifilament gray yarn of polyester or polyamide, or false twisted finished yarn with added twisting of 0 to 3000 T/m. When multifilaments are used they may be common ones, and are preferably 33 dtex/6f, 55 dtex/24f, 83 dtex/36f, 83 dtex/72f, 110 dtex/36f, 110 dtex/48f, 167 dtex/36f or 166 dtex/48f polyester or polyamide, for example. The yarn composing a woven fabric may consist of multifilament long fibers. The woven density of the yarn in a woven fabric is preferably 30/inch to 150/inch and more preferably 40/inch to 100/inch from the viewpoint of obtaining artificial leather that is flexible with excellent mechanical strength. In order to obtain satisfactory mechanical strength and suitable texture, the basis weight of the woven fabric is preferably 20 $g/m^2$ to 150 $g/m^2$. The presence or absence of false twisting, the number of twists, the multifilament single fiber fineness and woven density for a woven fabric also contribute to the entangling properties with the constituent fibers of the main fiber layer, and to the mechanical properties including flexibility, seam strength, tearing strength, tensile strength and elongation and stretchability of the artificial leather, and they may be selected as appropriate for the desired physical properties and intended use.

The lengths of the main fibers in step (1) are not particularly restricted but are preferably 2.5 mm to 90 mm. When a scrim layer is used, main fiber lengths within this range will result in adequate hydroentangling with the scrim layer. If the lengths of the main fibers are 2.5 mm or smaller, the entanglement effect with the scrim layer in the entangling step will be insufficiently exhibited, tending to result in mutual separation between the main fiber layer and scrim layer and potentially causing quality issues, including poor adhesion and poor outer appearance. The lengths of the main fibers are preferably 2.5 mm to 20 mm and more preferably 3 mm to 10 mm.

The lengths of the thermal bonding fibers composed of the thermoplastic resin are preferably 2.0 mm to 90 mm. When a scrim layer is used, thermal bonding fiber lengths in this range will result in sufficient hydroentangling with the scrim layer, and will allow adhesion even between the main fibers and scrim when the thermal bonding fibers are melted. If the lengths of the thermal bonding fibers are smaller than 2.0 mm, the entanglement effect with the scrim layer in the entangling step will be insufficiently exhibited, tending to result in failure of the main fibers and scrim to be adhered when the thermal bonding fiber melts, and potentially causing quality issues, including poor adhesion and poor outer appearance.

The artificial leather of the embodiment includes at least a front side fiber layer constituting a first surface, and when the artificial leather comprises a front side fiber layer with a scrim layer, for example, the first surface (the upper surface) will constitute the front side while the back side of the scrim layer will constitute the second surface (lower surface). When the artificial leather of the embodiment has a three-layer structure consisting of: front side fiber layer/scrim layer/back side fiber layer, the back side of the back side fiber layer will constitute the second surface.

According to one aspect of the embodiment, the material composing the back side fiber layer in the case of a front side fiber layer/scrim layer/back side fiber layer structure is not particularly restricted, but preferably it is the same type of material as the main fibers and/or thermoplastic resin of the front side fiber layer, from the viewpoint of exhibiting satisfactory recycling properties. When a back side fiber layer is included, since it is different from the front side fiber layer, a flame retardant, for example, may be added to impart additional desired properties.

The method for producing artificial leather of the embodiment is preferably a method in which a nonwoven fabric structure formed from the front side fiber layer combining the main fibers with fully-meltable thermal bonding fibers is heat treated to melt the thermal bonding fibers and form thermoplastic resin masses in the front side fiber layer.

The thermal bonding fibers are preferably fully-meltable fibers so that the molten resin masses have a sufficiently large number of points adhering the main fibers. Compared to using sheath/core fibers with a low-melting-point thermoplastic resin as the sheath, or side-by-side fibers with a low melting point thermoplastic resin on only one side, fully-meltable fibers are more preferred because they have more points adhering between the main fibers and result in more adhering component at the bonding sections, thus providing sufficient adhered strength. They are also preferred because the bonding points are not continuous in the same vicinity through the thermal bonding fibers, thus tending to result in a soft hand quality.

The resin component in the thermal bonding fibers is preferably 95% or greater, but infiltrating or adhering additives may also be included so long as the desired effect is exhibited. The types of additives may be titanium oxide, antioxidants, light fastness agents, antistatic agents, flame retardants, flexibilizers, fastness improvers, pigments such as carbon black, or dyes. The preferred range for the resin component in the thermal bonding fibers is 96% or greater, with 97% or greater being more preferred.

From the viewpoint of availability, the thermal bonding fibers (forming the front side fiber layer are preferably polyester fibers, polyamide fibers, acrylic fibers or polyolefin fibers. Suitable polyester fibers for use include polyethylene terephthalate, polytrimethylene terephthalate, polybutylene terephthalate, polyethylene naphthalate and polylactate, as well as their copolymers. Suitable polyamide fibers for use include nylon, and its copolymers. Acrylic fibers may be an acrylic acid ester or methacrylic acid ester polymer, or a copolymer of these. Polyolefin fibers may be polyethylene, polypropylene, polybutene, polystyrene, or a copolymer of these.

The weight-based mixing ratio of the thermal bonding fibers for this embodiment is preferably 3% to 25%. The mixing ratio is the value of the weight of the thermal bonding fibers with respect to the total weight of the main fibers and thermoplastic fibers, expressed as a percentage. If the weight-based mixing ratio is lower than 3% it will not be possible to obtain sufficient fusion strength, making it difficult to obtain satisfactory abrasion resistance and shape stability, while if the weight-based mixing ratio is higher than 25%, the hand quality will become stiffer. The weight-based mixing ratio is more preferably 4% to 20% and even more preferably 6% to 15%.

The melting points of the main fibers and thermal bonding fibers, as referred to herein, are the values measured using a DSC (differential scanning calorimeter). A differential scanning calorimeter is used to measure the difference in heat quantity between the measuring sample and a reference substance, and the melting point of the measuring sample is calculated. Specifically, the melting point is the peak top of the endothermic peak observed when increasing the temperature from 25°C to 250°C at 10°C/min.

The thermal bonding fibers do not necessarily need to consist entirely of a single polymer, and several different polymers may be used in admixture. From the viewpoint of high fusion strength and uniformity of dyeing, it is preferred use the same polymer system as the main fibers. The thermal bonding fibers used may be fibers directly spun by a melt spinning method, fibers obtained by a wet spinning method, or ultrafine fibers obtained by removing the sea component from sea-island fibers comprising copolymerized polyester as the sea component and a regular polyester as the island component. The fineness of the thermal bonding fibers is preferably 0.5 dtex to 2.2 dtex from the viewpoint of the sizes of the thermoplastic resin masses after melting. If the fineness is 2.2 dtex or lower, the thermoplastic resin masses will uniformly disperse on the surface of the front side fiber layer after melting, resulting in excellent appearance quality and hand quality. The fineness of the thermal bonding fibers is more preferably 0.6 dtex to 2.0 dtex and even more preferably 0.7 dtex to 1.5 dtex.

The thermal bonding fibers may also include different types of infiltrating or adhering additives, so long as the desired effect is exhibited. The types of additives may be titanium oxide, antioxidants, light fastness agents, antistatic agents, flame retardants, flexibilizers, fastness improvers, pigments such as carbon black, or dyes.

The method for forming the front side fiber layer may be a method of using the main fibers and/or thermal bonding fibers as staple fibers in a sheet forming method, carding method or airlay method, entangling the fibers to form a nonwoven fabric structure.

From the viewpoint of uniform dispersibility of the constituent fibers and ease of use of the ultrafine fibers, it is most preferred for the front side fiber layer to be formed by a sheet forming method.

While either a hydroenentangling method (also known as spunlace method) or needle punching method may be used for entangling between each layer for this embodiment, hydroenentangling which does not destroy the structure of the woven or knitted fabric scrim layer is preferred.

The method of evaluating the thermoplastic resin may be evaluation by cutting the artificial leather in a perpendicular manner and either observing using an optical microscope or electron beam microscope, or performing measurement on a three-dimensional image obtained by CT or MRI. It is necessary to accurately evaluate the locations, density, form and sizes of the thermal bonding resin not only on the front layer of the front side fiber layer but also in its interior, and such accurate analysis and evaluation can be carried out by continuous image analysis of a cross-section by X-ray CT as described in the Examples, or by mathematical analysis after performing appropriate image processing.

The nonwoven fabric for artificial leather obtained by the method described above may have the surface of the front side fiber layer raised or dyed for use as suede-like or nubuck-like artificial leather. The raising treatment used may be any publicly known method such as buffing with sandpaper. In this case, raising treatment before heat fusion of the thermal bonding fibers of the front side fiber layer can produce a suede-like surface texture. Raising treatment after heat fusion of the thermal bonding fibers, on the other hand, can produce a nubuck-like surface texture.

There are no particular restrictions on the dyeing treatment. For example, a disperse dye is commonly used when the main fibers are polyester fibers. The dyeing method may be any method well-known to those skilled in the art of dyeing, and a jet dyeing machine is suitable for use from the viewpoint of the level dyeing property for artificial leather. Artificial leather dyed in this manner is then subjected to soaping or reduction cleaning in the presence of a chemical reducing agent to remove the excess dye. The conditions for reduction cleaning are not particularly restricted, and any basic reducing agent or acidic reducing agent may be used by a common method, without any particular restrictions.

The thermal annealing (thermal annealing shrinkage) for the embodiment may employ a contact dryer such as a drum dryer or calender roll, or an air-through dryer such as a biaxial stretching film drawing machine or a pin tenter dryer. The thermal annealing temperature is a temperature at least 5°C higher and preferably at least 10°C higher than the melting point of the thermoplastic resin, and 240°C or lower. If the difference between the treatment temperature and the melting point of the thermoplastic fibers is less than 5°C it may not be possible to obtain an adequate adhering effect. If the treatment temperature is higher than 240°C, the polyester fibers may melt, resulting in failure to exhibit adequate surface quality and abrasion resistance.

The nonwoven fabric structure is preferably relaxed during thermal annealing. By carrying out the thermal annealing of the front side fiber web that is entangled with the scrim layer while slackening and causing shrinkage in the MD and CD, the entangled structure becomes relaxed, i.e. the main fibers become fixed without being in a state of tension, making it possible to obtain artificial leather with superior abrasion resistance and good texture compared to using a conventional method in which thermal annealing is carried out while producing tension and shrinkage using a pin tenter.

The shrinkage factor can be calculated by $(L_0 - L)/L \times 100$, where $L_0$ is the initial length of the front side fiber web before thermal annealing and L is the initial length of the front side fiber web after thermal annealing, the shrinkage factor of the front side fiber web in thermal annealing being preferably 0.5 to 5% in the MD and 1 to 8% in the CD.

The thickness of the artificial leather of the embodiment is preferably 0.40 mm to 1.50 mm. If the thickness of the artificial leather is within the range of 0.40 mm to 1.50 mm it will be possible to maintain sufficient bonding points per unit area and entanglement between the fibers in the ultrafine fiber layer of the front side, while also ensuring an adequate thickness for the ultrafine fiber layer, so that both a pliable touch and sufficient stretchability can be obtained.

The basis weight of the artificial leather of the embodiment is preferably 100 g/m² to 400 g/m². Limiting the basis weight to 100 g/m² to 400 g/m² will allow both a pliable touch and suitable hardness to be obtained.

The artificial leather of the embodiment preferably has no exposure of the scrim layer when subjected to abrasion of the surface under a pressing load of 12 kPa for less than 50,000 abrasion cycles according to JIS-L-1096 E (Martindale method), as one measure of satisfactory abrasion resistance for use as artificial leather.

Likewise, the abrasion loss is preferably 21 mg or less after 50,000 abrasion cycles of abrasion of the surface under a pressing load of 12 kPa according to JIS-L-1096 E (Martindale method).

(Aspect 2)

The invention will now be explained in detail using an embodiment.

One embodiment of the invention is artificial leather that includes at least a front side fiber layer constituting a first surface, and having the following features:

(1) the front side fiber layer includes at least one type of main fiber and thermoplastic resins having a melting point of 20°C to 170°C lower than the melting point of the main fibers;
(2) the main fibers have a fineness of 0.01 dtex to 0.5 dtex;
(3) at least part of the thermoplastic resin adheres between the main fibers;
(4) the number average volume of the thermoplastic resin in the first surface fiber layer is 5000 $\mu$m³ to 14,000 $\mu$m³ as measured by X-ray CT of the first surface, and
(5) the volume number density of the thermoplastic resin in the first surface fiber layer is $1.1 \times 10^{12}$/m³ to $3.0 \times 10^{12}$/m³.

The main fibers in the front side fiber layer are fibers included at 60 mass% or greater with respect to 100 mass% of the front side fiber layer. The percentage is more preferably 70 mass% or greater and even more preferably 80 mass% or greater. The upper limit is not particularly restricted but may be 99 mass% or lower.

From the viewpoint of strength, easier production of ultrafine fibers and general-purpose market distribution, the main fibers in the front side fiber layer are preferably polyester fibers, polyamide fibers, acrylic fibers or polyolefin fibers, but considering uses that require durability, such as in the field of car seats as mentioned above, polyethylene terephthalate is preferred from the standpoint of obtaining excellent color fastness without yellowing of the fibers themselves even after prolonged exposure to direct sunlight. More preferred, from the viewpoint of reducing environmental impact, are chemical-recycled or material-recycled polyethylene terephthalate, or polyethylene terephthalate obtained using a plant-derived starting material.

The polyester fibers suitable for use as the main fibers composing the front side fiber layer may be polyethylene terephthalate (PET), polytrimethylene terephthalate, polybutylene terephthalate, polyethylene naphthalate, polylactate, or copolymers of these. The polyamide fibers used may also be nylon, meta-aramid, para-aramid, or copolymers of these. Acrylic fibers may be an acrylic acid ester or methacrylic acid ester polymer, or a copolymer of these. Polyolefin fibers may be polyethylene, polypropylene, polybutene, polystyrene, or a copolymer of these. These fibers may be used alone or in mixtures of different polymer fibers in arbitrary proportions.

If a polyester resin is used to form not only the main fibers but also the thermoplastic resin having a melting point of 20°C to 170°C lower than the melting point of the main fibers in the front side fiber layer described below, as well as the scrim, then it will be possible to recover used PET from clothing or beverage bottles and to process it as recycled PET resin for use in production of artificial leather, and to produce recyclable artificial leather suited for a circular economy in which used artificial leather (with expired lifetime) is subjected to recycling treatment and used to form a heat-insulating material or filter material, for example, and the PET is further recovered from the used heat-insulating material or filter material.

From the viewpoint of helping to obtain a hand quality similar to natural leather or a suede-like or nubuck-like surface texture, the main fibers have a fineness of 0.5 dtex or lower, preferably a fineness of 0.35 dtex or lower and more preferably a fineness of 0.2 dtex or lower. From the viewpoint of production efficiency, production stability and abrasion resistance during production of the fibers, on the other hand the fineness is preferably 0.01 dtex or higher and more preferably 0.03 dtex or higher.

The main fibers used may be fibers directly spun by a melt spinning method, fibers obtained by a wet spinning method, or ultrafine fibers obtained by removing the sea component from sea-island fibers comprising copolymerized polyester as the sea component and a regular polyester as the island component.

The main fibers do not necessarily need to consist entirely of fibers composed of a single polymer, and they may be mixed with fibers composed of different types of polymers. Moreover the main fibers do not necessarily need to consist entirely of fibers of a single fineness, and they may be mixed with fibers having different finenesses.

The main fibers may also have infiltrating or adhering additive types, so long as the desired effect is exhibited. The types of additives may be titanium oxide, antioxidants, light fastness agents, antistatic agents, flame retardants, flexibilizers, fastness improvers, pigments such as carbon black, or dyes.

The thermoplastic resin composing the front side fiber layer, which has a melting point of 20°C to 170°C lower than the melting point of the main fibers, is preferably a polyester resin, polyamide resin, acrylic resin or polyolefin resin from the viewpoint of availability. Suitable polyester resins for use include polyethylene terephthalate, polytrimethylene terephthalate, polybutylene terephthalate, polyethylene naphthalate and polylactate, as well as their copolymers. Suitable polyamide resins for use include nylon, and its copolymers. Suitable acrylic resins for use include acrylic acid ester and methacrylic acid ester polymers, as well as their copolymers. Suitable polyolefin resins for use include polyethylene, polypropylene, polybutene and polystyrene, as well as their copolymers.

The thermoplastic resin, as well, does not necessarily need to consist entirely of a single polymer, and several different polymers may be used in admixture.

The thermoplastic resin may also include different types of infiltrating or adhering additives, so long as the desired effect is exhibited. The types of additives may be titanium oxide, antioxidants, light fastness agents, antistatic agents, flame retardants, flexibilizers, fastness improvers, pigments such as carbon black, or dyes.

The thermoplastic resin has a melting point of 20°C to 170°C lower than the melting point of the main fibers, and when two or more main fibers are present, it must have a melting point of 20°C to 170°C lower than the melting point of the main fibers with the lowest melting point. When the thermoplastic resin comprises different types of polymers mixed together as well, the thermoplastic resin with the highest melting point must have a melting point of 20°C to 170°C lower than the melting point of the main fibers with the lowest melting point. A melting point within this range allows the abrasion resistance and appearance quality described herein to be achieved, but from the viewpoint of satisfactorily maintaining appearance quality, the melting point of the thermoplastic resin is preferably a melting point of 40°C to 150°C lower and more preferably a melting point of 40°C to 100°C lower than the melting point of the main fibers.

The thermoplastic resin must be exposed on the surface of the front side fiber layer in the form of masses. If it is not exposed on the surface, the main fibers on the surface will not be adequately held by fusion of the resin, and this will tend to cause shedding or loss of the fibers, preventing the artificial leather from exhibiting satisfactory abrasion resistance. Some or all of the thermoplastic resin adheres between the main fibers. If the thermoplastic resin does not adhere together the main fibers, holding together of the main fibers will be inadequate, tending to result in shedding or loss of the fibers, and thus preventing the artificial leather from exhibiting satisfactory abrasion resistance and strength.

The thermoplastic resin must also be exposed in the form of masses on the cross-sections of the front side fiber layer. If it is present only on the front side but not present in the form of masses on the cross-sections, the main fibers will not be adequately held by fusion of the resin, and not only will the artificial leather fail to exhibit satisfactory abrasion resistance, but increase in the density of the front side fiber layer will also be inadequate and the luxurious moist texture characteristic of artificial leather will not be exhibited.

For this embodiment, it is necessary for the thermoplastic resin to adhere between the main fibers and for the number average volume of the thermoplastic resin to be 5000 $\mu m^3$ to 14,000 $\mu m^3$. If the number average volume of the thermoplastic resin exceeds 14,000 $\mu m^3$, the proportion of multiple main fibers being adhered by the same thermoplastic resin will increase, tending to result in a rougher feel and making it impossible to obtain a satisfactory luxurious quality for artificial leather. If the number average volume is less than 5000 $\mu m^3$, on the other hand, an adequate size will not be exhibited to allow each single main fiber to be surrounded, making it impossible to effectively hold the main fibers or to exhibit the abrasion resistance required for a car interior. The preferred range for the number average volume is 5500 $\mu m^3$ to 13,000 $\mu m^3$, with 5500 $\mu m^3$ to 12,000 $\mu m^3$ being more preferred.

The volume number density, as the number of thermoplastic resin masses per unit volume in the first surface fiber layer, must be $1.1 \times 10^{12}/m^3$ to $3.0 \times 10^{12}/m^3$. If the number for the thermoplastic resin per unit volume is less than $1.1 \times 10^{12}/m^3$, multiple main fibers will not be able to be bonded by multiple thermoplastic resin masses, resulting in more shedding of fiber bundles when abraded and making it impossible to exhibit adequate abrasion resistance. If the volume number density of the thermoplastic resin is greater than $3.0 \times 10^{12}/m^3$, on the other hand, the first surface fiber layer will become too hard, making it impossible to obtain a pliable luxurious quality as required for artificial leather. The volume number density of the thermoplastic resin is preferably $1.1 \times 10^{12}/m^3$ to $2.0 \times 10^{12}/m^3$ and more preferably $1.1 \times 10^{12}/m^3$ to $1.6 \times 10^{12}/m^3$.

From the viewpoint of obtaining higher stretch properties for this embodiment, the artificial leather preferably has the following features:

(6) at least part of the thermoplastic resin is exposed on the surface of the front side fiber layer in the form of resin masses, at a projected area mean value of $0.66 \times 10^{-9} m^2$ to $5.0 \times 10^{-9} m^2$ per resin mass;
(7) the front side fiber layer is integrally entangled with the woven scrim layer; and
(8) the artificial leather satisfies the following inequality:

$$220{,}000 \le (A) \times (B) \times (C) \le 600{,}000$$

where (A) is the sum of the elongation under 500 gf/cm constant load in the MD (%) and the elongation under 500 gf/cm constant load in the CD (%), (B) is the sum of the scrim layer woven density in the MD (number/2.54 cm) and the woven density in the CD (number/2.54 cm), and (C) is the fineness (denier) of the weaving yarn forming the scrim layer,
and which has an elongation of 3% or greater under 500 gf/cm constant load in the MD.

At least part of the thermoplastic resin is preferably exposed on the surface of the front side fiber layer in the form of resin masses at a projected area mean value of $0.66 \times 10^{-9} m^2$ to $5.0 \times 10^{-9} m^2$ and more preferably $0.66 \times 10^{-9} m^2$ to $3.0 \times 10^{-9} m^2$, per resin mass. If the average projected area is $5.0 \times 10^{-9} m^2$ or smaller, the sizes of the resin masses on the surface will be adequate compared to the main fibers, resulting in excellent appearance quality and tactile hand quality, while the density of the number of bonding points between main fibers will also be suitable, resulting in sufficient holding together of the main fibers and excellent abrasion resistance of the front side layer. The average projected area is preferably $3.0 \times 10^{-9} m^2$ or smaller and more preferably $1.5 \times 10^{-9} m^2$ or smaller. The average projected area is also preferably $0.66 \times 10^{-9} m^2$ or greater so that the resin masses will be large enough to adhere together the main fibers.

From the viewpoint of exhibiting high stretchability, the artificial leather of the embodiment preferably satisfies the following inequality:

$$220{,}000 \le (A) \times (B) \times (C) \le 600{,}000$$

where (A) is the sum of the elongation under 500 gf/cm constant load in the MD (%) and the elongation under 500 gf/cm constant load in the CD (%), (B) is the sum of the scrim layer woven density in the MD (number/2.54 cm) and the woven density in the CD (number/2.54 cm), and (C) is the single fiber fineness (denier) of the weaving yarn forming the scrim layer, and has an elongation of 3% or greater under 500 gf/cm constant load in the MD. Such high stretchability can be achieved by thermal bonding with the entangled structure in a relaxed state (also referred to herein as "thermal annealing shrinkage"), as explained below. The value of (A) × (B) × (C) is preferably 230,000 to 500,000 and more preferably 240,000 to 400,000. The elongation under a 500 gf/cm constant load in the MD is preferably 4% to 15% and more preferably 4.5% to 7%. For the MD and CD of the artificial leather, in the case of a full-width roll, they can be determined using the direction in which the artificial leather is taken up, or by marks from the pins of a pin tenter dryer that remain at the edge, or in the case of cut edges, by how the raised fibers are aligned in the MD when the surface is stroked, as characteristics of the process for producing the artificial leather.

The action mechanism by which stretchability is exhibited is understood to be in a complex correlation with the flexibility and elongation during elastic deformation of the artificial leather, and the surface quality. The flexibility and elongation during elastic deformation of the artificial leather largely depends on how the elastic deformation behavior of the scrim layer used as the center core material of the artificial leather is affected by the front and back ultrafine fiber layers and the sizes and numbers of the bonding points of the thermal bonding fibers bonding them together. For a car seat, as with a ceiling, artificial leather must undergo suitable elastic deformation to stretch the artificial leather over a complex and wide area while maintaining its surface quality, and in addition it must maintain its quality even after stretching. As a result of further verification with this in mind, the present inventors found that the aforementioned intended effect can be described by evaluating the elastic deformation behavior of the artificial leather based on the product of the fineness (denier) of the scrim layer and the woven density in the MD and CD, and further multiplying this by the elongation of the artificial leather under constant load. The value of (A) × (B) × (C) derived using this concept is affected by the bonding point density of the entangled thermal bonding fibers. If the value of (A) × (B) × (C) is 220,000 or greater, the scrim layer will have sufficient stretchability, thereby allowing stretching attachment without wrinkles during the stretching attachment step. If the value of (A) × (B) × (C) is 600,000 or less, the ultrafine fibers on the front layer will maintain suitable density and the artificial leather will exhibit its luxurious surface quality even after being stretched.

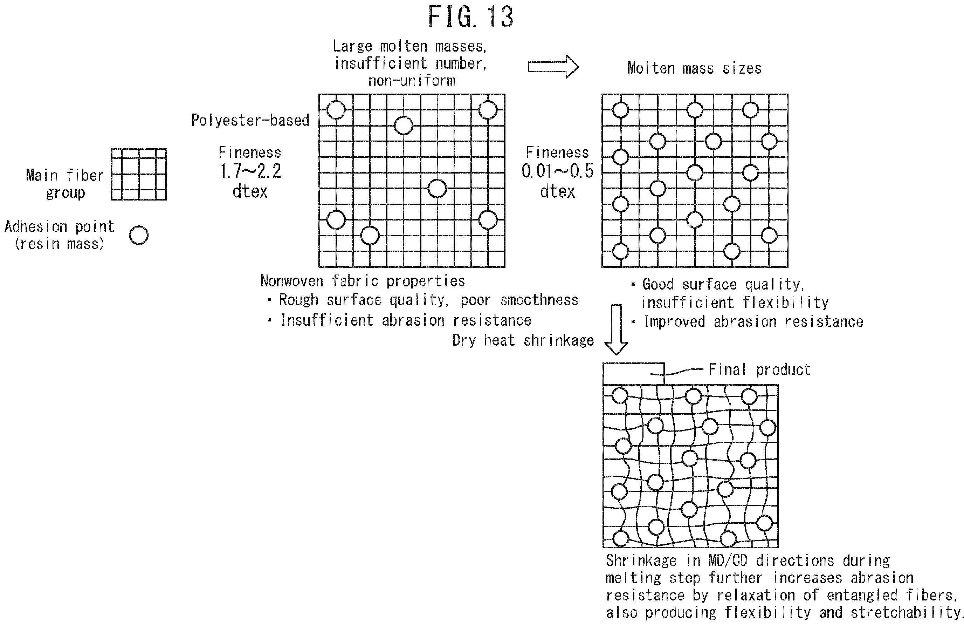

By carrying out thermal annealing of the front side fiber web that is entangled and integrated with the scrim layer while slackening and causing shrinkage in the MD and CD, the entangled structure becomes relaxed as shown in Fig. 13, i.e. the main fibers become fixed without being in a state of tension, making it possible to obtain artificial leather with the desired stretchability and flexibility, compared to using a conventional method in which heat fusion is carried out while producing tension and shrinkage using a pin tenter.

Another embodiment of the invention is a method for producing artificial leather having the following steps:

(1) a step in which the main fibers are mixed with thermal bonding fibers composed of the thermoplastic resin having a melting point of 20°C to 170°C lower than the melting point of the main fibers, so that the weight ratio of the thermal bonding fibers is 3% to 25% with respect to the front side fiber layer, and then the fibers are entangled by wet method (papermaking method) on a scrim layer, after which hydroentangling or needle punching is carried out to form a front side fiber web entangled with the scrim layer, and

(2) a step in which the entangled structure of the obtained front side fiber web is subjected to thermal annealing shrinkage at a temperature at or above the melting point of the thermal bonding fibers and below the melting point of the main fibers, and relaxed to form a front side fiber layer.

The mixing ratio of the thermal bonding fibers in the front side fiber layer for this embodiment is preferably 3% to 25%. The mixing ratio is the value of the weight of the thermal bonding fibers with respect to the total weight of the main fibers and thermoplastic fibers, expressed as a percentage. If the mixing ratio is lower than 3% it will not be possible to obtain sufficient fusion strength, making it difficult to obtain satisfactory abrasion resistance and shape stability, while if the mixing ratio is higher than 25%, the hand quality will become stiffer. The mixing ratio is more preferably 4% to 20% and even more preferably 6% to 15%.

The scrim layer of the artificial leather of the embodiment functions as a core material, and it is preferably a woven fabric or knitted fabric from the viewpoint of stabilizing fabrication of a sheet in the sheet-forming process, or helping to increase mechanical strength of the obtained artificial leather. From the viewpoint of color uniformity in dyeing, the material of the scrim layer is preferably the same polymer system as the main fibers, while for a knitted fabric, a single knit at 22-gauge to 28-gauge is preferred. A woven fabric is more preferred as it can exhibit higher dimensional stability and strength than a knitted fabric. The yarn composing the woven fabric may be monofilament or multifilament yarn. The single fiber fineness of the yarn is preferably 5.5 dtex or smaller to more easily obtain flexible artificial leather using an entangled sheet. The form of the yarn composing the woven fabric may be multifilament gray yarn of polyester or polyamide, or false twisted finished yarn with added twisting of 0 to 3000 T/m. When multifilaments are used they may be common ones, and are preferably 33 dtex/6f, 55 dtex/24f, 83 dtex/36f, 83 dtex/72f, 110 dtex/36f, 110 dtex/48f, 167 dtex/36f or 166 dtex/48f polyester or polyamide, for example. The yarn composing a woven fabric may consist of multifilament long fibers. The woven density of the yarn in a woven fabric is preferably 30/inch to 150/inch and more preferably 40/inch to 100/inch from the viewpoint of obtaining artificial leather that is flexible with excellent mechanical strength. In order to obtain satisfactory mechanical strength and suitable texture, the basis weight of the woven fabric is preferably 20 g/m² to 150 g/m². The presence or absence of false twisting, the number of twists, the multifilament single fiber fineness and woven density for a woven fabric also contribute to the entangling properties with the constituent fibers of the main fiber layer, and to the mechanical properties including flexibility, seam strength, tearing strength, tensile strength and elongation and stretchability of the artificial leather, and they may be selected as appropriate for the desired physical properties and intended use.

The woven density (number/2.54 cm) in the MD and CD of the scrim layer is preferably 40 to 100, more preferably 45 to 80 and even more preferably 50 to 70. It can be determined which direction is the MD in which the obtained artificial leather has been taken up onto the roll, and which is the CD perpendicular to the MD, in the case of a full-width roll, using the direction in which the artificial leather is taken up, or by marks from the pins of a pin tenter dryer that remain at the edge, or in the case of cut edges, by how the raised fibers are aligned in the MD when the surface is stroked, as characteristics of the process for producing the artificial leather.

The fineness of the weaving yarn forming the scrim layer is preferably 60 dtex to 200 dtex, more preferably 65 dtex to 170 dtex and even more preferably 70 dtex to 150 dtex, from the viewpoint of exhibiting satisfactory stretchability and mechanical strength.

The lengths of the main fibers in step (1) are not particularly restricted but are preferably 2.5 mm to 90 mm. Main fiber lengths within this range will result in adequate hydroentangling with the scrim layer. If the lengths of the main fibers are 2.5 mm or smaller, the entanglement effect with the scrim layer in the entangling step will be insufficiently exhibited, tending to result in mutual separation between the main fiber layer and scrim layer and potentially causing quality issues, including poor adhesion and poor outer appearance. The lengths of the main fibers are preferably 2.5 mm to 20 mm and more preferably 3 mm to 10 mm.

The artificial leather of the embodiment includes at least a front side fiber layer constituting a first surface, and when the artificial leather comprises a front side fiber layer with a scrim layer, for example, the first surface (the upper surface) will constitute the front side while the back side of the scrim layer will constitute the second surface (lower surface). When the artificial leather of the embodiment has a three-layer structure consisting of: front side fiber layer/scrim layer/back side fiber layer, the back side of the back side fiber layer will constitute the second surface.

According to one aspect of the embodiment, the material composing the back side fiber layer in the case of a front side fiber layer/scrim layer/back side fiber layer structure is not particularly restricted, but preferably it is the same type of material as the main fibers and/or thermoplastic resin of the front side fiber layer, from the viewpoint of exhibiting satisfactory recycling properties. When a back side fiber layer is included, since it is different from the front side fiber layer, a flame retardant, for example, may be added to impart additional desired properties.

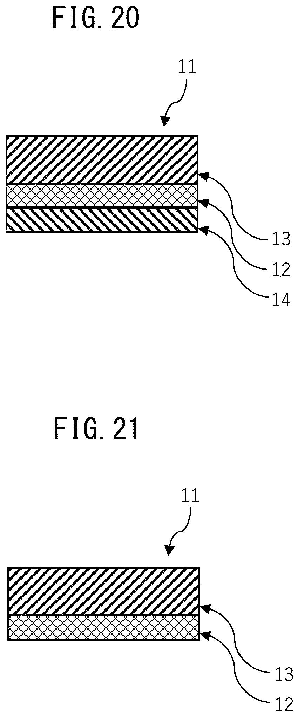

Fig. 20 shows a fiber sheet 11 including a woven or knitted fabric scrim 12, a front side fiber layer (A) 13 forming the front side and a fiber layer (B) 14 forming the back side. However, the fiber layer (B) 14 is optional and is not an essential element. Fig. 21 shows a fiber sheet 11 including a woven or knitted fabric scrim 12 and a front side fiber layer (A) 13.

The method for producing artificial leather of the embodiment is preferably a method in which a nonwoven fabric structure formed from the front side fiber layer combining at least one type of main fiber with fully-meltable type thermal bonding fibers having a melting point of 20°C to 170°C lower than the melting point of the main fibers, is heat treated to melt the thermal bonding fibers and form thermoplastic resin masses in the front side fiber layer.

The thermal bonding fibers are preferably fully-meltable fibers so that the molten resin masses have a sufficiently large number of points adhering the main fibers. Compared to using sheath/core fibers with a low-melting-point thermoplastic resin as the sheath, or side-by-side fibers with a low melting point thermoplastic resin on only one side, fully-meltable fibers are more preferred because they have more points adhering between the main fibers and result in more adhering component at the bonding sections, thus providing sufficient adhered strength. They are also preferred because the bonding points are not continuous in the same vicinity through the thermal bonding fibers, thus tending to result in a soft hand quality.

The thermal bonding fibers having a melting point of 20°C to 170°C lower than the melting point of the main fibers, which form the front side fiber layer, are preferably polyester fibers, polyamide fibers, acrylic fibers or polyolefin fibers, from the viewpoint of availability. Suitable polyester fibers for use include polyethylene terephthalate, polytrimethylene terephthalate, polybutylene terephthalate, polyethylene naphthalate and polylactate, as well as their copolymers. Suitable polyamide fibers for use include nylon, and its copolymers. Acrylic fibers may be an acrylic acid ester or methacrylic acid ester polymer, or a copolymer of these. Polyolefin fibers may be polyethylene, polypropylene, polybutene, polystyrene, or a copolymer of these.

The melting point, as referred to herein, is the value measured using a DSC (differential scanning calorimeter). A differential scanning calorimeter is used to measure the difference in heat quantity between the measuring sample and a reference substance, and the melting point of the measuring sample is calculated. Specifically, the melting point is the peak top of the endothermic peak observed when increasing the temperature from 25°C to 250°C at 10°C/min.

The thermal bonding fibers do not necessarily need to consist entirely of a single polymer, and several different polymers may be used in admixture. From the viewpoint of high fusion strength and uniformity of dyeing, it is preferred use the same polymer system as the main fibers. The thermal bonding fibers used may be fibers directly spun by a melt spinning method, fibers obtained by a wet spinning method, or ultrafine fibers obtained by removing the sea component from sea-island fibers comprising copolymerized polyester as the sea component and a regular polyester as the island component. The fineness of the thermal bonding fibers is preferably 0.5 dtex to 2.2 dtex from the viewpoint of the sizes of the thermoplastic resin masses after melting. If the fineness is 2.2 dtex or lower, the thermoplastic resin masses will uniformly disperse on the surface of the front side fiber layer after melting, resulting in excellent appearance quality and hand quality. The fineness of the thermal bonding fibers is more preferably 0.6 dtex to 2.0 dtex and even more preferably 0.7 dtex to 1.5 dtex.

The thermal bonding fibers may also include different types of infiltrating or adhering additives, so long as the desired effect is exhibited. The types of additives may be titanium oxide, antioxidants, light fastness agents, antistatic agents, flame retardants, flexibilizers, fastness improvers, pigments such as carbon black, or dyes.

The method for forming the front side fiber layer may be a method of using the main fibers and/or thermal bonding fibers as staple fibers in a sheet forming method, carding method or airlay method, entangling the fibers to form a nonwoven fabric structure.

From the viewpoint of uniform dispersibility of the constituent fibers and ease of use of the ultrafine fibers, it is most preferred for the front side fiber layer to be formed by a sheet forming method.

While either a hydroentangling method (also known as spunlace method) or needle punching method may be used for entangling between each layer for this embodiment, hydroentangling which does not destroy the structure of the woven or knitted fabric scrim layer is preferred.

Thermal annealing with entangled structure in a relaxed state (thermal annealing shrinkage)

The thermal annealing for the embodiment may employ a contact dryer such as a drum dryer or calender roll, or an air-through dryer such as a biaxial stretching film drawing machine or a pin tenter dryer. The treatment temperature may be a temperature of at least 5°C higher and preferably a temperature of at least 10°C higher than the melting point of the thermal bonding fibers composed of the thermoplastic resin having a melting point of 20°C to 170°C lower than the melting point of the main fibers in the front side fiber layer, and it may be 240°C or lower and preferably 200°C or lower. If the difference between the thermal annealing temperature and the melting point of the thermoplastic fibers is less than 5°C it may not be possible to obtain an adequate heat adhering effect. If the thermal annealing temperature is higher than 240°C, the polyester main fibers will melt causing impairment of the luxurious surface quality, and preventing abrasion resistance from being adequately exhibited.

Fig. 13 is a conceptual drawing which illustrates thermal annealing shrinkage. In order to exhibit the desired properties by the artificial leather of the embodiment, it is necessary to adhere between the main fibers by the melted thermoplastic resin during thermal annealing (thermal bonding) while the entangled structure of the main fibers are relaxed. The action mechanism for both abrasion resistance and hand quality of the artificial leather of the embodiment is understood to be in a complex correlation with the luxurious characteristic texture of artificial leather and the size and density of the thermoplastic resin in the front side fiber layer. Specifically, it is necessary for the design to be such that the distribution density of the thermoplastic resin in front side fiber layer increases while the size of the thermoplastic resin is limited to a given fixed value. If the size of the thermoplastic resin is too large, any unevenness will be felt by finger touch and the smooth texture will be impaired, making it impossible to obtain a luxurious tactile sensation as required for artificial leather. If the distribution density of the thermoplastic resin is too low, on the other hand, an adequate adhering effect between the main fibers will not be obtained and the frequency of shedding of collective fiber bundles from the base material during abrasion will increase, thus increasing the amount of abrasion loss and making it impossible to adequately maintain product life, while also tending to alter the outer appearance due to exposure of the scrim layer, and consequently failing to fully satisfy the performance required for the product.

The size and density of the thermoplastic resin (thermal bonding points) is important in order to exhibit the effect of the invention, but the method of evaluating the thermoplastic resin may be cutting the artificial leather in a perpendicular manner and either observing it using an optical microscope or electron beam microscope or performing measurement on a three-dimensional image obtained by CT or MRI. It is necessary to accurately evaluate the locations, density, form and sizes of the thermal bonding resin not only on the front layer but also in its interior, and such accurate analysis and evaluation can be carried out by continuous image analysis of a cross-section by X-ray CT, or by mathematical analysis after performing appropriate image processing.

In order to exhibit the effect of the invention it is also necessary to control the bonding point density during thermal annealing, and preferably the nonwoven fabric structure is relaxed while the thermal annealing effect is being exhibited. Preferably, the heat fusion of the front side fiber web that is entangled with the scrim layer is carried out while slackening and shrinking in the MD which is the traveling direction of the nonwoven fabric structure during the production process and in the CD which is the direction perpendicular to the MD, so that the entangled structure shown in Fig. 13 becomes relaxed, i.e. the main fibers become fixed without being in a state of tension, making it possible to obtain the desired size and density for the thermoplastic resin (thermal bonding points) and to obtain artificial leather with high abrasion resistance and good texture compared to using a conventional method in which heat fusion is carried out while producing tension and shrinkage using a pin tenter.

The shrinkage factor can be calculated by $(L_0 - L)/L \times 100$, where $L_0$ is the initial length of the front side fiber web before thermal annealing and L is the initial length of the front side fiber web after thermal annealing, the shrinkage factor of the front side fiber web in thermal annealing being preferably 0.5 to 5% in the MD and 1 to 8% in the CD.

A "+" symbol under "Thermal annealing shrinkage" in Tables 4 and 5 indicates that the nonwoven fabric was subjected to heat fusion treatment with shrinkage, "0" indicates that the nonwoven fabric was subjected to heat fusion treatment without expansion or shrinkage, and "-" indicates that the nonwoven fabric was subjected to heat fusion treatment in a stretched state.

The nonwoven fabric for artificial leather obtained by the method described above may have the surface of the front side fiber layer raised or dyed for use as suede-like or nubuck-like artificial leather. The raising treatment used may be any publicly known method such as buffing with sandpaper. In this case, raising treatment before heat fusion of the thermal bonding fibers of the front side fiber layer can produce a suede-like surface texture. Raising treatment after heat fusion of the thermal bonding fibers, on the other hand, can produce a nubuck-like surface texture.

There are no particular restrictions on the dyeing treatment. A disperse dye is commonly used when the main fibers are polyester fibers, for example. The dyeing method may be any method well-known to those skilled in the art of dyeing, and a jet dyeing machine is suitable for use from the viewpoint of the level dyeing property for artificial leather. Artificial leather dyed in this manner is then subjected to soaping or reduction cleaning in the presence of a chemical reducing agent to remove the excess dye. The conditions for reduction cleaning are not particularly restricted, as it is sufficient if the conditions are suitable for chemical stability of the main fibers or thermoplastic resin, while any basic reducing agent or acidic reducing agent may be used by a common method, without any particular restrictions.

The thickness of the artificial leather of the embodiment is preferably 0.40 mm to 1.50 mm. If the thickness of the artificial leather is within the range of 0.40 mm to 1.50 mm it will be possible to maintain sufficient bonding points per unit area and entanglement between the fibers in the ultrafine fiber layer of the front side, while also ensuring an adequate thickness for the ultrafine fiber layer, so that both a pliable touch and sufficient stretchability can be obtained.

The basis weight of the artificial leather of the embodiment is preferably 100 g/m$^2$ to 400 g/m$^2$. Limiting the basis weight to 100 g/m$^2$ to 400 g/m$^2$ will allow both a pliable touch and suitable hardness to be obtained. The basis weight is more preferably 200 g/m$^2$ to 300 g/m$^2$.

The artificial leather of the embodiment preferably has a hand quality value (flexural rigidity per unit width in KES pure bending measurement, as a substitute property for hand quality) of 1.0 gfcm$^2$/cm or lower as measured by the method described below, which is satisfactory hand quality for use as artificial leather. For measurement of the hand quality value, while this is not limitative, it is preferred to use a sample with a width of 20 cm for evaluation using a commercially available KES pure bending tester, calculating the resulting flexural rigidity per unit width. With a hand quality value of 1.0 gfcm$^2$/cm or lower it will be possible to obtain satisfactory quality, characteristic of artificial leather.

The artificial leather of the embodiment preferably has no exposure of the scrim layer when subjected to abrasion of the surface under a pressing load of 12 kPa for less than 50,000 abrasion cycles according to JIS-L-1096 E (Martindale method), as one measure of satisfactory abrasion resistance for use as artificial leather.

The artificial leather of the embodiment preferably exhibits flame retardance of grade 4 or higher in a combustibility test according to US Federal Motor Vehicle Safety Standards "FMVSS No. 302". If flame retardance of grade 4 or higher is maintained then the artificial leather can clear the flammability standards used for automobiles or aircraft, allowing its use for a wider range of purposes.

EXAMPLES

(Aspect 1)

The present invention will now be explained in greater detail using Examples and Comparative Examples, with the understanding that the invention is not limited only to the Examples. The values for the physical properties used in the Examples were measured by the following methods.

(1) Force curve measurement preprocessing

The artificial leather was cut to a width of 5 mm and a length of 10 mm to fabricate a sample sheet. The sheet was mixed with 9.7 ml of Quetol812 by Nisshin-EM, 3.7 ml of MNA, 6.6 ml of DDSA and 0.34 ml of DMP-30 to prepare a resin composition, and after vacuum defoaming, the sample prepared with the epoxy resin was packed into a silicon embedding plate and hardened for 3 days in a thermostatic bath at 60°C. The composite of the sample and cured epoxy resin was removed and a cryomicrotome (Ultracut-UCT by Leica) was used to prepare a precision cross-section of the sample.

(2) Force curve measurement

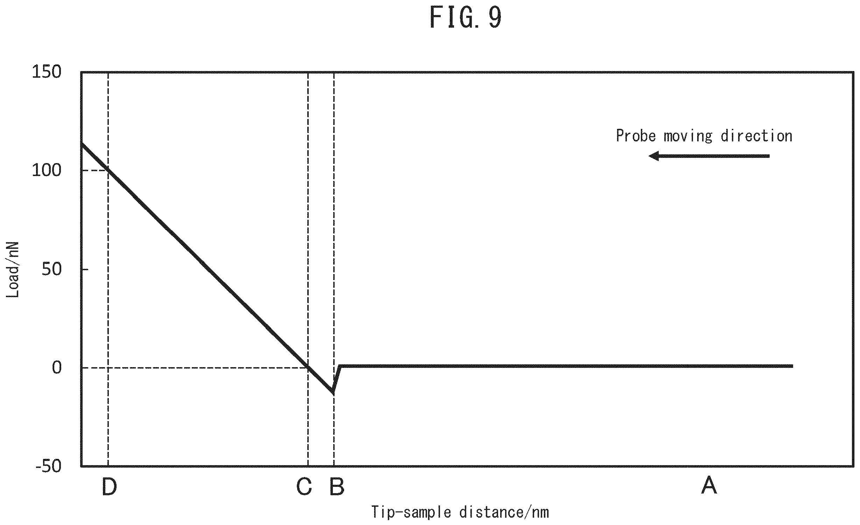

The amount of deformation of the thermoplastic resin can be measured with force curve mode of an atomic force microscope. The cantilever of the atomic force microscope was pressed and then retracted in the direction perpendicular to the thermoplastic resin in the cryomicrotome-prepared sample cross-section, to obtain the force curve shown in Fig. 9.

The atomic force microscope used was a Dimension Icon by Bruker equipped with a force curve measurement function, and the probe was an RTESPA-type silicon single crystal probe by Bruker.

The probe was calibrated prior to measurement. First, the bending sensitivity of the cantilever was measured on a sapphire board. The spring constant of the cantilever was then measured by the thermal vibration method and calibration was performed, to conduct AFM image measurement of the cryomicrotome-prepared sample cross-section under conditions allowing quantification of the tip-sample distance and the load, determining the location of the thermoplastic resin based on its shape characteristics. The epoxy resin is observed as the matrix having the widest area while the main fiber cross-sections are observed to have fixed round or elliptical shapes, and the thermoplastic resin component is observed as amorphous regions. During measurement of the force curve of the thermoplastic resin, a location at least 1 $\mu$m from the interface between the thermoplastic resin and epoxy resin and from the interface between the thermoplastic resin and main fiber was selected as the measurement location in order to avoid the effect of hardness of the epoxy resin or yarn. The load during measurement was 200 nN, and the force curve measurement was conducted with a measuring frequency of 1 Hertz. In the force curve shown in Fig. 9, with tip-sample distance on the abscissa and load on the ordinate, the distance between CD is the deformation of the thermoplastic resin as the probe approached the sample, where A is the state before contact, B is the point where the load became negative due to jump-in at the start of contact, C is the point where the load increased to 0 and D is the point where the load reached 200 nN. The force curve measurement was conducted 15 times selecting different thermoplastic resin regions, and the added average of the deformation of the thermoplastic resin was used as the measured value.

(3) Number average volume ($\mu$m$^3$) and volume number density ($10^{12}$/m$^3$)

[Image measurement using X-ray CT]

Observation of the mass portions at the bonding points was in the thickness direction of the artificial leather using an X-ray CT apparatus ("Nano3DX High-Resolution 3D X-ray microscope" by Rigaku Corp.), photographing a 3D image with the center section of the cross-section in the thickness direction as the center point of the observation region. The image measurement was performed using copper as the X-ray target, under conditions with a tube voltage of 40 kV, a tube current of 30 mA, an exposure time of 12 seconds/image and a spatial resolution of 1.08 $\mu$m/pixel. The same procedure was used to take 1000 images for every 180° rotation angle, to obtain 3D measurement data.

[Image processing method, analysis method, and calculation method for added average value of long axis/short axis quotient, with elliptical approximation]

The detailed process for processing and analysis of the acquired 3D measurement data is described below. The image analysis software used was "ImageJ (version 1.51j8), US National Institutes of Health).

(i: Image rotation) The in-plane direction of the artificial leather is aligned with the xz axis plane, and 3D image is rotated with the thickness direction of the artificial leather on the y-axis.

(ii: Trimming) The 3D image is trimmed to a rectangular solid. The y-axis is set as a range so that the entire thickness fits without excess space outside the film. The x-axis and z-axis are each set as the maximum range so that no pixel outside the measuring field is present within the trimmed rectangular solid, after setting the y-axis range.

(iii: Axis setting) The pixel count in the x-axis direction of the trimmed image is x0, the pixel count in the y-axis direction is y0, and the pixel count in the z-axis direction is z0.

(iv: Filtering) A median filter is applied with the condition of radius 2 pix.

(v: Region partitioning) The Otsu method is used, with partitioning into two regions: an air region excluding the artificial leather and a main fiber region forming the artificial leather. The pixel brightness values are set as 0 in the air region excluding the artificial leather and 255 in the main fiber region forming the artificial leather.

(vi: Segmentation) The pixels with 255 brightness values are segmented by image processing, partitioning 255 brightness value subregions that are connected in three dimensions. Of the 255 brightness value subregions connected in three dimensions, the subregions with total pixel counts (pix) of 10,000 or less are considered to be noise and are removed by changing their brightness values to 0.

(vii: Noise removal) The pixels with brightness values of 0 that are surrounded by brightness values of 255 in three dimensions are considered to be noise and are removed by setting their brightness values to 255.

(viii: Calculation of void percentage) A 2D image on the xz plane is cut out from the 3D image to a thickness of 1 pix in the thickness direction, and the void percentage on that plane is calculated by the following formula:

Void percentage = Number of pixels of brightness value 0 in 2D image / total number of pixels in 2D image.

(ix: Thickness distribution of void percentage) The procedure of (viii) above is carried out for all of the y data, and the distribution of the void percentage in the y-axis direction is calculated.

(x: Calculation of high-brightness pixel size) The sizes of pixels with brightness values of 255 are calculated by the Thickness method. This method produces a 3D image in which the value of the diameter of the maximum sphere at the location of each pixel has been represented as a brightness value. The Thickness method is described in the publication: "A new method for the model-independent assessment of thickness in three-dimensional images" T. Hildebrand and P. Ruegsegger, J. of Microscopy, 185 (1996) 67-75, and can be performed, for example, by executing "Thickness" in the plugin BoneJ for the image analysis software ImageJ. In order to remove structures of 12 $\mu$m or smaller, as main fibers, from the scope of analysis in the obtained image, the brightness values of pixels corresponding to $\leq$12 $\mu$m are set to 0.

(xi: Binarization) the image obtained in (x) above is binarized, setting all pixels with brightness values other than 0 to 255, and leaving pixels with brightness values of 0 as 0.

(xii: Particle analysis) The image obtained in (xi) above is used for 3D particle analysis. Portions with brightness values of 255 connected in three dimensions are considered to be one particle, and the center coordinate (XC, YC, ZC) of each particle and the pixel count are calculated.

(xiii: Definition of front side, scrim layer and back side) Since 95% of the area in the void percentage distribution data obtained in (ix) above is to be used for analysis (excluding the outer surfaces of the front side and back side for analysis), y1 is defined as the y-axis value furthest from the front side where the void percentage is 0.95 or greater. When a scrim layer is present, the portion with the void percentage numerical value obtained in (viii) and showing fibers with a fineness of 100 dtex or greater is defined as the edge of the front side, and its coordinate is set as y2.

(xiv: Pixel count total calculation and data analysis) The number of particles with coordinate values satisfying y1 < YC < y2 among all of the particles calculated in (xii) is calculated as the particle count, and the total pixel count of the particles is calculated.

Number average volume ($\mu$m$^3$) = Total pixel count of particles $*p*p*p*10^{18} \div$ number of particles

Volume number density ($10^{12}$/m$^3$) = Number of particles $\div$ (|y2 - y1|$*p*x0*p*z0*p$)

In the formulas, p represents the pixel size (m/pix), using the actual size (m) for each pixel (pix).

(xv: Calculation of average of quotient of long axis divided by short axis (long axis/short axis ratio), using elliptical approximation, and data analysis) The image obtained in (xi) is used for 3D particle analysis. A portion with brightness value of 255 connected in three dimensions is considered to be one particle, and the particle is approximated as an oval sphere. The three axes of the oval sphere are designated as the long axis, middle axis and short axis in order of length, and the length (pix) on each axis is determined. The average value for the quotient of the long axis divided by the short axis for each particle, approximated as elliptical, was calculated by the following formula:

Quotient of long axis divided by short axis (long axis / short axis ratio for thermoplastic resin) = long axis length / short axis length.

When the long axis of the approximated oval sphere was larger than the smallest axis of the analyzed rectangular solid, this was ignored as an error and the aspect ratio was recorded as 0. The mean aspect ratio was calculated by the following formula:

$$\text{Average quotient of long axis divided by short axis} = \text{Sum of quotient of long axis divided}$$

$$\text{by short axis for particles} \div (\text{total number of particles} - \text{total number excluded}).$$

(4) Melting point

The melting point of the thermal bonding fibers was determined with a DSCQ 100 by TA Instruments using aluminum as the reference substance, setting 3 mg of thermal bonding fibers in a nitrogen atmosphere, increasing the temperature from 25°C to 250°C at 10°C/min and then quenching, and recording the melting point as the peak top of the endothermic peak appearing during a second temperature increase under the same conditions.

(5) Explanation of bonding point structure

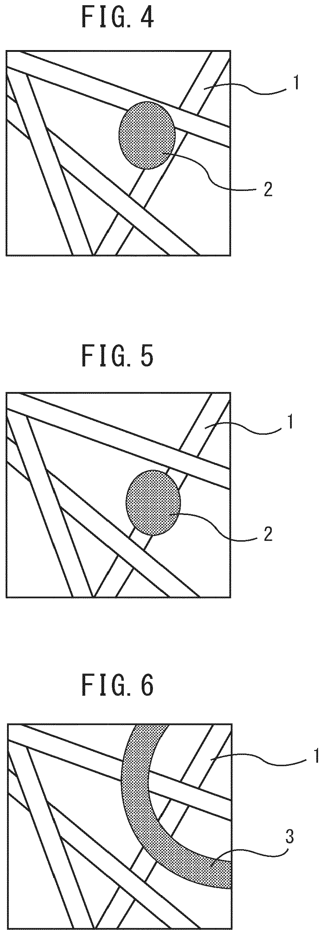

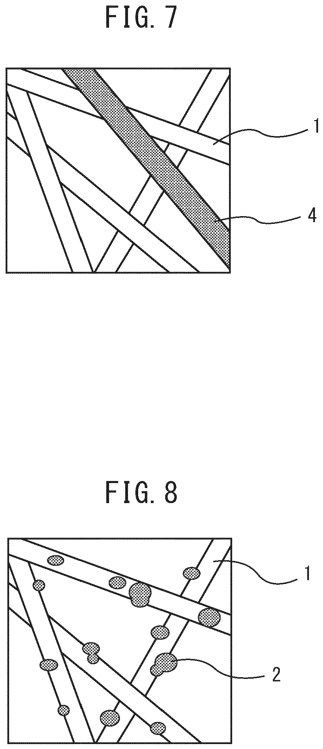

Regarding the states of resin masses and their assessment as shown in the drawings, Fig. 1 is an example where one is exposed on the surface of the front side fiber layer but does not adhere between main fibers, and Fig. 2 is an example where one is exposed on the surface and adheres between main fibers. Fig. 3 is an example where one is not exposed on the surface and yet adheres between main fibers, Fig. 4 is an example where one is exposed on the surface and adheres between main fibers, and Fig. 5 is an example where one is exposed on the surface but does not adhere between main fibers. Fig. 6 is an example where a thermal bonding fiber is unmelted and cannot be considered as a resin mass. Fig. 7 is an example where a sheath-core fiber is used as the thermal bonding fiber, and the thermal bonding fiber retains its fiber shape and cannot be considered as a resin mass. Fig. 8 is an example of resin masses exposed on the surface, but without adhering between the main fibers. In these drawings, 1 is a main fiber, 2 is a resin mass, 3 is an unmelted resin and 4 is a sheath-core fiber.

The phrase "adheres between main fibers" means that at least two main fibers pass through the interior of the resin mass (thermoplastic resin), creating a physically bonded state.

(6) Fineness

A sample of the front side or back side fiber layer of the artificial leather was photographed at 10 arbitrary locations using a microscope at a magnification of 2500x, and the fiber diameters were measured at 50 points, recording the average as the mean fiber size. This was converted to fineness [dtex] of the main fiber, based on the obtained mean fiber size and the main fiber density.

The artificial leather was immersed in ethanol, encapsulated in a gelatin capsule and freeze-dried with liquid nitrogen, and then the entire capsule was cut with a knife and the cut cross-section of the sample after returning to ordinary temperature was observed using a scanning electron microscope (JSM-5610 by JEOL Corp.) under conditions of WD = 10 mm, 200x magnification, measuring the thickness of the weaving yarn forming the scrim layer at 5 points in each of 20 obtained images, to determine the fineness of the weaving yarn forming the scrim layer.

The fineness of the thermal bonding fibers is the fineness of the starting staple fibers, and it was evaluated using a scanning electron microscope. Specifically, adhesive carbon tape was attached onto the evaluation stage, 0.05 g of the starting staple fibers was placed over it, removing the excess thermal bonding fibers with an air duster, and the sample was observed under conditions of WD = 10 mm, 200x magnification, measuring the thickness at 5 points for each of the 20 obtained images.

(7) Abrasion resistance and abrasion loss

The sample surface was abraded with a pressing load of 12 kPa, by the method specified according to JIS-L-1096 E (Martindale method). As the evaluation standard in this test method, the front side layer of the sample was abraded, and the number of abrasion cycles required to produce a portion with the scrim layer exposed was evaluated on the following evaluation scale (grade).

(Evaluation scale)

PP: Significant shedding of fibers at 5000 abrasion cycles, making evaluation impossible.
P: Exposure of scrim layer at less than 30,000 abrasion cycles.
F: Exposure of scrim layer at 30,000 or more and less than 40,000 abrasion cycles.

G: Exposure of scrim layer at 40,000 or more and less than 50,000 abrasion cycles.
VG: Exposure of scrim layer at 50,000 or more abrasion cycles.

An artificial leather sample (circular with diameter of 40 mm) as specified by JIS-L-1096 E (Martindale method) was measured for change in weight [mg] before and after 50,000 cycles under a pressing load of 12 kPa, and the result was evaluated as the abrasion loss. The measurement was performed 3 times and the added average was taken as the result.

(8) Tactile sensation evaluation

The dyed artificial leather sample was cut out to 25 cm squares which were arranged on a desk with the front sides facing upwards, and 20 blindfolded subjects (10 males and 10 females, in pairs from 20-year-old to 60-year old age groups) were asked to examine the tactile sensation along the raised surface. They were simultaneously asked to conduct a similar tactile sensation test for natural suede, and the flexibility and luxurious quality of the front side was organoleptically evaluated on a scale of 5 points (with suede as 5), rounding the points to two decimal places.

G: Average of 4.0 points or more in organoleptic evaluation.
F: Average of 3.0 or more and less than 4.0 points in organoleptic evaluation.
P: Average of less than 3.0 points in organoleptic evaluation.

(9) Reduced viscosity

After adding 0.35 g of resin as a thermal bonding fiber starting material to 0.25 deciliters of 1,1,1,3,3,3-hexafluoro-2-propanol (HFIP), a magnetic stirrer was used for stirring at room temperature for 3 hours to prepare a diluted solution. The obtained diluted solution was added to an Ubbellohde viscosity tube (tube diameter: 0.03), the number of seconds the diluted solution and HFIP solvent took to fall in a water bath prepared at $25°C \pm 0.1°C$ was measured, and the relative viscosity ($\eta sp$) was calculated. After measurement, the diluted solution was weighed and dried for 1 hour in an oven at 120°C, and the polymer concentration C (g/dl) was calculated from the resulting solid content. The relative viscosity ($\eta sp$) was divided by the polymer concentration C (g/dl) to obtain the reduced viscosity $\eta sp/c$. The solvent and its amount may be changed depending on the type of resin used as the starting material for the thermal bonding fibers.

(10) TOF-SIMS measurement pretreatment

The artificial leather was cut to a width of 5 mm and a length of 10 mm to fabricate a sample sheet. It was then mixed with 9.7 ml of Quetol812 by Nisshin-EM, 3.7 ml of MNA, 6.6 ml of DDSA and 0.34 ml of DMP-30 to prepare a resin composition, and after vacuum defoaming, the sample prepared with the epoxy resin was packed into a silicon embedding plate and hardened for 3 days in a thermostatic bath at 60°C. The composite of the sample and cured epoxy resin was removed and a cryomicrotome (Ultracut-UCT by Leica) was used to prepare a cross-section of the sample.

(11) TOF-SIMS measurement

Prior to TOF-SIMS measurement, contamination on the front side of the artificial leather sample cross-section was cleaned off using a gas cluster beam (GCIB). The spectrum was extracted from the thermoplastic resin region in the measured image, and used for measurement of the ion intensity for m/z = 27 (fragment ion: $C_2H_3$), m/z = 71 (fragment ion: $C_4H_7O$) and m/z = 104 (fragment ion: $C_7H_4O$) for the positive ion and the ion intensity for m/z = 25 (fragment ion: $C_2H$) and m/z = 42 (fragment ion: CNO) for the negative ion.

Five regions identified as thermoplastic resin regions by this method were repeatedly measured in the same manner and their added average was determined to be the ion intensity, also calculating their ratio. Regions where the spectrum is not clearly apparent are excluded as being non-thermoplastic resin regions.

For identification of the thermoplastic resin regions, a cryomicrotome-prepared sample cross-section is measured using an atomic force microscope and the locations of the thermoplastic resin are determined by the shape characteristics. The epoxy resin is observed as the matrix having the widest area, while the yarn cross-sections are observed as fixed round or elliptical shapes. The thermoplastic resin component is observed as amorphous regions. In order to avoid effects from the epoxy resin or yarn during analysis of the thermoplastic resin, a location at least 2 $\mu$m from the interface between the thermoplastic resin and epoxy resin and from the interface between the thermoplastic resin and yarn is selected for analysis.

The conditions used for TOF-SIMS measurement were the following.

(Measuring conditions)

Device: nanoTOF (Ulvac-Phi, Inc.)
Analysis software: WinCadenceN (Ulvac-Phi, Inc.)
Primary ion: $Bi_3^{2+}$
Acceleration voltage: 30 kV
Ionic current ~0.1 nA (DC)
Bunching: On
Analysis area: 50 $\mu$m $\times$ 50 $\mu$m
Analysis time: 30 min
Detection ions: Positive and negative ions
Neutralization: Positive ion: electron gun + Ar monomer ion, negative ion: electron gun

(Cleaning conditions)

Sputter ion GCIB ($Ar_{2500+}$)
Acceleration voltage: 20 kV
Ionic current: 5 nA
Sputter area: 600 $\mu$m $\times$ 600 $\mu$m
Sputter time: 30 sec

(12) Flame retardance

This was measured based on US Federal Motor Vehicle Safety Standards "FMVSS No. 302". A grade was assessed in the measurement, using the following evaluation scale.

(Evaluation scale)

Grade 5: Burning part did not reach the burning speed region.
Grade 4: Burning length of less than 50 mm, and burning time within 60 seconds.
Grade 3: Average burning speed of less than 50 mm/min, with sample fully burned.
Grade 2: Average burning speed of 50 mm/min or greater and less than 100 mm/min, with sample fully burned.
Grade 1: Average burning speed of 100 mm/min or greater, with sample fully burned.

(13) Flexural modulus of thermoplastic resin (MPa)

Pellets of the thermoplastic resin were inserted into a pressing machine with a spacer and subjected to melt compression under conditions with a temperature of 20°C higher than the melting point, 10 MPa, 5 min, to mold a square sheet with side lengths of 150 mm and a thickness of 4 mm. A 80 mm $\times$ 10 mm test piece was cut out from the sheet and a universal material tester (Model 5967 by Instron) was used for a 3-point bending test by the secant method of JIS K7171 (speed: 2 mm/min, span: 64 mm), evaluating the stress gradient in a specified deflection zone (0.05-0.25%). Evaluation was carried out for 6 sample test pieces, and the average was calculated.

(Aspect 1-1)

The method for producing the resins to be used for thermal bonding fibers is described below.

[Resin Production Example 1-1-1]

Polycondensation reaction was conducted using dimethyl terephthalate, 1,4-butanediol and polytetramethylene glycol, to obtain a polyester-polyether block copolymer elastomer. The specific test conditions are specified below. After adding 1000 g of dimethyl terephthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 1210 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-1-1. The melting point of polyester-polyether block copolymer elastomer resin 1-1-1 was 182°C, and the reduced viscosity was 3.0 dl/g.

[Resin Production Example 1-1-2]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 780 g of dimethyl terephthalate, 220 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 500 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-1-2. The melting point of the polyester-polyether block copolymer elastomer resin was 170°C, and the reduced viscosity was 2.3 dl/g.

[Resin Production Example 1-1-3]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 740 g of dimethyl terephthalate, 260 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. Polytetramethylene glycol with a number-average molecular weight of 2000 was then added at 750 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-1-3. The melting point of polyester-polyether block copolymer elastomer resin 3 was 155°C, and the reduced viscosity was 3.0 dl/g.

[Resin Production Example 1-1-4]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 770 g of dimethyl terephthalate, 230 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. After adding 1120 g of polytetramethylene glycol with a number-average molecular weight of 2900, polycondensation reaction was carried out to obtain polyester-polyether block copolymer elastomer resin 4. The melting point of polyester-polyether block copolymer elastomer resin 1-1-4 was 163°C, and the reduced viscosity was 2.9 dl/g.

[Resin Production Example 1-1-5]

Polycondensation reaction of succinic anhydride and 1,4-butanediol was carried out to obtain polybutylene succinate. After adding 500 g of succinic anhydride and 500 g of 1,4-butanediol into a stirrer-equipped 2 L autoclave reactor by Sibata Scientific Technology, Ltd., with 1.3 g of tetrabutoxytitanium(IV) and 0.8 g of toluene-4-sulfonic acid as catalysts, the mixture was pressurized to 2 atmospheres with nitrogen, and reaction was carried out at 270°C for 6 hours while stirring at 50 rpm for polycondensation reaction to obtain polybutylene succinate resin 1-1-5. The obtained polybutylene succinate resin 1-1-5 had a melting point of 115°C and a reduced viscosity of 1.9 dl/g.

The method for producing the thermal bonding fibers is described above.

[Fiber Production Example 1-1-1]

Resin 1-1-1 produced in Resin Production Example 1-1-1 was dried for 8 hours with a dehumidifying dryer at a drying temperature of 80°C, after which it was melt extruded with a single-screw extruder at an extrusion temperature of 230°C, and discharged from a spinneret with 400 nozzle holes and a nozzle diameter of 0.15 mm at a spinning temperature of 250°C and a discharge throughput of 0.05 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 200 dtex/400 filament.

The obtained multifilament was bundled as a group of 50 strands to form a tow, and cut to 5 mm fiber lengths with a rotary cutter at a cutting speed of 80 m/min and a cutting tension of 0.01 g/d, to obtain thermal bonding fibers composed of resin 1-1-1 at 0.5 dtex.

[Fiber Production Example 1-1-2]

A multifilament with a fineness of 280 dtex/400 filament was obtained in the same manner as Production Example 1-1-1, except that discharge was at a discharge throughput of 0.07 g/min per hole. The obtained multifilament was cut under the same cutting conditions as Fiber Production Example 1-1-1, to obtain thermal bonding fibers composed of resin 1-1-1 at 0.7 dtex.

[Fiber Production Example 1-1-3]

A multifilament with a fineness of 440 dtex/400 filament was obtained in the same manner as Fiber Production Example 1-1-1, except that discharge was at a discharge throughput of 0.11 g/min per hole. The obtained multifilament was cut under the same cutting conditions as Production Example 1, to obtain thermal bonding fibers composed of resin 1-1-1 at 1.1 dtex.

[Fiber Production Example 1-1-4]

A multifilament with a fineness of 440 dtex/400 filament was obtained in the same manner as Fiber Production Example 1-1-1, except that discharge was at a discharge throughput of 0.11 g/min per hole. The obtained multifilament was cut under the same cutting conditions as Fiber Production Example 1 except for a fiber length of 8 mm, to obtain thermal bonding fibers composed of resin 1-1-1 at 1.1 dtex.

[Fiber Production Example 1-1-5]

A multifilament with a fineness of 720 dtex/400 filament was obtained in the same manner as Fiber Production Example 1-1-1, except that discharge was at a discharge throughput of 0.18 g/min per hole. The obtained multifilament was cut under the same cutting conditions as Fiber Production Example 1, to obtain thermal bonding fibers composed of resin 1-1-1 at 1.8 dtex.

[Fiber Production Example 1-1-6]

A multifilament with a fineness of 400 dtex/400 filament was obtained in the same manner as Fiber Production Example 1-1-1, except that resin 1-1-2 produced in Resin Production Example 1-1-2 was discharged at a discharge throughput of 0.10 g/min per hole. The obtained multifilament was cut under the same cutting conditions as Fiber Production Example 1-1-1, to obtain thermal bonding fibers composed of resin 1-1-2 at 1.0 dtex.

[Fiber Production Example 1-1-7]

A multifilament with a fineness of 400 dtex/400 filament was obtained in the same manner as Fiber Production Example 1-1-1, except that resin 1-1-3 produced in Resin Production Example 1-1-3 was used, with a discharge throughput of 0.10 g/min per hole. The obtained multifilament was cut under the same cutting conditions as Fiber Production Example 1-1-1, to obtain thermal bonding fibers composed of resin 1-1-3 at 1.0 dtex.

[Fiber Production Example 1-1-8]

A multifilament with a fineness of 400 dtex/400 filament was obtained in the same manner as Fiber Production Example 1-1-1, except that resin 1-1-3 produced in Resin Production Example 1-1-3 was used, with a discharge throughput of 0.10 g/min per hole. The obtained multifilament was cut under the same cutting conditions as Fiber Production Example 1-1-1 except for a fiber length of 3 mm, to obtain thermal bonding fibers composed of resin 1-1-3 at 1.0 dtex.

[Fiber Production Example 1-1-9]

A multifilament with a fineness of 400 dtex/400 filament was obtained in the same manner as Fiber Production Example 1-1-1, except that resin 1-1-4 produced in Resin Production Example 1-1-4 was used, with a discharge throughput of 0.10 g/min per hole. The obtained multifilament was cut under the same cutting conditions as Production Example 1-1-1, to obtain thermal bonding fibers composed of resin 1-1-4 at 1.0 dtex.

[Fiber Production Example 1-1-10]

A multifilament with a fineness of 560 dtex/400 filament was obtained in the same manner as Fiber Production Example 1-1-1, except that resin 1-1-4 produced in Resin Production Example 1-1-4 was used, with a discharge throughput of 0.14 g/min per hole. The obtained multifilament was cut under the same cutting conditions as Fiber Production Example 1-1-1 except for a fiber length of 7 mm, to obtain thermal bonding fibers composed of resin 1-1-4 at 1.4 dtex.

[Fiber Production Example 1-1-11]

A polyamide copolymer (PEBAX 4033SP-01 by Arkema) was dried for 8 hours with a dehumidifying dryer at a drying temperature of 80°C, after which it was melt extruded with a single-screw extruder at an extrusion temperature of 250°C, and discharged from a spinneret with 400 nozzle holes and a nozzle diameter of 0.15 mm at a spinning temperature of 260°C and a discharge throughput of 0.10 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 400 dtex/400 filament. The obtained multifilament was cut under the same cutting conditions as Fiber Production Example 1-1-1, to obtain thermal bonding fibers composed of PEBAX 4033SP-01 at 1.0 dtex.

[Fiber Production Example 1-1-12]

A multifilament with a fineness of 400 dtex/400 filament was obtained in the same manner as Fiber Production Example 1-1-11, except for using a polyamide copolymer (PEBAX 6333SP-01 by Arkema). The obtained multifilament was cut under the same cutting conditions as Fiber Production Example 1-1-1, to obtain thermal bonding fibers composed of PEBAX 6333SP-01 at 1.0 dtex.

[Fiber Production Example 1-1-13]

A multifilament with a fineness of 2000 dtex/400 filament was obtained in the same manner as Fiber Production Example 1-1-1, except that discharge was at a discharge throughput of 0.50 g/min per hole, and cooling of the filament was by contact of cooling air at a wind speed of 0.9 m/s. The obtained multifilament was cut under the same cutting conditions as Fiber Production Example 1-1-1, to obtain thermal bonding fibers composed of resin 1-1-1 at 5.0 dtex.

[Fiber Production Example 1-1-14]

Resin 1-1-5 produced in Resin Production Example 1-1-5 was dried for 8 hours with a dehumidifying dryer at a drying temperature of 60°C, after which it was melt extruded with a single-screw extruder at an extrusion temperature of 180°C, and discharged from a spinneret with 400 nozzle holes and a nozzle diameter of 0.15 mm at a spinning temperature of 190°C and a discharge throughput of 0.10 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 400 dtex/400 filament. The obtained multifilament was cut under the same cutting conditions as Fiber Production Example 1-1-1, to obtain thermal bonding fibers composed of polybutylene succinate at 1.0 dtex.

[Example 1-1-1]

Polyethylene terephthalate fibers with a single fiber fineness of 0.15 dtex and a melting point of 255°C were produced by a direct spinning method, and cut to lengths of 5 mm as main fibers. The thermal bonding fibers used were the fibers produced in Fiber Production Example 1-1-1 (resin 1, fineness: 0.5 dtex, fiber length: 5 mm). The staple fibers were dispersed in water to a weight ratio of main fiber: thermal bonding fiber = 90: 10 to prepare a slurry. A sheet for front side fibers with a basis weight of 130 g/m$^2$ was formed from the slurry by a sheet forming method. Polyethylene terephthalate fibers with a single fiber fineness of 0.15 dtex and a melting point of 255°C were also produced by a direct spinning method, and cut to lengths of 5 mm as main fibers. The staple fibers were dispersed in water to a weight ratio of main fiber: thermal bonding fiber = 97:3 to prepare a slurry. A sheet for back side fibers with a basis weight of 50 g/mwas formed from the slurry by a sheet forming method. The two layers were layered together with a woven scrim having a basis weight of 100 g/m$^2$ and composed of 166 dtex/48f polyethylene terephthalate fibers, with the sum of the woven density in the MD and the woven density in the CD being 120 (per 2.54 cm), to form the three-layer structure: front side fiber layer/scrim layer/back side fiber layer. The obtained three-layered body was sprayed with a high-speed water stream using a straight-flow injection nozzle for intertwining entanglement, after which it was dried for 5 minutes at 130°C using an air-through dryer, to obtain a nonwoven fabric with a three-layer structure.

The surface of the front side fiber layer of the obtained nonwoven fabric was subjected to raising treatment by buffing with 400 mesh sandpaper, and then the nonwoven fabric was slackened to a shrinkage factor of 5% in both the MD and CD using an X4HDHT biaxial stretching test apparatus by Toyo Seiki Seisakusho, Ltd., anchoring at the centers on both sides and at the four corners with a compressed air grip. The nonwoven fabric was then subjected to thermal annealing treatment in a chamber at 190°C for 5 minutes to obtain a nonwoven fabric for artificial leather. A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 1.

[Example 1-1-2]

Suede-like artificial leather 2 was obtained in the same manner as Example 1-1-1, except that the fibers produced in Fiber Production Example 1-1-2 (resin 1-1-1, fineness: 0.7 dtex, fiber length: 5 mm) were used as the thermal bonding fibers. The production conditions and evaluation results for the obtained artificial leather are shown in Table 1.

[Example 1-1-3]

Suede-like artificial leather 3 was obtained in the same manner as Example 1-1-1, except that the thermal bonding fibers were changed to the fibers produced in Fiber Production Example 1-1-3 (resin 1-1-1, fineness: 1.1 dtex, fiber length: 5 mm), and the basis weight of the sheet for front side fibers was 140 g/m$^2$. The production conditions and evaluation results for the obtained artificial leather are shown in Table 1.

[Example 1-1-4]

Suede-like artificial leather 4 was obtained in the same manner as Example 1-1-1, except that the thermal bonding fibers were changed to the fibers produced in Fiber Production Example 1-1-4 (resin 1-1-1, fineness: 1.1 dtex, fiber length: 8 mm), the weight ratio of the sheet for front side fibers was main fiber: thermal bonding fiber = 94:6, and the basis weight of the sheet for front side fibers was 130 g/m$^2$. The production conditions and evaluation results for the obtained artificial leather are shown in Table 1.

[Example 1-1-5]

Suede-like artificial leather 5 was obtained in the same manner as Example 1-1-1, except that the thermal bonding fibers used were the fibers produced in Fiber Production Example 1-1-5 (resin 1-1-1, fineness: 1.8 dtex, fiber length: 5 mm), and the basis weight of the sheet for front side fibers was 90 g/m$^2$. The production conditions and evaluation results for the obtained artificial leather are shown in Table 1.

[Example 1-1-6]

Suede-like artificial leather 6 was obtained in the same manner as Example 1-1-1, except that the thermal bonding fibers used were the fibers produced in Fiber Production Example 1-1-6 (resin 1-1-2, fineness: 1.0 dtex, fiber length: 5 mm), the basis weight of the sheet for front side fibers was 110 g/m$^2$, the weight ratio of the front side fiber layer was main fiber: thermal bonding fiber = 92:8, and the thermal annealing temperature was 180°C. The production conditions and evaluation results for the obtained artificial leather are shown in Table 1.

[Example 1-1-7]

Suede-like artificial leather 7 was obtained in the same manner as Example 1-1-1, except that the thermal bonding fibers used were the fibers produced in Fiber Production Example 1-1-7 (resin 1-1-3, fineness: 1.0 dtex, fiber length: 5 mm), the basis weight of the sheet for front side fibers was 110 g/m², and the thermal annealing temperature was 165°C. The production conditions and evaluation results for the obtained artificial leather are shown in Table 1.

[Example 1-1-8]

Suede-like artificial leather 8 was obtained in the same manner as Example 1-1-1, except that the thermal bonding fibers used were the fibers produced in Fiber Production Example 1-1-8 (resin 1-1-3, fineness: 1.0 dtex, fiber length: 3 mm), the weight ratio of the front side fiber layer was main fiber:thermal bonding fiber = 91:9, and the thermal annealing temperature was 165°C. The production conditions and evaluation results for the obtained artificial leather are shown in Table 1.

[Example 1-1-9]

Suede-like artificial leather 9 was obtained in the same manner as Example 1-1-1, except that the thermal bonding fibers used were the fibers produced in Fiber Production Example 1-1-9 (resin 1-1-4, fineness: 1.0 dtex, fiber length: 5 mm), the weight ratio of the front side fiber layer was main fiber:thermal bonding fiber = 95:5, and the thermal annealing temperature was 170°C. The production conditions and evaluation results for the obtained artificial leather are shown in Table 1.

[Example 1-1-10]

Suede-like artificial leather 9 was obtained in the same manner as Example 1-1-1, except that the thermal bonding fibers used were the fibers produced in Fiber Production Example 1-1-10 (resin 1-1-4, fineness: 1.4 dtex, fiber length: 7 mm), the basis weight of the sheet for front side fibers was 110 g/m², the weight ratio of the front side fiber layer was main fiber thermal bonding fiber = 93:7, and the thermal annealing temperature was 170°C. The production conditions and evaluation results for the obtained artificial leather are shown in Table 1.

[Example 1-1-11]

Suede-like artificial leather 11 was obtained in the same manner as Example 1-1-1, except that the thermal bonding fibers used were the fibers produced in Fiber Production Example 1-1-11 (PEBAX 4033 SP01, fineness: 1.0 dtex, fiber length: 5 mm), and the thermal annealing temperature was 170°C. The production conditions and evaluation results for the obtained artificial leather are shown in Table 1.

[Example 1-1-12]

Suede-like artificial leather 12 was obtained in the same manner as Example 1-1-1, except that the thermal bonding fibers used were the fibers produced in Fiber Production Example 1-1-11 (PEBAX 4033SP01, fineness: 1.0 dtex, fiber length: 5 mm), the basis weight of the sheet for front side fibers was 110 g/m², the weight ratio of the front side fiber layer was main fiber:thermal bonding fiber = 93:7, and the thermal annealing temperature was 170°C. The production conditions and evaluation results for the obtained artificial leather are shown in Table 1.

[Comparative Example 1-1-1]

Suede-like artificial leather 13 was obtained in the same manner as Example 1-1-1, except that the thermal bonding fibers were Fully-meltable CASVEN 8000 thermal bonding stable fibers (Unitika, Ltd.) having a single fiber fineness of 0.7 dtex and lengths of 5 mm. The production conditions and evaluation results for the obtained artificial leather are shown in Table 1.

[Comparative Example 1-1-2]

Suede-like artificial leather 14 was obtained in the same manner as Example 1-1-1, except that the thermal bonding fibers were Fully-meltable CASVEN 8000 thermal bonding stable fibers (Unitika, Ltd.) having a single fiber fineness of 1.1 dtex and lengths of 5 mm, and the basis weight of the sheet for front side fibers was 110 g/m². The production conditions and evaluation results for the obtained artificial leather are shown in Table 1.

[Comparative Example 1-1-3]

Suede-like artificial leather 15 was obtained in the same manner as Example 1-1-1, except that the thermal bonding fibers used were the fibers produced in Fiber Production Example 12 (PEBAX 6333SP-01, fineness: 1.1 dtex, fiber length: 5 mm), the basis weight of the sheet for front side fibers was 110 g/m$^2$, and the thermal annealing temperature was 180°C. The production conditions and evaluation results for the obtained artificial leather are shown in Table 1.

[Comparative Example 1-1-4]

Suede-like artificial leather 16 was obtained in the same manner as Example 1-1-3, except that the basis weight of the sheet for front side fibers was 140 g/m$^2$, and the thermal annealing temperature was 130°C. The production conditions and evaluation results for the obtained artificial leather are shown in Table 1.

[Comparative Example 1-1-5]

Suede-like artificial leather 17 was obtained in the same manner as Example 1-1-1, except that the thermal bonding fibers used were the fibers produced in Fiber Production Example 1-1-13 (resin 1-1-1, fineness: 5.0 dtex, fiber length: 5 mm), and the basis weight of the sheet for front side fibers was 140 g/m$^2$. The production conditions and evaluation results for the obtained artificial leather are shown in Table 1.

[Comparative Example 1-1-6]

Suede-like artificial leather 18 was obtained in the same manner as Example 1-1-1, except that the thermal bonding fibers used were the fibers produced in Fiber Production Example 1-1-14 (polybutylene succinate, fineness: 1.0 dtex, fiber length: 5 mm), and the thermal annealing temperature was 130°C. The production conditions and evaluation results for the obtained artificial leather are shown in Table 1. No bonding points were detected in the artificial leather.

[Table 1]

| | Main fibers | | Basis weight | Thermoplastic resin (thermal bonding fibers) | | | | | Mixing ratio | | Thermal annealing tempera-ture | Deforma-tion of ther-moplastic resin | Numbe r aver-age volume | Volume number density of thermo-plastic res-in | Long axis/ short axis ratio of thermo-plasti c resin | Abra-sion re-sistance | Abra-sio n loss | Tactile sensation evaluation |
| | Fineness | Fiber length | | Starting resin for thermal bonding fibers | Resin chemical composi-tion | Melt-ing point | Fine-ness | Fiber length | Front side fiber layer | Back side fiber layer | | | | | | | | |
| Units | dtex | mm | g/m² | | | °C | dtex | mm | % | % | °C | nm | μm³ | 10¹²/m³ | - | MD | mg | Orga-noleptic evaluation |
| Exam-ple 1-1-1 | 0.15 | 5 | 280 | Resin 1-1-1 | Esteric elastomer | 182 | 0.5 | 5 | 10 | 3 | 190 | 74 | 3600 | 4.2 | 1.9 | VG | 14 | G |
| Example 1-1-2 | 0.15 | 5 | 280 | Resin 1-1-1 | Esteric elastomer | 182 | 0.7 | 5 | 10 | 3 | 190 | 76 | 5800 | 2.4 | 2.2 | VG | 14 | G |
| Example 1-1-3 | 0.15 | 5 | 290 | Resin 1-1-1 | Esteric elastomer | 182 | 1.1 | 5 | 10 | 3 | 190 | 73 | 10,800 | 1.8 | 2.6 | VG | 16 | G |
| Example 1-1-4 | 0.15 | 5 | 280 | Resin 1-1-1 | Esteric elastomer | 182 | 1.1 | 8 | 6 | 3 | 190 | 79 | 15,800 | 0.7 | 4.3 | VG | 19 | G |
| Example 1-1-5 | 0.15 | 5 | 240 | Resin 1-1-1 | Esteric elastomer | 182 | 1.8 | 5 | 10 | 3 | 190 | 71 | 22,000 | 0.8 | 3.4 | VG | 18 | G |
| Example 1-1-6 | 0.15 | 5 | 260 | Resin 1-1-2 | Esteric elastomer | 170 | 1.0 | 5 | 8 | 3 | 180 | 33 | 10,300 | 1.4 | 2.7 | G | 21 | G |
| Example 1-1-7 | 0.15 | 5 | 260 | Resin 1-1-3 | Esteric elastomer | 155 | 1.0 | 5 | 10 | 3 | 165 | 102 | 12,800 | 1.5 | 2.6 | VG | 15 | G |
| Example 1-1-8 | 0.15 | 5 | 280 | Resin 1-1-3 | Esteric elastomer | 155 | 1.0 | 3 | 9 | 3 | 165 | 98 | 8600 | 2.0 | 2.2 | VG | 16 | G |

(continued)

| | Main fibers | | Basis weight | Thermoplastic resin (thermal bonding fibers) | | | | | Mixing ratio | | Thermal annealing tempera-ture | Deforma-tion of ther-moplastic resin | Numbe r aver-age volume | Volume number density of thermo-plastic res-in | Long axis/ short axis ratio of thermo-plasti c resin | Abra-sion re-sistance | Abra-sio n loss | Tactile sensation evaluation |
| | Fineness | Fiber length | | Starting resin for thermal bonding fibers | Resin chemical composi-tion | Melt-ing point | Fine-ness | Fiber length | Front side fiber layer | Back side fiber layer | | | | | | | | |
| Units | dtex | mm | g/m² | | | °C | dtex | mm | % | % | °C | nm | μm³ | 10¹²/m³ | - | MD | mg | Orga-noleptic evaluation |
| Exam-ple 1-1-9 | 0.15 | 5 | 280 | Resin 1-1-4 | Esteric elastomer | 163 | 1.0 | 5 | 7 | 3 | 170 | 107 | 12,400 | 1.0 | 3.0 | VG | 19 | G |
| Example 1-1-10 | 0.15 | 5 | 260 | Resin 1-1-4 | Esteric elastomer | 163 | 1.4 | 7 | 5 | 3 | 170 | 106 | 16,700 | 0.5 | 3.8 | G | 22 | G |
| Example 1-1-11 | 0.15 | 5 | 280 | PEBAX 4033SP0 1 | Nylon elastomer | 160 | 1.0 | 5 | 10 | 3 | 170 | 76 | 11,100 | 1.7 | 2.6 | VG | 18 | G |
| Example 1-1-12 | 0.15 | 5 | 260 | PEBAX 4033SP0 1 | Nylon elastomer | 160 | 1.0 | 5 | 7 | 3 | 170 | 74 | 10,600 | 1.1 | 2.4 | VG | 19 | G |
| Comp. Ex. 1-1-1 | 0.15 | 5 | 280 | CASVEN 8000 | Esteric resin | 178 | 0.7 | 5 | 10 | 3 | 190 | 9 | 6200 | 2.5 | 2.3 | VG | 17 | F |
| Comp. Ex. 1-1-2 | 0.15 | 5 | 260 | CASVEN 8000 | Esteric resin | 178 | 1.1 | 5 | 10 | 3 | 190 | 11 | 11,100 | 1.7 | 2.5 | VG | 16 | F |
| Comp. Ex. 1-1-3 | 0.15 | 5 | 260 | PEBAX 6333SP0 1 | Nylon elastomer | 169 | 1.1 | 5 | 10 | 3 | 180 | 16 | 9900 | 1.8 | 2.9 | VG | 18 | P |

(continued)

| | Main fibers | | Basis weight | Thermoplastic resin (thermal bonding fibers) | | | | | Mixing ratio | | Thermal annealing temperature | Deformation of thermoplastic resin | Number average volume | Volume number density of thermoplastic resin | Long axis/short axis ratio of thermoplastic resin | Abrasion resistance | Abrasion loss | Tactile sensation evaluation |
| | Fineness | Fiber length | | Starting resin for thermal bonding fibers | Resin chemical composition | Melting point | Fineness | Fiber length | Front side fiber layer | Back side fiber layer | | | | | | | | |
| Units | dtex | mm | g/m² | | | °C | dtex | mm | % | % | °C | nm | μm³ | 10¹²/m³ | - | MD | mg | Organoleptic evaluation |
| Comp. Ex. 1-1-4 | 0.15 | 5 | 290 | Resin 1-1-1 | Esteric elastomer | 182 | 1.1 | 5 | 10 | 3 | 130 | 70 | 485,000 | 0.03 | 256 | P | - | F |
| Comp. Ex. 1-1-5 | 0.15 | 5 | 290 | Resin 1-1-1 | Esteric elastomer | 182 | 5.0 | 5 | 10 | 3 | 190 | 73 | 54,400 | 0.3 | 3.9 | F | 25 | P |
| Comp. Ex. 1-1-6 | 0.15 | 5 | 280 | Resin 1-1-5 | Esteric resin | 115 | 1.0 | 5 | 10 | 3 | 130 | - | - | - | - | P | - | G |

(Aspect 1-2)

Methods for producing the resins as starting materials for the thermal bonding resin will now be described.

[Resin Production Example 1-2-1]

Polycondensation reaction was conducted using dimethyl terephthalate, 1,4-butanediol and polytetramethylene glycol, to obtain a polyester-polyether block copolymer elastomer. The specific test conditions are specified below. After adding 1000 g of dimethyl terephthalate, 650 g of 1,4-butanediol and 8.3 g of tetrabutoxytitanium(IV) into a pressurized stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 1210 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1. The melting point of polyester-polyether block copolymer elastomer resin 1-2-1 was 182°C, and the reduced viscosity was 3.0 dl/g.

[Resin Production Example 1-2-2]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 780 g of dimethyl terephthalate, 220 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 500 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-2-2. The melting point of the polyester-polyether block copolymer elastomer resin was 170°C, and the reduced viscosity was 2.3 dl/g.

[Resin Production Example 1-2-3]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 740 g of dimethyl terephthalate, 260 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. Polytetramethylene glycol with a number-average molecular weight of 2000 was then added at 750 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-2-3. The melting point of polyester-polyether block copolymer elastomer resin 1-2-3 was 155°C, and the reduced viscosity was 3.0 dl/g.

[Resin Production Example 1-2-4]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 770 g of dimethyl terephthalate, 230 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. After adding 1120 g of polytetramethylene glycol with a number-average molecular weight of 2900, polycondensation reaction was carried out to obtain polyester-polyether block copolymer elastomer resin 1-2-4. The melting point of polyester-polyether block copolymer elastomer resin 1-2-4 was 163°C, and the reduced viscosity was 2.9 dl/g.

[Resin Production Example 1-2-5]

Polycondensation reaction was conducted using dimethyl terephthalate, 1,4-butanediol and polytetramethylene glycol, to obtain a polyester-polyether block copolymer elastomer. The specific test conditions are specified below. After adding 1000 g of dimethyl terephthalate, 650 g of 1,4-butanediol and 8.3 g of tetrabutoxytitanium(IV) into a pressurized stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. After adding 2400 g of polytetramethylene glycol with a number-average molecular weight of 2000, polycondensation reaction was carried out to obtain polyester-polyether block copolymer elastomer resin 1-2-5. The melting point of polyester-polyether block copolymer elastomer resin 1-2-5 was 160°C, and the reduced viscosity was 4.4 dl/g.

[Resin Production Example 1-2-6]

Polycondensation reaction was conducted using dimethyl terephthalate, dimethyl isophthalate, ethylene glycol and polytetramethylene glycol, to obtain a polyester-polyether block copolymer elastomer. The specific test conditions are specified below. After adding 800 g of dimethyl terephthalate, 200 g of dimethyl isophthalate, 450 g of ethylene glycol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. After adding 780 g of polytetramethylene glycol with a number-average molecular weight of 2900, polycondensation reaction was carried out to obtain polyester-polyether block copolymer elastomer resin 1-2-6. The melting point of polyester-polyether block copolymer elastomer resin 1-2-6 was 171°C, and the reduced viscosity was 3.1 dl/g.

[Resin Production Example 1-2-7]

Polycondensation reaction of succinic anhydride and 1,4-butanediol was carried out to obtain polybutylene succinate. After adding 500 g of succinic anhydride and 500 g of 1,4-butanediol into a stirrer-equipped 2 L autoclave reactor by Sibata Scientific Technology, Ltd., with 1.3 g of tetrabutoxytitanium(IV) and 0.8 g of toluene-4-sulfonic acid as catalysts, the mixture was pressurized to 2 atmospheres with nitrogen, and reaction was carried out at 270°C for 6 hours while stirring at 50 rpm for polycondensation reaction to obtain polybutylene succinate resin 1-2-7. The obtained polybutylene succinate resin 1-2-7 had a melting point of 115°C and a reduced viscosity of 1.9 dl/g.
The method for producing the thermal bonding fibers is described above.

[Fiber Production Example 1-2-1]

Resin 1-2-1 was dried for 8 hours with a dehumidifying dryer at a drying temperature of 80°C, after which it was melt extruded with a single-screw extruder at an extrusion temperature of 230°C, and discharged from a spinneret with 400 nozzle holes and a nozzle diameter of 0.15 mm at a spinning temperature of 250°C and a discharge throughput of 0.05 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 200 dtex/400 filament.
The obtained multifilament was bundled as a group of 50 strands to form a tow, and cut to 5 mm fiber lengths with a rotary cutter at a cutting speed of 80 m/min and a cutting tension of 0.01 g/d, to obtain thermal bonding fibers composed of resin 1-2-1 at 0.5 dtex.

[Fiber Production Example 1-2-2]

A multifilament with a fineness of 280 dtex/400 filament was obtained in the same manner as Fiber Production Example 1-2-1, except that discharge was at a discharge throughput of 0.07 g/min per hole. The obtained multifilament was cut under the same cutting conditions as Fiber Production Example 1-2-1, to obtain thermal bonding fibers composed of resin 1-2-1 at 0.7 dtex.

[Fiber Production Example 1-2-3]

A multifilament with a fineness of 440 dtex/400 filament was obtained in the same manner as Fiber Production Example 1-2-1, except that discharge was at a discharge throughput of 0.11 g/min per hole. The obtained multifilament was cut under the same cutting conditions as Fiber Production Example 1-2-1, to obtain thermal bonding fibers composed of resin 1-2-1 at 1.1 dtex.

[Fiber Production Example 1-2-4]

A multifilament with a fineness of 440 dtex/400 filament was obtained in the same manner as Fiber Production Example 1-2-1, except that discharge was at a discharge throughput of 0.11 g/min per hole. The obtained multifilament was cut under the same cutting conditions as Fiber Production Example 1-2-1 except for a fiber length of 8 mm, to obtain thermal bonding fibers composed of resin 1-2-1 at 1.1 dtex.

[Fiber Production Example 1-2-5]

A multifilament with a fineness of 720 dtex/400 filament was obtained in the same manner as Fiber Production Example 1-2-1, except that discharge was at a discharge throughput of 0.18 g/min per hole. The obtained multifilament was cut under the same cutting conditions as Fiber Production Example 1-2-1, to obtain thermal bonding fibers composed of resin 1-2-1 at 1.8 dtex.

[Fiber Production Example 1-2-6]

A multifilament with a fineness of 400 dtex/400 filament was obtained in the same manner as Fiber Production Example 1-2-1, except that resin 1-2-2 synthesized in Resin Production Example 1-2-2 was used, with a discharge throughput of 0.10 g/min per hole. The obtained multifilament was cut under the same cutting conditions as Fiber Production Example 1-2-1, to obtain thermal bonding fibers composed of resin 1-2-2 at 1.0 dtex.

[Fiber Production Example 1-2-7]

A multifilament with a fineness of 400 dtex/400 filament was obtained in the same manner as Fiber Production Example 1-2-1, except that resin 1-2-3 synthesized in Resin Production Example 1-2-3 was used, with a discharge throughput of 0.10 g/min per hole. The obtained multifilament was cut under the same cutting conditions as Fiber Production Example 1-2-1, to obtain thermal bonding fibers composed of resin 1-2-3 at 1.0 dtex.

[Fiber Production Example 1-2-8]

A multifilament with a fineness of 400 dtex/400 filament was obtained in the same manner as Fiber Production Example 1-2-1, except that the resin used was resin 1-2-3, with a discharge throughput of 0.10 g/min per hole. The obtained multifilament was cut under the same cutting conditions as Fiber Production Example 1-2-1 except for a fiber length of 3 mm, to obtain thermal bonding fibers composed of resin 1-2-3 at 1.0 dtex.

[Fiber Production Example 1-2-9]

A multifilament with a fineness of 400 dtex/400 filament was obtained in the same manner as Fiber Production Example 1-2-1, except that the resin used was resin 1-2-4, with a discharge throughput of 0.10 g/min per hole. The obtained multifilament was cut under the same cutting conditions as Fiber Production Example 1-2-1, to obtain thermal bonding fibers composed of resin 1-2-4 at 1.0 dtex.

[Fiber Production Example 1-2-10]

A multifilament with a fineness of 560 dtex/400 filament was obtained in the same manner as Fiber Production Example 1-2-1, except that the resin used was resin 1-2-4, with a discharge throughput of 0.14 g/min per hole. The obtained multifilament was cut under the same cutting conditions as Fiber Production Example 1-2-1 except for a fiber length of 7 mm, to obtain thermal bonding fibers composed of resin 1-2-4 at 1.4 dtex.

[Fiber Production Example 1-2-11]

A multifilament with a fineness of 400 dtex/400 filament was obtained in the same manner as Fiber Production Example 1-2-1, except that the resin used was resin 1-2-5, with a discharge throughput of 0.10 g/min per hole. The obtained multifilament was cut under the same cutting conditions as Fiber Production Example 1-2-1, to obtain thermal bonding fibers composed of resin 1-2-5 at 1.0 dtex.

[Fiber Production Example 1-2-12]

The resin synthesized in Resin Production Example 1-2-6 was dried for 8 hours with a dehumidifying dryer at a drying temperature of 80°C, after which it was melt extruded with a single-screw extruder at an extrusion temperature of 250°C, and discharged from a spinneret with 400 nozzle holes and a nozzle diameter of 0.15 mm at a spinning temperature of 260°C and a discharge throughput of 0.10 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 400 dtex/400 filament. The obtained multifilament was cut under the same cutting conditions as Fiber Production Example 1-2-1, to obtain thermal bonding fibers composed of resin 1-2-6 at 1.0 dtex.

[Fiber Production Example 1-2-13]

A multifilament with a fineness of 2000 dtex/400 filament was obtained in the same manner as Fiber Production Example 1-2-1, except that discharge was at a discharge throughput of 0.50 g/min per hole, and cooling of the filament was by contact of cooling air at a wind speed of 0.9 m/s. The obtained multifilament was cut under the same cutting conditions as Fiber Production Example 1-2-1, to obtain thermal bonding fibers composed of resin 1-2-1 at 5.0 dtex.

[Fiber Production Example 1-2-14]

Using resin 1-2-7 as the resin, it was dried for 8 hours with a dehumidifying dryer at a drying temperature of 60°C, after which it was melt extruded with a single-screw extruder at an extrusion temperature of 180°C, and discharged from a spinneret with 400 nozzle holes and a nozzle diameter of 0.15 mm at a spinning temperature of 190°C and a discharge throughput of 0.10 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 400 dtex/400 filament. The obtained multifilament was cut under the same cutting conditions as Fiber Production Example 1-2-1, to obtain thermal bonding fibers composed of polybutylene succinate at 1.0 dtex.

[Example 1-2-1]

Polyethylene terephthalate fibers with a single fiber fineness of 0.15 dtex and a melting point of 255°C were produced by a direct spinning method, and cut to lengths of 5 mm as main fibers. The thermal bonding fibers used were the fibers produced in Fiber Production Example 1-2-1 (staple fiber fineness: 0.5 dtex, fiber length: 5 mm). The staple fibers were dispersed in water to a weight ratio of main fiber:thermal bonding fiber = 90: 10 to prepare a slurry. A sheet for front side fibers with a basis weight of 130 g/m² was formed from the slurry by a sheet forming method. Polyethylene terephthalate fibers with a single fiber fineness of 0.15 dtex and a melting point of 255°C were also produced by a direct spinning method, and cut to lengths of 5 mm as main fibers. The staple fibers were dispersed in water to a weight ratio of main fiber:thermal bonding fiber = 97:3 to prepare a slurry. A sheet for back side fibers with a basis weight of 50 g/m was formed from the slurry by a sheet forming method. The two layers were layered together with a woven scrim having a basis weight of 100 g/m² and composed of 166 dtex/48f polyethylene terephthalate fibers, with the sum of the woven density in the MD and the woven density in the CD being 120 (per 2.54 cm), to form the three-layer structure: front side fiber layer/scrim layer/back side fiber layer. The obtained three-layered body was sprayed with a high-speed water stream using a straight-flow injection nozzle for intertwining entanglement, after which it was dried for 5 minutes at 130°C using an air-through dryer, to obtain a nonwoven fabric with a three-layer structure.

The surface of the front side fiber layer of the obtained nonwoven fabric was subjected to raising treatment by buffing with 400 mesh sandpaper, and then the nonwoven fabric was slackened to a shrinkage factor of 5% in both the MD and CD using an X4HDHT biaxial stretching test apparatus by Toyo Seiki Seisakusho, Ltd., anchoring at the centers on both sides and at the four corners with a compressed air grip. The nonwoven fabric was then subjected to thermal annealing treatment in a chamber at 190°C for 5 minutes to obtain a nonwoven fabric for artificial leather. A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 2.

[Example 1-2-2]

Suede-like artificial leather 2 was obtained in the same manner as Example 1-2-1, except that the fibers produced in Fiber Production Example 1-2-2 (staple fiber fineness: 0.7 dtex, fiber length: 5 mm) were used as the thermal bonding fibers. The production conditions and evaluation results for the obtained artificial leather are shown in Table 2.

[Example 1-2-3]

Suede-like artificial leather 3 was obtained in the same manner as Example 1-2-1, except that the thermal bonding fibers were changed to the fibers produced in Fiber Production Example 1-2-3, and the basis weight of the sheet for front side fibers was 140 g/m². The production conditions and evaluation results for the obtained artificial leather are shown in Table 2.

[Example 1-2-4]

Suede-like artificial leather 4 was obtained in the same manner as Example 1-2-1, except that the thermal bonding fibers were changed to the fibers produced in Fiber Production Example 1-2-4, the weight ratio of the sheet for front side fibers was main fiber:thermal bonding fiber = 94:6, and the basis weight of the sheet for front side fibers was 130 g/m². The production conditions and evaluation results for the obtained artificial leather are shown in Table 2.

[Example 1-2-5]

Suede-like artificial leather 5 was obtained in the same manner as Example 1-2-1, except that the fibers produced in Fiber Production Example 1-2-5 were used as the thermal bonding fibers. The production conditions and evaluation results for the obtained artificial leather are shown in Table 2.

[Example 1-2-6]

Suede-like artificial leather 6 was obtained in the same manner as Example 1-2-1, except that the thermal bonding fibers used were the fibers produced in Fiber Production Example 1-2-6, the basis weight of the sheet for front side fibers was 120 g/m², the weight ratio of the front side fiber layer was main fiber:thermal bonding fiber = 92:8, and the thermal annealing temperature was 180°C. The production conditions and evaluation results for the obtained artificial leather are shown in Table 2.

[Example 1-2-7]

Suede-like artificial leather 7 was obtained in the same manner as Example 1-2-1, except that the thermal bonding fibers used were the fibers produced in Fiber Production Example 1-2-7, the basis weight of the sheet for front side fibers was 110 g/m², and the thermal annealing temperature was 165°C. The production conditions and evaluation results for the obtained artificial leather are shown in Table 2.

[Example 1-2-8]

Suede-like artificial leather 8 was obtained in the same manner as Example 1-2-1, except that the thermal bonding fibers used were the fibers produced in Fiber Production Example 1-2-8, the weight ratio of the front side fiber layer was main fiber:thermal bonding fiber = 91:9, and the thermal annealing temperature was 165°C. The production conditions and evaluation results for the obtained artificial leather are shown in Table 2.

[Example 1-2-9]

Suede-like artificial leather 9 was obtained in the same manner as Example 1-2-1, except that the thermal bonding fibers used were the fibers produced in Fiber Production Example 1-2-9, the weight ratio of the front side fiber layer was main fiber:thermal bonding fiber = 93:7, and the thermal annealing temperature was 170°C. The production conditions and evaluation results for the obtained artificial leather are shown in Table 2.

[Example 1-2-10]

Suede-like artificial leather 10 was obtained in the same manner as Example 1-2-1, except that the thermal bonding fibers used were the fibers produced in Fiber Production Example 1-2-10, the basis weight of the sheet for front side fibers was 110 g/m², the weight ratio of the front side fiber layer was main fiber:thermal bonding fiber = 95:5, and the thermal annealing temperature was 170°C. The production conditions and evaluation results for the obtained artificial leather are shown in Table 2.

[Example 1-2-11]

Suede-like artificial leather 11 was obtained in the same manner as Example 1-2-1, except that the fibers produced in Fiber Production Example 1-2-11 were used as the thermal bonding fibers, and the thermal annealing temperature was 170°C. The production conditions and evaluation results for the obtained artificial leather are shown in Table 2.

[Example 1-2-12]

Suede-like artificial leather 12 was obtained in the same manner as Example 1-2-1, except that the fibers produced in Fiber Production Example 1-2-12 were used as the thermal bonding fibers, and the thermal annealing temperature was 180°C. The production conditions and evaluation results for the obtained artificial leather are shown in Table 2.

[Comparative Example 1-2-1]

Suede-like artificial leather 13 was obtained in the same manner as Example 1-2-1, except that the thermal bonding fibers were Fully-meltable CASVEN 8000 thermal bonding stable fibers (Unitika, Ltd.) having a single fiber fineness of 0.7 dtex and lengths of 5 mm. The production conditions and evaluation results for the obtained artificial leather are shown in Table 2.

[Comparative Example 1-2-2]

Suede-like artificial leather 14 was obtained in the same manner as Example 1-2-1, except that the thermal bonding fibers were Fully-meltable CASVEN 8000 thermal bonding stable fibers (Unitika, Ltd.) having a single fiber fineness of 1.1 dtex and lengths of 5 mm, and the basis weight of the sheet for front side fibers was 110 g/m$^2$. The production conditions and evaluation results for the obtained artificial leather are shown in Table 2.

[Comparative Example 1-2-3]

Suede-like artificial leather 15 was obtained in the same manner as Example 1-2-3, except that the basis weight of the sheet for front side fibers was 140 g/m$^2$, and the thermal annealing temperature was 130°C. The production conditions and evaluation results for the obtained artificial leather are shown in Table 2.

[Comparative Example 1-2-4]

Suede-like artificial leather 16 was obtained in the same manner as Example 1-2-1, except that the thermal bonding fibers used were the fibers produced in Fiber Production Example 1-2-13, and the basis weight of the sheet for front side fibers was 140 g/m$^2$. The production conditions and evaluation results for the obtained artificial leather are shown in Table 2.

[Comparative Example 1-2-5]

Suede-like artificial leather 17 was obtained in the same manner as Example 1-2-1, except that the fibers produced in Fiber Production Example 1-2-14 were used as the thermal bonding fibers, and the thermal annealing temperature was 130°C. The production conditions and evaluation results for the obtained artificial leather are shown in Table 2. No bonding points were detected in the artificial leather.

[Comparative Example 1-2-6]

Polyethylene terephthalate fibers with a single fiber fineness of 0.15 dtex and a melting point of 255°C were produced by a direct melt spinning method, and cut to lengths of 5 mm as main fibers. The main fibers were dispersed in water to prepare a slurry. A sheet for front side fibers with a basis weight of 130 g/m$^2$ was formed from the slurry by a sheet forming method. Polyethylene terephthalate fibers with a single fiber fineness of 0.15 dtex and a melting point of 255°C were also produced by a direct melt spinning method, and cut to lengths of 5 mm as main fibers. The main fibers were dispersed in water to prepare a slurry. A sheet for back side fibers with a basis weight of 50 g/mwas formed from the slurry by a sheet forming method. The two layers were layered together with a woven scrim having a basis weight of 100 g/m$^2$ and composed of 166 dtex/48f polyethylene terephthalate fibers, with the sum of the woven density in the MD and the woven density in the CD being 120 (per 2.54 cm), to form the three-layer structure: front side fiber layer/scrim layer/back side fiber layer. The obtained three-layered body was sprayed with a high-speed water stream using a straight-flow injection nozzle for intertwining entanglement, after which it was dried for 5 minutes at 130°C using an air-through dryer, to obtain a nonwoven fabric with a three-layer structure.

After raising treatment of the surface of the front side fiber layer of the obtained nonwoven fabric by buffing with 400 mesh sandpaper, a polymer elastomer impregnating liquid was prepared by addition of a polyether water-dispersed polyurethane emulsion (EVAFANOL AP-700 by Nicca Chemical Co., Ltd.) at 9 mass% with respect to the polymer elastomer impregnating liquid and sodium sulfate ($Na_2SO_4$) as an impregnation aid at 3 mass% with respect to the polymer elastomer impregnating liquid, and the impregnating liquid was used to impregnate the raised three-layer structure nonwoven fabric to an adhesion rate of 130% by weight. A pin tenter dryer was then used for heat drying at 130°C to obtain artificial leather fabric.

A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 1. When the bonding points were evaluated by X-ray CT using the same method as Example 1, they were found to be sufficiently smaller than the volume of the thermal bonding resin, and since the structures of 12 μm and smaller were processed as noise (procedure x: Calculation of high-brightness pixel size), evaluation was not possible. The added average of the sizes of the 30 adhered points created by the polyether-based water-dispersed polyurethane emulsion was 4 μm diameter or smaller, as observed at 2000x magnification with the SEM used for evaluation of the fineness.

[Table 2]

| | Main fibers | | Basis weight | Thermoplastic resin (thermal bonding fibers) | | | | | | | Thermal annealing temperature | Deform-ation of thermo-plastic resin | Fragment ion ratio: C4H7O (m/z=71)/ C2H3 (m/z=27.0) | Fragment ion ratio: C7H4O (m/z=104)/ C2H3 (m/z=27.0) | NegativeCNO (m/z=42.0)/ C2H (m/z=25.0) | Number average volume | Volume number density of thermoplastic resin | Long axis/short axis ratio of thermo-plastic resin | Abrasion resistance | Abrasi on loss | Tactile sensatio n evaluati on | Flame retardance |
|---|---|---|---|---|---|---|---|---|---|---|---|---|---|---|---|---|---|---|---|---|---|---|
| | Fine-ness | Fiber length | | Resin type | Resin chemical composition | Melting point | Fineness | Fiber length | Mixing ratio | | | | | | | | | | | | | |
| | | | | | | | | | Front side fiber layer | Back side fiber layer | | | | | | | | | | | | |
| Units | dTex | mm | g/m² | | | °C | dtex | mm | % | % | °C | nm | Positive ion | Positive ion | Negative ion | μm³ | 10¹²/m³ | MD | | mg | Organo-leptic evalua-tion | Grade |
| Example 1-2-1 | 0.15 | 5 | 280 | Resin 1-2-1 | Esteric elastomer | 182 | 0.5 | 5 | 10 | 3 | 190 | 74 | 1.88 | 0.64 | 0.02 | 3600 | 4.2 | 1.9 | VG | 14 | G | 5 |
| Example 1-2-2 | 0.15 | 5 | 280 | Resin 1-2-1 | Esteric elastomer | 182 | 0.7 | 5 | 10 | 3 | 190 | 76 | 1.91 | 0.65 | 0.01 | 5800 | 2.4 | 2.2 | VG | 14 | G | 5 |
| Example 1-2-3 | 0.15 | 5 | 290 | Resin 1-2-1 | Esteric elastomer | 182 | 1.1 | 5 | 10 | 3 | 190 | 73 | 1.87 | 0.62 | 0.02 | 10,800 | 1.8 | 2.6 | VG | 16 | G | 5 |
| Example 1-2-4 | 0.15 | 5 | 280 | Resin 1-2-1 | Esteric elastomer | 182 | 1.1 | 8 | 6 | 3 | 190 | 79 | 1.88 | 0.66 | 0.02 | 15,800 | 0.7 | 4.3 | VG | 19 | G | 5 |
| Example 1-2-5 | 0.15 | 5 | 280 | Resin 1-2-1 | Esteric elastomer | 182 | 1.8 | 5 | 10 | 3 | 190 | 71 | 1.87 | 0.67 | 0.01 | 22,000 | 0.8 | 3.4 | VG | 18 | G | 5 |
| Example 1-2-6 | 0.15 | 5 | 270 | Resin 1-2-2 | Esteric elastomer | 170 | 1.0 | 5 | 8 | 3 | 180 | 33 | 1.12 | 0.82 | 0.02 | 10,300 | 1.4 | 2.7 | G | 21 | G | 5 |
| Example 1-2-7 | 0.15 | 5 | 260 | Resin 1-2-3 | Esteric elastomer | 155 | 1.0 | 5 | 10 | 3 | 165 | 102 | 1.39 | 0.73 | 0.02 | 12,800 | 1.5 | 2.6 | VG | 15 | G | 5 |
| Example 1-2-8 | 0.15 | 5 | 280 | Resin 1-2-3 | Esteric elastomer | 155 | 1.0 | 3 | 9 | 3 | 165 | 98 | 1.41 | 0.71 | 0.01 | 8600 | 2.0 | 2.2 | VG | 16 | G | 5 |
| Example 1-2-9 | 0.15 | 5 | 280 | Resin 1-2-4 | Esteric elastomer | 163 | 1.0 | 5 | 7 | 3 | 170 | 107 | 1.79 | 0.63 | 0.02 | 12,400 | 1.0 | 3.0 | VG | 19 | G | 5 |
| Example 1-2-10 | 0.15 | 5 | 260 | Resin 1-2-4 | Esteric elastomer | 163 | 1.4 | 7 | 5 | 3 | 170 | 106 | 1.80 | 0.64 | 0.02 | 16,700 | 0.5 | 3.8 | G | 22 | G | 5 |
| Example 1-2-11 | 0.15 | 5 | 290 | Resin 1-2-5 | Esteric elastomer | 160 | 1.0 | 5 | 10 | 3 | 170 | 89 | 2.21 | 0.56 | 0.02 | 13,400 | 1.5 | 3.2 | VG | 18 | G | 5 |
| Example 1-2-12 | 0.15 | 5 | 280 | Resin 1-2-6 | Esteric elastomer | 171 | 1.0 | 5 | 10 | 3 | 180 | 82 | 1.69 | 0.69 | 0.02 | 11,400 | 1.6 | 2.8 | VG | 17 | G | 5 |
| Comp. Ex. 1-2-1 | 0.15 | 5 | 280 | CASVEN 8000 | Esteric resin | 178 | 0.7 | 5 | 10 | 3 | 190 | 9 | 0.10 | 0.59 | 0.02 | 6200 | 2.5 | 2.3 | VG | 17 | F | 5 |
| Comp. Ex. 1-2-2 | 0.15 | 5 | 260 | CASVEN 8000 | Esteric resin | 178 | 1.1 | 5 | 10 | 3 | 190 | 11 | 0.09 | 0.59 | 0.020 | 11,100 | 1.7 | 2.5 | VG | 16 | F | 5 |
| Comp. Ex. 1-2-3 | 0.15 | 5 | 290 | Resin 1-2-1 | Esteric elastomer | 182 | 1.1 | 5 | 10 | 3 | 130 | 70 | 1.86 | 0.64 | 0.02 | 485,000 | 0.03 | 256 | P | - | F | 5 |
| Comp. Ex. 1-2-4 | 0.15 | 5 | 290 | Resin 1-2-1 | Esteric elastomer | 182 | 5.0 | 5 | 10 | 3 | 190 | 73 | 1.89 | 0.63 | 0.02 | 54,400 | 0.3 | 3.9 | F | 25 | P | 5 |
| Comp. Ex. 1-2-5 | 0.15 | 5 | 280 | Resin 1-2-7 | Esteric resin | 115 | 1.0 | 5 | 10 | 3 | 130 | 6 | - | - | - | - | - | - | P | - | G | 5 |
| Comp. Ex. 1-2-6 | 0.15 | 5 | 270 | AP-700 | Urethane | - | - | - | - | | 130 | - | 1.03 | 0.06 | 1.01 | Not evaluable | Not evaluable | - | VG | 15 | G | 2 |

(Aspect 1-3)

Methods for producing the resins as starting materials for the thermal bonding resin will now be described.

[Resin Production Example 1-3-1]

Polycondensation reaction was conducted using dimethyl terephthalate, 1,4-butanediol and polytetramethylene glycol, to obtain a polyester-polyether block copolymer elastomer. The specific test conditions are specified below. After adding 1000 g of dimethyl terephthalate, 650 g of 1,4-butanediol and 8.3 g of tetrabutoxytitanium(IV) into a pressurized stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 1210 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-3-1. The melting point of polyester-polyether block copolymer elastomer resin 1 was 182°C, and the reduced viscosity was 3.0 dl/g.

[Resin Production Example 1-3-2]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 780 g of dimethyl terephthalate, 220 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, after which reaction was carried out at 250°C for 5 hours while stirring at 50 rpm for polycondensation reaction to obtain a prepolymer. Polytetramethylene glycol with a number-average molecular weight of 2000 was then added at 500 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-3-2. The melting point of the polyester-polyether block copolymer elastomer resin was 170°C, and the reduced viscosity was 2.3 dl/g.

[Resin Production Example 1-3-3]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 740 g of dimethyl terephthalate, 260 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. Polytetramethylene glycol with a number-average molecular weight of 2000 was then added at 750 g, polycondensation reaction was continued for 2 hours at 250°C while depressurizing to 0.01 atmospheres, and the mixture was restored to ordinary temperature and ordinary pressure and then removed out of the reactor to obtain polyester-polyether block copolymer elastomer resin 1-3-3. The melting point of polyester-polyether block copolymer elastomer resin 1-3-3 was 155°C, and the reduced viscosity was 3.0 dl/g.

[Resin Production Example 1-3-4]

Polycondensation reaction was conducted using dimethyl terephthalate, dimethyl isophthalate, 1,4-butanediol and polytetramethylene glycol, to obtain a polyester-polyether block copolymer elastomer. The specific test conditions are specified below. After adding 770 g of dimethyl terephthalate, 230 g of dimethyl isophthalate, 650 g of 1,4-butanediol and 8.3 g of tetrabutoxytitanium(IV) into a stirrer-equipped 5 L autoclave reactor by Sibata Scientific Technology, Ltd., the mixture was adequately exchanged with nitrogen and pressurized to 2 atmospheres with nitrogen, and reaction was carried out at 250°C for 5 hours while stirring at 50 rpm for polycondensation reaction to obtain a prepolymer. After adding 1120 g of polytetramethylene glycol with a number-average molecular weight of 2900, polycondensation reaction was carried out to obtain polyester-polyether block copolymer elastomer resin 1-3-4. The melting point of polyester-polyether block copolymer elastomer resin 1-3-4 was 163°C, and the reduced viscosity was 2.9 dl/g.

The method for producing the thermal bonding fibers is described above.

[Fiber Production Example 1-3-1]

Resin 1-3-1 produced in Resin Production Example 1-3-1 was dried for 8 hours with a dehumidifying dryer at a drying temperature of 80°C, after which it was melt extruded with a single-screw extruder at an extrusion temperature of 230°C, and discharged from a spinneret with 400 nozzle holes and a nozzle diameter of 0.15 mm at a spinning temperature of 250°C and a discharge throughput of 0.05 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 200 dtex/400 filament.
The obtained multifilament was bundled as a group of 50 strands to form a tow, and cut to 5 mm fiber lengths with a rotary cutter at a cutting speed of 80 m/min and a cutting tension of 0.01 g/d, to obtain thermal bonding fibers composed of resin 1-3-1 at 0.5 dtex.

[Fiber Production Example 1-3-2]

A multifilament with a fineness of 280 dtex/400 filament was obtained in the same manner as Fiber Production Example 1-3-1, except that discharge was at a discharge throughput of 0.07 g/min per hole. The obtained multifilament was cut under the same cutting conditions as Fiber Production Example 1-3-1, to obtain thermal bonding fibers composed of resin 1-3-1 at 0.7 dtex.

[Fiber Production Example 1-3-3]

A multifilament with a fineness of 440 dtex/400 filament was obtained in the same manner as Fiber Production Example 1-3-1, except that discharge was at a discharge throughput of 0.11 g/min per hole. The obtained multifilament was cut under the same cutting conditions as Fiber Production Example 1-3-1, to obtain thermal bonding fibers composed of resin 1-3-1 at 1.1 dtex.

[Fiber Production Example 1-3-4]

A multifilament with a fineness of 720 dtex/400 filament was obtained in the same manner as Fiber Production Example 1-3-1, except that discharge was at a discharge throughput of 0.18 g/min per hole. The obtained multifilament was cut under the same cutting conditions as Fiber Production Example 1-3-1, to obtain thermal bonding fibers composed of resin 1-3-1 at 1.8 dtex.

[Fiber Production Example 1-3-5]

A multifilament with a fineness of 400 dtex/400 filament was obtained in the same manner as Fiber Production Example 1-3-1, except that resin 1-3-2 produced in Resin Production Example 1-3-2 was discharged at a discharge throughput of 0.10 g/min per hole. The obtained multifilament was cut under the same cutting conditions as Fiber Production Example 1-3-1, to obtain thermal bonding fibers composed of resin 1-3-2 at 1.0 dtex.

[Fiber Production Example 1-3-6]

A multifilament with a fineness of 400 dtex/400 filament was obtained in the same manner as Fiber Production Example 1-3-1, except that resin 1-3-3 produced in Resin Production Example 1-3-3 was discharged at a discharge throughput of 0.10 g/min per hole. The obtained multifilament was cut under the same cutting conditions as Fiber Production Example 1-3-1, to obtain thermal bonding fibers composed of resin 1-3-3 at 1.0 dtex.

[Fiber Production Example 1-3-7]

A multifilament with a fineness of 720 dtex/400 filament was obtained in the same manner as Fiber Production Example 1-3-1, except that resin 1-3-3 produced in Resin Production Example 1-3-3 was discharged at a discharge throughput of 0.18 g/min per hole. The obtained multifilament was cut under the same cutting conditions as Fiber Production Example 1-3-1, to obtain thermal bonding fibers composed of resin 1-3-3 at 1.8 dtex.

[Fiber Production Example 1-3-8]

A multifilament with a fineness of 400 dtex/400 filament was obtained in the same manner as Fiber Production Example 1-3-1, except that resin 1-3-4 produced in Resin Production Example 1-3-4 was discharged at a discharge throughput of 0.10 g/min per hole. The obtained multifilament was cut under the same cutting conditions as Fiber Production Example 1-3-1, to obtain thermal bonding fibers composed of resin 1-3-4 at 1.0 dtex.

[Fiber Production Example 1-3-9]

A polyamide copolymer (PEBAX 6333SP-01 by Arkema) was dried for 8 hours with a dehumidifying dryer at a drying temperature of 80°C, after which it was melt extruded with a single-screw extruder at an extrusion temperature of 250°C, and discharged from a spinneret with 400 nozzle holes and a nozzle diameter of 0.15 mm at a spinning temperature of 260°C and a discharge throughput of 0.10 g/min per hole, and subsequently contacted with 20°C cooling air at a wind speed of 0.5 m/s, at a location about 100 mm to 900 mm below the spinneret, to cool the filament, and taken up at a take-up speed of 1000 m/min to obtain a multifilament with a fineness of 400 dtex/400 filament. The obtained multifilament was cut under the same cutting conditions as Fiber Production Example 1-3-1, to obtain thermal bonding fibers composed of PEBAX 6333SP-01 at 1.0 dtex.

[Fiber Production Example 1-3-10]

A multifilament with a fineness of 2000 dtex/400 filament was obtained in the same manner as Fiber Production Example 1-3-1, except that discharge was at a discharge throughput of 0.50 g/min per hole, and cooling of the filament was by contact of cooling air at a wind speed of 0.9 m/s. The obtained multifilament was cut under the same cutting conditions as Fiber Production Example 1-3-1, to obtain thermal bonding fibers composed of resin 1-3-1 at 5.0 dtex.

[Example 1-3-1]

Polyethylene terephthalate fibers with a single fiber fineness of 0.15 dtex and a melting point of 255°C were produced by a direct spinning method, and cut to lengths of 5 mm as main fibers. The thermal bonding fibers used were the fibers produced in Fiber Production Example 1-3-1 (fineness: 0.5 dtex, fiber length: 5 mm). The fibers were dispersed in water to a weight ratio of main fiber:thermal bonding fiber = 90: 10 to prepare a slurry. A sheet for front side fibers with a basis weight of 130 g/m$^2$ was formed from the slurry by a sheet forming method.

A sheet for back side fibers having a basis weight of 50 g/m$^2$ was produced by a sheet forming method from the slurry dispersed in water to a weight ratio of main fiber:thermal bonding fibers = 97:3. The two layers were layered together with a woven scrim having a basis weight of 100 g/m$^2$ and composed of 166 dtex/48f polyethylene terephthalate fibers, with the sum of the woven density in the MD and the woven density in the CD being 120 (per 2.54 cm), to form the three-layer structure: front side fiber layer/scrim layer/back side fiber layer. The obtained three-layered body was sprayed with a high-speed water stream using a straight-flow injection nozzle for intertwining enentanglement, after which it was dried for 5 minutes at 130°C using an air-through dryer, to obtain a nonwoven fabric with a three-layer structure.

The surface of the front side fiber layer of the obtained nonwoven fabric was subjected to raising treatment by buffing with 400 mesh sandpaper, and then the nonwoven fabric was slackened to a shrinkage factor of 5% in both the MD and CD using an X4HDHT biaxial stretching test apparatus by Toyo Seiki Seisakusho, Ltd., anchoring at the centers on both sides and at the four corners with a compressed air grip. The nonwoven fabric was then subjected to thermal annealing treatment in a chamber at 190°C for 5 minutes to obtain a nonwoven fabric for artificial leather. A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 3.

[Example 1-3-2]

Suede-like artificial leather 2 was obtained in the same manner as Example 1-3-1, except that the fibers produced in Fiber Production Example 1-3-2 (resin 1-3-1, fineness: 0.7 dtex, fiber length: 5 mm) were used as the thermal bonding fibers. The production conditions and evaluation results for the obtained artificial leather are shown in Table 3.

[Example 1-3-3]

Suede-like artificial leather 3 was obtained in the same manner as Example 1-3-1, except that the thermal bonding fibers used were the fibers produced in Fiber Production Example 1-3-3 (resin 1-3-1, fineness: 1.1 dtex, fiber length: 5 mm), and the basis weight of the sheet for front side fibers was 140 g/m$^2$. The production conditions and evaluation results for the obtained artificial leather are shown in Table 3.

[Example 1-3-4]

Suede-like artificial leather 4 was obtained in the same manner as Example 1-3-1, except that the thermal bonding fibers used were the fibers produced in Fiber Production Example 1-3-3 (resin 1-3-1, fineness: 1.1 dtex, fiber length: 5 mm), the basis weight of the sheet for front side fibers was 150 g/m$^2$, the weight ratio of the front side fiber layer was main fiber:thermal bonding fiber = 88:12, and the weight ratio of the back side fiber layer was main fiber:thermal bonding fiber = 95:5. The production conditions and evaluation results for the obtained artificial leather are shown in Table 3.

[Example 1-3-5]

Suede-like artificial leather 5 was obtained in the same manner as Example 1-3-1, except that the fiber lengths of the fibers produced in Fiber Production Example 1-3-3 (resin 1-3-1, fineness: 1.1 dtex) as the thermal bonding fibers were changed to 3 mm, the weight ratio of the sheet for front side fibers was main fiber:thermal bonding fiber = 94:6, and the basis weight of the sheet for front side fibers was 110 g/m$^2$. The production conditions and evaluation results for the obtained artificial leather are shown in Table 3.

[Example 1-3-6]

Suede-like artificial leather 6 was obtained in the same manner as Example 1-3-1, except that the fiber lengths of the fibers produced in Fiber Production Example 1-3-3 (resin 1-3-1, fineness: 1.1 dtex) as the thermal bonding fibers were changed to 8 mm, and the weight ratio of the sheet for front side fibers was main fiber:thermal bonding fiber = 94:6. The production conditions and evaluation results for the obtained artificial leather are shown in Table 3.

[Example 1-3-7]

Suede-like artificial leather 7 was obtained in the same manner as Example 1-3-1, except that the thermal bonding fibers used were the fibers produced in Fiber Production Example 1-3-4 (resin 1-3-1, fineness: 1.8 dtex, fiber length: 5 mm), and the basis weight of the sheet for front side fibers was 90 g/m$^2$. The production conditions and evaluation results for the obtained artificial leather are shown in Table 3.

[Example 1-3-8]

Suede-like artificial leather 8 was obtained in the same manner as Example 1-3-1, except that the thermal bonding fibers were changed to the fibers produced in Fiber Production Example 1-3-4 (resin 1-3-1, fineness: 1.8 dtex, fiber length: 5 mm), the weight ratio of the sheet for front side fibers was main fiber:thermal bonding fiber = 94:6, and the basis weight of the sheet for front side fibers was 80 g/m$^2$. The production conditions and evaluation results for the obtained artificial leather are shown in Table 3.

[Example 1-3-9]

Suede-like artificial leather 9 was obtained in the same manner as Example 1-3-1, except that the thermal bonding fibers used were the fibers produced in Fiber Production Example 1-3-5 (resin 1-3-2, fineness: 1.0 dtex, fiber length: 5 mm), the basis weight of the sheet for front side fibers was 110 g/m$^2$, the weight ratio of the front side fiber layer was main fiber:thermal bonding fiber = 92:8, and the thermal annealing temperature was 180°C. The production conditions and evaluation results for the obtained artificial leather are shown in Table 3.

[Example 1-3-10]

Suede-like artificial leather 10 was obtained in the same manner as Example 1-3-1, except that the thermal bonding fibers used were the fibers produced in Fiber Production Example 1-3-6 (resin 1-3-3, fineness: 1.0 dtex, fiber length: 5 mm), the basis weight of the sheet for front side fibers was 110 g/m$^2$, and the thermal annealing temperature was 165°C. The production conditions and evaluation results for the obtained artificial leather are shown in Table 3.

[Example 1-3-11]

Suede-like artificial leather 11 was obtained in the same manner as Example 1-3-1, except that the thermal bonding fibers used were the fibers produced in Fiber Production Example 1-3-7 (resin 1-3-3, fineness: 1.8 dtex, fiber length: 5 mm), the basis weight of the sheet for front side fibers was 120 g/m$^2$, and the thermal annealing temperature was 165°C. The production conditions and evaluation results for the obtained artificial leather are shown in Table 3.

[Example 1-3-12]

Suede-like artificial leather 12 was obtained in the same manner as Example 1-3-1, except that the thermal bonding fibers used were the fibers produced in Fiber Production Example 1-3-8 (resin 1-3-4, fineness: 1.0 dtex, fiber length: 5 mm), and the thermal annealing temperature was 170°C. The production conditions and evaluation results for the obtained artificial leather are shown in Table 3.

[Example 1-3-13]

Suede-like artificial leather 13 was obtained in the same manner as Example 1-3-1, except that the thermal bonding fibers used were the fibers produced in Fiber Production Example 1-3-8 (resin 1-3-4, fineness: 1.0 dtex, fiber length: 5 mm), the weight ratio of the front side fiber layer was main fiber:thermal bonding fiber = 93:7, and the thermal annealing temperature was 170°C. The production conditions and evaluation results for the obtained artificial leather are shown in Table 3.

[Comparative Example 1-3-1]

Suede-like artificial leather 14 was obtained in the same manner as Example 1-3-1, except that the thermal bonding fibers used were Fully-meltable CASVEN 8000 thermal bonding stable fibers (Unitika, Ltd.) having a single fiber fineness of 0.7 dtex and lengths of 5 mm. The production conditions and evaluation results for the obtained artificial leather are shown in Table 3.

[Comparative Example 1-3-2]

Suede-like artificial leather 15 was obtained in the same manner as Example 1-3-1, except that the thermal bonding fibers used were Fully-meltable CASVEN 8000 thermal bonding stable fibers (Unitika, Ltd.) having a single fiber fineness of 1.1 dtex and lengths of 5 mm, and the basis weight of the sheet for front side fibers was 110 g/m$^2$. The production conditions and evaluation results for the obtained artificial leather are shown in Table 3.

[Comparative Example 1-3-3]

Suede-like artificial leather 16 was obtained in the same manner as Example 1-3-1, except that the thermal bonding fibers used were Fully-meltable MELTY 4080 thermal bonding stable fibers (Unitika, Ltd.) having a single fiber fineness of 2.2 dtex and lengths of 5 mm, and the basis weight of the sheet for front side fibers was 110 g/m$^2$. The production conditions and evaluation results for the obtained artificial leather are shown in Table 3.

[Comparative Example 1-3-4]

Suede-like artificial leather 18 was obtained in the same manner as Example 1-3-1, except that the thermal bonding fibers used were the fibers produced in Fiber Production Example 1-3-9 (PEBAX 6333SP-01, staple fiber fineness: 1.1 dtex, fiber length: 5 mm), the basis weight of the sheet for front side fibers was 110 g/m$^2$, and the thermal annealing temperature was 180°C. The production conditions and evaluation results for the obtained artificial leather are shown in Table 3.

[Comparative Example 1-3-5]

Suede-like artificial leather 19 was obtained in the same manner as Example 1-3-1, except that the thermal bonding fibers used were the fibers produced in Fiber Production Example 1-3-10 (resin 1-3-1, fineness: 5.0 dtex, fiber length: 5 mm), and the basis weight of the sheet for front side fibers was 140 g/m$^2$. The production conditions and evaluation results for the obtained artificial leather are shown in Table 3.

[Comparative Example 1-3-6]

Suede-like artificial leather 20 was obtained in the same manner as Example 1-3-1, except that the thermal bonding fibers used were core-sheath composite thermal bonding stable fibers CASVEN 8080 (2.2T5-8080 type by Unitika, Ltd.) having a single fiber fineness of 2.2 dtex and a length of 5 mm, composed of polyethylene terephthalate copolymer with a melting point of 181°C as the sheath and polyethylene terephthalate mono-copolymer with a melting point of 255°C as the core. The production conditions and evaluation results for the obtained artificial leather are shown in Table 3.

[Comparative Example 1-3-7]

Suede-like artificial leather 21 was obtained in the same manner as Example 1-3-3, except that thermal annealing temperature was 130°C. The production conditions and evaluation results for the obtained artificial leather are shown in Table 3.

[Table 3]

| | Main fibers | | | Thermoplastic resin (hermal bonding fibers) | | | | | | | | Thermal anneal-ing tem-peratur e | Deforma-tion of ther-moplastic resin | Numb er av-erage vol-ume | Volume number density of thermo-plastic res-in | Long axis/ short axis ratio of thermo-plastic res-in | Abra-sion re-sistance | Abra-sio n loss | Tactile sensation evaluation |
| | Fine-ness | Fiber length | Basis weight of arti-ficial leather | Type of resin used | Resin chemical composi-tion | Melt-ing point | Flexu-ral modu-lus | Fine-ness | Fiber lengt h | Mixing ra-tio | | | | | | | | | |
| | | | | | | | | | | Front side fiber layer | Back side fiber layer | | | | | | | | |
| Units | dtex | mm | g/m² | | | °C | MPa | dtex | mm | % | % | °C | nm | μm³ | 10¹² /m³ | - | MD | mg | Orga-noleptic evaluation |
| Exam-ple 1-3-1 | 0.15 | 5 | 280 | Resin 1-3-1 | | 182 | 76 | 0.5 | 5 | 10 | 3 | 190 | 74 | 3600 | 4.2 | 1.9 | VG | 14 | G |
| Example 1-3-2 | 0.15 | 5 | 280 | Resin 1-3-1 | | 182 | 76 | 0.7 | 5 | 10 | 3 | 190 | 76 | 5800 | 2.4 | 2.2 | VG | 14 | G |
| Example 1-3-3 | 0.15 | 5 | 290 | Resin 1-3-1 | | 182 | 76 | 1.1 | 5 | 10 | 3 | 190 | 73 | 10,800 | 1.8 | 2.6 | VG | 16 | G |
| Example 1-3-4 | 0.15 | 5 | 300 | Resin 1-3-1 | | 182 | 76 | 1.1 | 5 | 12 | 5 | 190 | 73 | 11,100 | 2.0 | 2.7 | VG | 15 | G |
| Example 1-3-5 | 0.15 | 5 | 260 | Resin 1-3-1 | | 182 | 76 | 1.1 | 3 | 6 | 3 | 190 | 78 | 8800 | 1.1 | 2.1 | VG | 19 | G |
| Example 1-3-6 | 0.15 | 5 | 280 | Resin 1-3-1 | Polyester and aliphatic polyether copolymer | 182 | 76 | 1.1 | 8 | 6 | 3 | 190 | 79 | 15,800 | 0.7 | 4.3 | VG | 19 | G |

(continued)

| Units | Main fibers | | | Type of resin used | Resin chemical composition | Thermoplastic resin (hermal bonding fibers) | | | | | | Thermal annealing temperature | Deformation of thermoplastic resin | Number average volume | Volume number density of thermoplastic resin | Long axis/short axis ratio of thermoplastic resin | Abrasion resistance | Abrasion loss | Tactile sensation evaluation |
|---|---|---|---|---|---|---|---|---|---|---|---|---|---|---|---|---|---|---|---|
| | Fineness | Fiber length | Basis weight of artificial leather | | | Melting point | Flexural modulus | Fineness | Fiber length | Mixing ratio Front side fiber layer | Back side fiber layer | | | | | | | | |
| Units | dtex | mm | g/m² | | | °C | MPa | dtex | mm | % | % | °C | nm | μm³ | 10¹² /m³ | - | MD | mg | Organoleptic evaluation |
| Example 1-3-7 | 0.15 | 5 | 240 | Resin 1-3-1 | | 182 | 76 | 1.8 | 5 | 10 | 3 | 190 | 71 | 22,000 | 0.8 | 3.4 | VG | 18 | G |
| Example 1-3-8 | 0.15 | 5 | 230 | Resin 1-3-1 | | 182 | 76 | 1.8 | 5 | 6 | 3 | 190 | 75 | 23,100 | 0.5 | 3.5 | VG | 20 | G |
| Example 1-3-9 | 0.15 | 5 | 260 | Resin 1-3-2 | | 170 | 118 | 1.0 | 5 | 8 | 3 | 180 | 33 | 10,300 | 1.4 | 2.7 | G | 21 | G |
| Example 1-3-10 | 0.15 | 5 | 260 | Resin 1-3-3 | | 155 | 49 | 1.0 | 5 | 10 | 3 | 165 | 102 | 12,800 | 1.5 | 2.6 | VG | 15 | G |
| Example 1-3-11 | 0.15 | 5 | 270 | Resin 1-3-3 | | 155 | 49 | 1.8 | 5 | 10 | 3 | 165 | 99 | 17,100 | 1.0 | 3.7 | VG | 16 | G |
| Example 1-3-12 | 0.15 | 5 | 280 | Resin 1-3-4 | | 163 | 53 | 1.0 | 5 | 10 | 3 | 170 | 106 | 11,900 | 1.6 | 2.9 | VG | 17 | G |
| Example 1-3-13 | 0.15 | 5 | 280 | Resin 1-3-4 | | 163 | 53 | 1.0 | 5 | 7 | 3 | 170 | 107 | 12,400 | 1.0 | 3.0 | VG | 19 | G |

(continued)

| Units | Main fibers | | Basis weight of artificial leather | Thermoplastic resin (hermal bonding fibers) | | | | | | Mixing ratio | | Thermal annealing temperature | Deformation of thermoplastic resin | Number average volume | Volume number density of thermoplastic resin | Long axis/short axis ratio of thermoplastic resin | Abrasion resistance | Abrasion loss | Tactile sensation evaluation |
|---|---|---|---|---|---|---|---|---|---|---|---|---|---|---|---|---|---|---|---|
| | Fineness | Fiber length | | Type of resin used | Resin chemical composition | Melting point | Flexural modulus | Fineness | Fiber length | Front side fiber layer | Back side fiber layer | | | | | | | | |
| Units | dtex | mm | g/m² | | | °C | MPa | dtex | mm | % | % | °C | nm | μm³ | 10¹² /m³ | - | MD | mg | Organoleptic evaluation |
| Comp. Ex. 1-3-1 | 0.15 | 5 | 280 | CASVEN 8000 | Esteric resin | 178 | - | 0.7 | 5 | 10 | 3 | 190 | 9 | 6200 | 2.5 | 2.3 | VG | 17 | F |
| Comp. Ex. 1-3-2 | 0.15 | 5 | 260 | CASVEN 8000 | Esteric resin | 178 | - | 1.1 | 5 | 10 | 3 | 190 | 11 | 11,100 | 1.7 | 2.5 | VG | 16 | F |
| Comp. Ex. 1-3-3 | 0.15 | 5 | 260 | MELTY 4080 | Esteric resin | 110 | - | 2.2 | 5 | 10 | 3 | 190 | 7 | 25,400 | 0.7 | 3.1 | P | - | P |
| Comp. Ex. 1-3-4 | 0.15 | 5 | 260 | PEBAX 6333SP 01 | Nylon elastomer | 169 | 285 | 1.1 | 5 | 10 | 3 | 180 | 16 | 9900 | 1.8 | 2.9 | VG | 18 | P |
| Comp. Ex. 1-3-5 | 0.15 | 5 | 290 | Resin 1-3-1 | Polyester and aliphatic polyether copolymer | 182 | 76 | 5.0 | 5 | 10 | 3 | 190 | 73 | 54,400 | 0.3 | 3.9 | F | 25 | P |
| Comp. Ex. 1-3-6 | 0.15 | 5 | 280 | CASVEN 8080 | Esteric resin | 181 | | 2.2 | 5 | 10 | 3 | 190 | 8 | 28,500 | 0.4 | 7.8 | P | | P |

(continued)

| | Main fibers | | | Thermoplastic resin (hermal bonding fibers) | | | | | | | | | Thermal anneal-ing tem-peratur e | Deforma-tion of ther-moplastic resin | Numb er av-erage vol-ume | Volume number density of thermo-plastic res-in | Long axis/ short axis ratio of thermo-plastic res-in | Abra-sion re-sistance | Abra-sio n loss | Tactile sensation evaluation |
|---|---|---|---|---|---|---|---|---|---|---|---|---|---|---|---|---|---|---|---|---|
| | Fine-ness | Fiber lengt h | Basis weight of arti-ficial leather | Type of resin used | Resin chemical composi-tion | Melt-ing point | Flexu-ral modu-lus | Fine-ness | Fiber lengt h | Mixing ra-tio | | | | | | | | | | |
| | | | | | | | | | | Front side fiber layer | Back side fiber layer | | | | | | | | |
| Units | dtex | mm | g/m$^2$ | | | °C | MPa | dtex | mm | % | % | °C | nm | μm$^3$ | 10$^{12}$ /m$^3$ | - | MD | mg | Orga-noleptic evaluation |
| Comp. Ex. 1-3-7 | 0.15 | 5 | 290 | Resin 1-3-1 | Polyester and aliphatic polyether copolymer | 182 | 76 | 1.1 | 5 | 10 | 3 | 130 | 70 | 485,000 | 0.03 | 256 | P | | F |

(Aspect 2)

The present invention will now be explained in greater detail using Examples and Comparative Examples, with the understanding that the invention is not limited only to the Examples. The values for the physical properties used in the Examples were measured by the following methods.

(1) Evaluation of number average volume and volume number density of thermoplastic resin (resin masses)

Observation of the mass volume of each thermoplastic resin was in the thickness direction of the artificial leather using an X-ray CT apparatus ("Nano3DX High-Resolution 3D X-ray microscope" by Rigaku Corp.), photographing a 3D image with the center section of the crosssection in the thickness direction as the center point of the observation region. The image measurement was performed using copper as the X-ray target, under conditions with a tube voltage of 40 kV, a tube current of 30 mA, an exposure time of 12 seconds/image and a spatial resolution of 1.08 $\mu$m/pixel. The same procedure was used to take 1000 images for every 180° rotation angle, to obtain 3D measurement data.

The detailed process for processing and analysis of the acquired 3D measurement data is described below. The image analysis software used was "Imaged (version 1.51j8), US National Institutes of Health).

(i: Image rotation) The in-plane direction of the artificial leather is aligned with the xz axis plane, and 3D image is rotated with the thickness direction of the artificial leather on the y-axis.

(ii: Trimming) The 3D image is trimmed to a rectangular solid. The y-axis is set as a range so that the entire thickness fits without excess space outside the film. The x-axis and z-axis are each set as the maximum range so that no pixel outside the measuring field is present within the trimmed rectangular solid, after setting the y-axis range.

(iii: Axis setting) The pixel count in the x-axis direction of the trimmed image is x0, the pixel count in the y-axis direction is y0, and the pixel count in the z-axis direction is z0.

(iv: Filtering) A median filter is applied with the condition of radius 2 pix.

(v: Region partitioning) The Otsu method is used, with partitioning into two regions: an air region excluding the artificial leather and a main fiber region forming the artificial leather. The pixel brightness values are set as 0 in the air region excluding the artificial leather and 255 in the main fiber region forming the artificial leather.

(vi: Segmentation) The pixels with 255 brightness values are segmented by image processing, partitioning 255 brightness value subregions that are connected in three-dimensions. Of the 255 brightness value subregions connected in three dimensions, the subregions with total pixel counts (pix) of 10,000 or less are considered to be noise and are removed by changing their brightness values to 0.

(vii: Noise removal) The pixels with brightness values of 0 that are surrounded by brightness values of 255 in three dimensions are considered to be noise and are removed by setting their brightness values to 255.

(viii: Calculation of void percentage) A 2D image on the xz plane is cut out from the 3D image to a thickness of 1 pix in the thickness direction, and the void percentage on that plane is calculated by the following formula:

$$\text{Void percentage} = \text{Number of pixels of brightness value 0 in 2D image / total number of pixels in 2D image}.$$

(ix: Thickness distribution of void percentage) The procedure of (viii) above is carried out for all of the y data, and the distribution of the void percentage in the y-axis direction is calculated.

(x: Calculation of high-brightness pixel size) The sizes of pixels with brightness values of 255 are calculated by the Thickness method. This method produces a 3D image in which the value of the diameter of the maximum sphere at the location of each pixel has been represented as a brightness value. The Thickness method is described in the publication: "A new method for the model-independent assessment of thickness in three-dimensional images" T. Hildebrand and P. Ruegsegger, J. of Microscopy, 185 (1996) 67-75, and can be performed, for example, by executing "Thickness" in the plugin BoneJ for the image analysis software ImageJ. In order to remove structures of 12 $\mu$m or smaller, as main fibers, from the scope of analysis in the obtained image, the brightness values of pixels corresponding to ≤12 $\mu$m are set to 0.

(xi: Binarization) the image obtained in (x) above is binarized, setting all pixels with brightness values other than 0 to 255, and leaving pixels with brightness values of 0 as 0.

(xii: Particle analysis) The image obtained in (xi) above is used for 3D particle analysis. Portions with brightness values of 255 connected in three dimensions are considered to be one particle, and the center coordinate (XC, YC, ZC) of each particle and the pixel count are calculated.

(xiii: Definition of front side, scrim layer and back side) Since 95% of the area in the void percentage distribution data obtained in (ix) above is to be used for analysis (excluding the outer surfaces of the front side and back side for analysis), y1 is defined as the y-axis value furthest from the front side where the void percentage is 0.95 or greater. When a scrim layer is present, the portion with the void percentage numerical value obtained in (viii) and showing fibers with a fineness of 100 dtex or greater is defined as the edge of the front side, and its coordinate is set as y2.

(xiv: Pixel count total calculation and data analysis) The number of particles with coordinate values satisfying y1 < YC < y2 among all of the particles calculated in (xii) is calculated as the particle count, and the total pixel count of the particles is calculated.

$$\text{Number average volume } (\mu m^3) = \text{Total pixel count of particles} *p*p*p*10^{18} \div \text{number of particles}$$

$$\text{Volume number density } (10^{12}/m^3) = \text{Number of particles} \div (|y2 - y1|*p*x0*p*z0*p)$$

In the formulas, p represents the pixel size (m/pix), using the actual size (m) for each pixel (pix).

(2) Average projected area of resin masses

A total of 100 images were taken with a scanning electron microscope at 250x magnification, at 100 arbitrary locations on the sample nonwoven fabric surface. Each image was used to determine the area of 100 resin mass points, and the average was calculated as the average projected area for the resin masses. Additional photographs were taken if 100 resin mass points were not photographed with the 100 images.

A JSM-5610 scanning electron microscope by JEOL Corp. was used for photographing. ImageJ image processing software was used to calculate the area of the resin masses. The area obtained by outlining the resin masses in the images at 250x magnification was calculated by conversion to the captured image scale. The outlined area was rounded to the nearest $0.001 \times 10^{-9}$ m². The average value for the area of the 100 resin mass points was rounded to the nearest $0.01 \times 10^{-9}$ m².

The resin masses were sorted on an assessment scale counting the number where the resin masses adhered between the main fibers and the resin masses were exposed on the surface of the front side fiber layer. If a resin mass adheres between main fibers, then it may be said to be in integral contact with two or more main fibers. When a resin mass is exposed on the surface of the front side fiber layer, then it may be said to be exposed on the surface in the image without the entire surface of the resin mass being hidden by the main fibers. Figs. 1 to 8 are schematic diagrams showing examples of the state of resin masses in front side fiber layers. Regarding the states of resin masses and their assessment as shown in the drawings, Fig. 1 is an example where one is exposed on the surface of the front side fiber layer but does not adhere between main fibers, and Fig. 2 is an example where one is exposed on the surface and adheres between main fibers. Fig. 3 is an example where one is not exposed on the surface and yet adheres between main fibers, Fig. 4 is an example where one is exposed on the surface and adheres between main fibers, and Fig. 5 is an example where one is exposed on the surface but does not adhere between main fibers. Fig. 6 is an example where a thermal bonding fiber is unmelted and cannot be considered as a resin mass. Fig. 7 is an example where a sheath-core fiber is used as the thermal bonding fiber, and the thermal bonding fiber retains its fiber shape and cannot be considered as a resin mass. Fig. 8 is an example of resin masses exposed on the surface, but without adhering between the main fibers. In these drawings, 1 is a main fiber, 2 is a resin mass, 3 is an unmelted resin and 4 is a sheath-core fiber.

The phrase "adheres between main fibers" means that at least two main fibers pass through the interior of the resin mass (thermoplastic resin), creating a physically bonded state.

(3) Fineness

A sample of the front side or back side fiber layer of the artificial leather was photographed at 10 arbitrary locations using a microscope at a magnification of 2500x, and the fiber diameters were measured at 50 points, recording the average as the mean fiber size. This was converted to fineness [dtex] of the main fiber, based on the obtained mean fiber size and the main fiber density.

The artificial leather was immersed in ethanol, encapsulated in a gelatin capsule and freezedried with liquid nitrogen, and then the entire capsule was cut with a knife and the cut crosssection of the sample after returning to ordinary temperature was observed using a scanning electron microscope (JSM-5610 by JEOL Corp.) under conditions of WD = 10 mm, 200x magnification, measuring the thickness of the weaving yarn forming the scrim layer for 5 points in each of 20 obtained images, to determine the fineness of the weaving yarn forming the scrim layer.

The fineness of a thermal bonding strand is the fineness of the starting staple fibers, and it was evaluated using a scanning electron microscope. Specifically, adhesive carbon tape was attached onto the evaluation stage, 0.05 g of the starting staple fibers was placed over it, removing the excess thermal bonding strand with an air duster, and this sample was observed under conditions of WD = 10 mm, 200x magnification, measuring the thickness at 5 points for each of the 20 obtained images.

(4) Abrasion resistance and abrasion loss

The sample surface was abraded with a pressing load of 12 kPa, by the method specified according to JIS-L-1096 E (Martindale method). As the evaluation standard in this test method, the front side layer of the sample was abraded and the number of abrasion cycles required to produce a portion with the scrim layer exposed was evaluated on the following evaluation scale (grade).

(Evaluation scale)

PP: Significant shedding of fibers at 5000 abrasion cycles, making evaluation impossible.
P: Exposure of scrim layer at less than 30,000 abrasion cycles.
F: Exposure of scrim layer at 30,000 or more and less than 40,000 abrasion cycles.
G: Exposure of scrim layer at 40,000 or more and less than 50,000 abrasion cycles.
VG: Exposure of scrim layer at 50,000 or more abrasion cycles.

An artificial leather sample (circular with diameter of 40 mm) as specified by JIS-L-1096 E (Martindale method) was measured for change in weight [mg] before and after 50,000 cycles under a pressing load of 12 kPa, and the result was evaluated as the abrasion loss. The measurement was performed 3 times and the added average was taken as the result.

(5) Melting point

The melting point of the thermal bonding fibers was measured with a DSCQ100 by TA Instruments using aluminum as the reference substance, setting the thermal bonding fibers in a nitrogen atmosphere, increasing the temperature from 25°C to 250°C at 10°C/min and then quenching, and recording the melting point as the peak top of the endothermic peak appearing during a second temperature increase under the same conditions.

(6) Tactile sensation evaluation

The dyed artificial leather sample was cut out to 25 cm squares which were arranged on a desk with the front sides facing upwards, and 20 blindfolded subjects (10 males and 10 females, in pairs from 20-year-old to 60-year old age groups) were asked to examine the tactile sensation along the raised surface. They were simultaneously asked to conduct a similar tactile sensation test for natural suede, and the flexibility and luxurious quality of the front side was organoleptically evaluated on a scale of 5 points (with suede as 5), rounding the points to two decimal places.

G: Average of 3.5 points or more in organoleptic evaluation.
F: Average of 2.5 points or more and less than 3.5 points in organoleptic evaluation.
P: Average of less than 2.5 points in organoleptic evaluation.

(7) Hand quality

A hand quality test was carried out using a KES Pure Bending Tester (KES FB2-A Automated Forward Bending Tester by KATO TECH), and using a sample with a width of 20 cm and a length of 20 cm under SENS 4 conditions. The evaluation scale was as follows. A hand quality value of 1.0 gf·cm$^2$ or lower was considered acceptable.

(Evaluation scale)

P: Hand quality value of greater than 1.0 gf·cm$^2$.
G: Hand quality value of 1.0 gf-cm$^2$ or lower.

(8) Front side fiber layer/scrim entangling strength (N/cm)

The obtained artificial leather sample was cut into two pieces of size 2.5 cm in the MD and 25 cm in the CD, while taking care to ensure perpendicular orientation in each direction. Hot-melt tape with a width of 2.5 cm and a length of 20 cm (S1297-1X10-FT hot-melt tape by Harness Co.) was sandwiched between the front side and back side of the sample, and contact bonding was carried out by pressing at 105°C for 2 minutes using a Hariron presser. After confirming that the bonding surface was sufficiently firm (>20 N/cm), a notch was cut out with a cutter knife from a section on the front side of the sample at the bonding surface where the hot-melt tape was not attached, preparing a sample with the scrim layer and front side as the release surface, which was subjected to a 10 cm tensile test using a tensile tester (RTF-2410 universal testing machine by TENSILON) under conditions with a pull rate of 1.0 cm/sec and a load cell of 50 N. The maximum average of the tensile strength per unit width measured during this time was calculated, and the average of repeating the procedure 3 times was used as the front side fiber layer/scrim entangling strength.
The evaluation was conducted on the following scale.

P: Strength of lower than 4 N/cm.
G: Strength of 4 N/cm or higher.

(9) Elongation under constant load of 500 gf/cm

The elongation of the artificial leather at a load of 500 g/cm was measured by constant speed stretching at 10 cm/min with a test piece width of 2.5 cm and a grip spacing of 10 cm, according to JIS-L-1096 (2015 edition): 8-14 "Tensile strength and elongation percentage"

(Method A: strip method).

(10) Stretch suitability for automobile ceiling material

The adhesive solution of the composition was coated onto the back side of the obtained artificial leather using a comma coater and dried for 2 minutes at 120°C, to obtain artificial leather having a polyurethane resin adhesive layer.

(Adhesive solution composition)

Polycarbonate-based polyurethane resin solution (solid content: 35%, water: 65%): 100 parts
Carbodiimide-based crosslinking agent ($\geq$ 3 functional groups): 10 parts
Thickener (polyacrylamide): 1 part
Defoaming material: 0.1 part

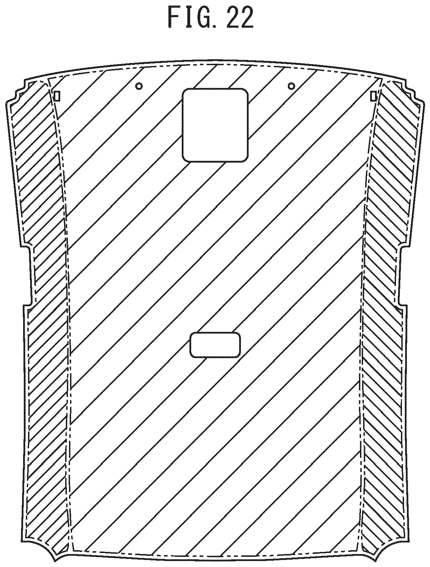

The artificial leather with an adhesive layer obtained in this manner was overlaid with the vehicle roof panel test piece (PET) shown in Fig. 22 and attached by thermocompression bonding using a metal nip roll heated to 120°C. It was then aged for 72 hours at 60°C to firmly attach the test pieces to the artificial leather. The artificial leather having the polyurethane resin adhesive layer, attached at the sections with two holes indicating the margins of the obtained test piece, was punched using a Thomson punching machine, and the stretch suitability for an automobile ceiling material was evaluated on the following scale.

(Evaluation scale)

P: Satisfactory attachment not possible due to wrinkles, edge contraction, or curling from the holes.
G: Satisfactory attachment.

(Aspect 2-1)

[Example 2-1-1]

Polyethylene terephthalate fibers with a single fiber fineness of 0.15 dtex and a melting point of 255°C were produced by a direct spinning method, and cut to lengths of 5 mm as main fibers. The thermal bonding fibers used were Fully-meltable thermal bonding fibers (CASVEN 8000 by Unitika, Ltd.) with a single fiber fineness of 0.7 dtex and lengths of 5 mm and composed of a polyethylene terephthalate copolymer with a melting point of 178°C. The staple fibers were dispersed in water to a weight ratio of main fiber:thermal bonding strand = 90: 10 to prepare a slurry. A sheet for front side fibers with a basis weight of 130 g/m$^2$ was formed from the slurry by a sheet forming method.

Polyethylene terephthalate fibers with a single fiber fineness of 0.15 dtex and a melting point of 255°C were also produced by a direct spinning method, and cut to lengths of 5 mm as main fibers. The thermal bonding fibers used were Fully-meltable thermal bonding fibers (CASVEN 8000 by Unitika, Ltd.) with a single fiber fineness of 0.7 dtex and lengths of 5 mm and composed of a polyethylene terephthalate copolymer with a melting point of 178°C. The staple fibers were dispersed in water to a weight ratio of main fiber:thermal bonding fiber = 97:3 to prepare a slurry. A sheet for back side fibers with a basis weight of 50 g/m$^2$ was formed from the slurry by a sheet forming method. The two layers were layered together with a woven scrim having a basis weight of 100 g/m$^2$ and composed of 166 dtex/48f polyethylene terephthalate fibers, with the sum of the woven density in the MD and the woven density in the CD being 120 (per 2.54 cm), to form the three-layer structure: front side fiber layer/scrim layer/back side fiber layer. The obtained three-layered body was sprayed with a high-speed water stream using a straight-flow injection nozzle for intertwining enentanglement, after which it was dried for 5 minutes at 130°C using an air-through dryer, to obtain a nonwoven fabric with a three-layer structure.

The surface of the front side fiber layer of the obtained nonwoven fabric was subjected to raising treatment by buffing with 400 mesh sandpaper, and then the nonwoven fabric was slackened to a shrinkage factor of 5% in both the MD and CD using an X4HDHT biaxial stretching test apparatus by Toyo Seiki Seisakusho, Ltd., anchoring at the centers on both sides and at the four corners with a compressed air grip. The nonwoven fabric was then subjected to thermal annealing treatment in a chamber at 190°C for 5 minutes to obtain a nonwoven fabric for artificial leather. A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 1.

[Example 2-1-2]

Suede-like artificial leather 2 was obtained in the same manner as Example 2-1-1, except that the weight ratio of the back side fiber layer was main fiber:thermal bonding fiber = 95:5. The production conditions and evaluation results for the obtained artificial leather are shown in Table 4.

[Example 2-1-3]

Suede-like artificial leather 3 was obtained in the same manner as Example 2-1-1, except that the basis weight of the sheet for front side fibers was 110 g/m$^2$ and the single fiber fineness of the thermal bonding fibers was 1.1 dtex. The production conditions and evaluation results for the obtained artificial leather are shown in Table 4.

[Example 2-1-4]

Suede-like artificial leather 4 was obtained in the same manner as Example 2-1-1, except that the single fiber fineness of the thermal bonding fibers was 1.1 dtex, the weight ratio of the sheet for front side fibers was main fiber:thermal bonding fiber = 92:8, and the basis weight of the sheet for front side fibers was 130 g/m$^2$. The production conditions and evaluation results for the obtained artificial leather are shown in Table 4.

[Example 2-1-5]

Suede-like artificial leather 5 was obtained in the same manner as Example 2-1-1, except that the weight ratio of the sheet for front side fibers was main fiber:thermal bonding fiber = 93:7, and the basis weight of the sheet for front side fibers was 80 g/m$^2$. The production conditions and evaluation results for the obtained artificial leather are shown in Table 4.

[Example 2-1-6]

Suede-like artificial leather 6 was obtained in the same manner as Example 2-1-1, except that the fineness of the thermal bonding fibers was 1.1 dtex, the weight ratio of the sheet for front side fibers was main fiber:thermal bonding strand = 88:12, and the weight ratio of the sheet for back side fibers was main fiber thermal bonding fiber = 95:5. The production conditions and evaluation results for the obtained artificial leather are shown in Table 4.

[Example 2-1-7]

Suede-like artificial leather 7 was obtained in the same manner as Example 2-1-1, except that the basis weight of the sheet for front side fibers was 110 g/m$^2$, the basis weight of the sheet for back side fibers was 50 g/m$^2$, the weight ratio of the sheet for front side fibers was main fiber:thermal bonding strand = 87:13, and the weight ratio of the sheet for back side fibers was main fiber:thermal bonding strand = 95:5. The production conditions and evaluation results for the obtained artificial leather are shown in Table 4.

[Example 2-1-8]

Suede-like artificial leather 8 was obtained in the same manner as Example 2-1-1, except that the fineness of the thermal bonding fibers was 0.5 dtex, the basis weight of the sheet for front side fibers was 110 g/m$^2$, the weight ratio of the sheet for back side fibers was main fiber:thermal bonding fiber = 95:5, a pin tenter dryer was used for heating for 5 minutes at 190°C without slackening the base fabric in the MD or CD, and thermal annealing shrinkage was not carried out.

[Comparative Example 2-1-1]

Suede-like artificial leather 9 was obtained in the same manner as Example 2-1-1, except that a pin tenter dryer was used for heating for 5 minutes at 190°C without slackening the base fabric in the MD or CD, and thermal annealing shrinkage was not carried out. The production conditions and evaluation results for the obtained artificial leather are shown in Table 4.

[Comparative Example 2-1-2]

Suede-like artificial leather 10 was obtained in the same manner as Example 2-1-1, except that the basis weight of the sheet for front side fibers was 140 g/m$^2$, the weight ratio of the sheet for front side fibers was main fiber:thermal bonding fiber = 95:5, the weight ratio of the sheet for back side fibers was main fiber:thermal bonding fiber = 95:5, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 4.

[Comparative Example 2-1-3]

Suede-like artificial leather 11 was obtained in the same manner as Example 2-1-1, except that the lengths of the main fibers were 2 mm, the single fiber fineness of the thermal bonding fibers was 1.1 dtex, the basis weight of the sheet for front side fibers was 110 g/m$^2$, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 4.

[Comparative Example 2-1-4]

Suede-like artificial leather 12 was obtained in the same manner as Example 2-1-1, except that the basis weight of the sheet for front side fibers was 150 g/m$^2$, and 3% elongation in both the MD and CD was carried out instead of thermal annealing shrinkage. The production conditions and evaluation results for the obtained artificial leather are shown in Table 4.

[Comparative Example 2-1-5]

Suede-like artificial leather 13 was obtained in the same manner as Example 2-1-1, except that the basis weight of the sheet for front side fibers was 20 g/m$^2$, the weight ratio of the sheet for front side fibers was main fiber:thermal bonding fiber = 70:30, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 4.

[Comparative Example 2-1-6]

Suede-like artificial leather 14 was obtained in the same manner as Example 2-1-1, except that the basis weight of the sheet for front side fibers was 125 g/m$^2$, the weight ratio of the sheet for front side fibers was main fiber:thermal bonding fiber = 97.5:2.5, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 4.

[Comparative Example 2-1-7]

Suede-like artificial leather 15 was obtained in the same manner as Example 2-1-1, except that the thermal bonding fibers used were sheath-core thermal bonding fibers (MELTY 4080 by Unitika, Ltd.) having a single fiber fineness of 2.2 dtex and lengths of 5 mm, and composed of a polyethylene terephthalate copolymer with a melting point of 110°C, the basis weight of the sheet for front side fibers was 139 g/m$^2$, and heat treatment was carried out with 3% elongation in both the MD and CD at a temperature of 125°C, instead of thermal annealing shrinkage. The production conditions and evaluation results for the obtained artificial leather are shown in Table 4.

[Comparative Example 2-1-8]

Suede-like artificial leather 17 was obtained in the same manner as Example 2-1-1, except that the single fiber fineness of the thermal bonding fibers was 1.1 dtex, the basis weight of the sheet for front side fibers was 220 g/m$^2$, the weight ratio of the sheet for front side fibers was main fiber:thermal bonding fiber = 80:20, a pin tenter dryer was used for heating for 5 minutes at 130°C without slackening the base fabric in the MD or CD, and thermal annealing shrinkage was not carried out. No molten resin formation was found. The production conditions and evaluation results for the obtained artificial leather are shown in Table 4.

[Comparative Example 2-1-9]

Suede-like artificial leather 18 was obtained in the same manner as Example 2-1-1, except that the thermal bonding fibers used were sheath-core thermal bonding fibers having a single fiber fineness of 2.2 dtex and lengths of 5 mm (MELTY 4080 by Unitika, Ltd., melting point: 110°C), and composed of a polyethylene terephthalate copolymer with a melting point of 110°C, the basis weight of the sheet for front side fibers was 180 g/m$^2$, the weight ratio of the sheet for front side fibers was main fiber:thermal bonding fiber = 85:15, the weight ratio of the sheet for back side fibers was main fiber:thermal bonding fiber = 96:4, and a pin tenter dryer was used for heating for 5 minutes at 80°C without slackening the base fabric in the MD or CD. No molten resin formation was found. The production conditions and evaluation results for the obtained artificial leather are shown in Table 4.

[Table 4]

| | Main fibers | | Basis weight of artificial leather | Thermoplastic resin ( thermal bonding fibers) | | | | Mixing ratio | | Thermal annealing temperature | Thermal annealing shrinkage | Number average volume | Volume number density of thermoplastic resin | Abrasion resistance | Abrasion loss | Tactile sensation evaluation |
| | Fineness | Fiber length | | Type | Melting point | Fineness | Fiber length | Front side fiber layer | Back side fiber layer | | | | | | | |
| Units | dtex | mm | g/m² | - | °C | dtex | mm | % | % | °C | - | μm³ | 10¹²/m³ | MD | mg | Organoleptic evaluation |
| Example 2-1-1 | 0.15 | 5 | 280 | Fully-meltable | 178 | 0.7 | 5 | 10% | 3% | 190°C | + | 6200 | 2.2 | VG | 17 | G |
| Example 2-1-2 | 0.15 | 5 | 280 | Fully-meltable | 178 | 0.7 | 5 | 10% | 5% | 190°C | + | 5800 | 2.3 | VG | 18 | G |
| Example 2-1-3 | 0.15 | 5 | 260 | Fully-meltable | 178 | 1.1 | 5 | 10% | 3% | 190°C | + | 11,100 | 1.7 | VG | 16 | G |
| Example 2-1-4 | 0.15 | 5 | 280 | Fully-meltable | 178 | 1.1 | 5 | 8% | 3% | 190°C | + | 10,800 | 1.1 | VG | 18 | G |
| Example 2-1-5 | 0.15 | 5 | 230 | Fully-meltable | 178 | 0.7 | 5 | 7% | 3% | 190°C | + | 5400 | 1.8 | VG | 19 | G |
| Example 2-1-6 | 0.15 | 5 | 280 | Fully-meltable | 178 | 1.1 | 5 | 12% | 5% | 190°C | + | 12,800 | 1.5 | VG | 14 | G |
| Example 2-1-7 | 0.15 | 5 | 260 | Fully-meltable | 178 | 0.7 | 5 | 13% | 5% | 190°C | + | 6000 | 2.8 | VG | 14 | G |
| Example 2-1-8 | 0.15 | 5 | 260 | Fully-meltable | 178 | 0.5 | 5 | 10% | 5% | 190°C | 0 | 8800 | 1.9 | VG | 15 | G |
| Comp. Ex. 2-1-1 | 0.15 | 5 | 280 | Fully-meltable | 178 | 0.7 | 5 | 10% | 3% | 190°C | 0 | 14,300 | 0.9 | G | 21 | F |
| Comp. Ex. 2-1-2 | 0.15 | 5 | 290 | Fully-meltable | 178 | 0.7 | 5 | 5% | 5% | 190°C | 0 | 14,500 | 0.4 | P | - | F |
| Comp. Ex. 2-1-3 | 0.15 | 2 | 260 | Fully-meltable | 178 | 1.1 | 5 | 10% | 3% | 190°C | 0 | 19,800 | 0.7 | G | 22 | P |

(continued)

| | Main fibers | | Basis weight of artificial leather | Thermoplastic resin (thermal bonding fibers) | | | | Mixing ratio | | Thermal annealing temperature | Thermal annealing shrinkage | Number average volume | Volume number density of thermoplastic resin | Abrasion resistance | Abrasion loss | Tactile sensation evaluation |
|---|---|---|---|---|---|---|---|---|---|---|---|---|---|---|---|---|
| | Fineness | Fiber length | | Type | Melting point | Fineness | Fiber length | Front side fiber layer | Back side fiber layer | | | | | | | |
| Units | dtex | mm | g/m² | - | °C | dtex | mm | % | % | °C | - | µm³ | 10¹²/m³ | MD | mg | Organoleptic evaluation |
| Comp. Ex. 2-1-4 | 0.15 | 5 | 300 | Fully-meltable | 178 | 0.7 | 5 | 10% | 3% | 190°C | - | 15,800 | 0.8 | F | 24 | P |
| Comp. Ex. 2-1-5 | 0.15 | 5 | 170 | Fully-meltable | 178 | 0.7 | 5 | 30% | 3% | 190°C | 0 | 16,200 | 22 | P | - | P |
| Comp. Ex. 2-1-6 | 0.15 | 5 | 275 | Fully-meltable | 178 | 0.7 | 5 | 2.5% | 3% | 190°C | 0 | 14,300 | 0.2 | P | - | F |
| Comp. Ex. 2-1-7 | 0.15 | 5 | 289 | Sheath-core | 110 | 2.2 | 5 | 10% | 3% | 125°C | - | 24,600 | 0.3 | F | 23 | P |
| Comp. Ex. 2-1-8 | 0.15 | 5 | 370 | Fully-meltable | 178 | 1.1 | 5 | 20% | 3% | 130°C | 0 | No fusible resin | - | PP | - | G |
| Comp. Ex. 2-1-9 | 0.15 | 5 | 330 | Sheath-core | 110 | 22 | 5 | 15% | 4% | 80°C | 0 | No fusible resin | - | PP | - | G |

(Aspect 2-2)

[Example 2-2-1]

Polyethylene terephthalate fibers with a single fiber fineness of 0.15 dtex and a melting point of 255°C were produced by a direct spinning method, and cut to lengths of 5 mm as main fibers. The thermal bonding fibers used were Fully-meltable thermal bonding fibers (CASVEN 8000 by Unitika, Ltd.) with a single fiber fineness of 0.7 dtex and lengths of 5 mm and composed of a polyethylene terephthalate copolymer with a melting point of 178°C. The staple fibers were dispersed in water to a weight ratio of main fiber:thermal bonding strand = 90: 10 to prepare a slurry. A sheet for front side fibers with a basis weight of 130 g/m$^2$ was formed from the slurry by a sheet forming method. Polyethylene terephthalate fibers with a single fiber fineness of 0.15 dtex and a melting point of 255°C were also produced by a direct spinning method, and cut to lengths of 5 mm as main fibers. The thermal bonding fibers used were Fully-meltable thermal bonding fibers (CASVEN 8000 by Unitika, Ltd.) with a single fiber fineness of 0.7 dtex and lengths of 5 mm and composed of a polyethylene terephthalate copolymer with a melting point of 178°C. The staple fibers were dispersed in water to a weight ratio of main fiber:thermal bonding fiber = 97:3 to prepare a slurry. A sheet for back side fibers with a basis weight of 50 g/mwas formed from the slurry by a sheet forming method. The two layers were layered together with a woven scrim to form a three-layer structure: front side fiber layer/scrim layer/back side fiber layer. The obtained three-layered body was sprayed with a high-speed water stream using a straight-flow injection nozzle for intertwining entanglement, after which it was dried for 5 minutes at 130°C using an air-through dryer, to obtain a nonwoven fabric with a three-layer structure. For the scrim layer there was used a woven fabric having a basis weight of 100 g/m$^2$, composed of 166 dtex/48f polyethylene terephthalate fibers, with the sum of the woven density in the MD and the woven density in the CD being 120 (per 2.54 cm).

The surface of the front side fiber layer of the obtained nonwoven fabric was subjected to raising treatment by buffing with 400 mesh sandpaper, and then the nonwoven fabric was slackened to a shrinkage factor of 5% in both the MD and CD using an X4HDHT biaxial stretching test apparatus by Toyo Seiki Seisakusho, Ltd., anchoring at the centers on both sides and at the four corners with a compressed air grip. The nonwoven fabric was then subjected to thermal annealing treatment in a chamber at 190°C for 5 minutes to obtain a nonwoven fabric for artificial leather. A blue disperse dye (BlueFBL by Sumitomo Chemical Co., Ltd.) was then used for dyeing at 130°C with a jet dyeing machine, and reduction cleaning treatment was carried out at 80°C to obtain suede-like artificial leather. The production conditions and evaluation results for the obtained artificial leather are shown in Table 5.

[Example 2-2-2]

Suede-like artificial leather 2 was obtained in the same manner as Example 2-2-1, except that the weight ratio of the back side fiber layer was main fiber:thermal bonding fiber = 95:5. The production conditions and evaluation results for the obtained artificial leather are shown in Table 5.

[Example 2-2-3]

Suede-like artificial leather 3 was obtained in the same manner as Example 2-2-1, except that the basis weight of the sheet for front side fibers was 110 g/m$^2$. The production conditions and evaluation results for the obtained artificial leather are shown in Table 5.

[Example 2-2-4]

Suede-like artificial leather 4 was obtained in the same manner as Example 2-2-1, except that the weight ratio of the sheet for front side fibers was main fiber:thermal bonding fiber = 80:20, and the weight ratio of the sheet for back side fibers was main fiber:thermal bonding strand = 96:4. The production conditions and evaluation results for the obtained artificial leather are shown in Table 5.

[Example 2-2-5]

Suede-like artificial leather 5 was obtained in the same manner as Example 2-2-1, except that the staple fiber fineness of the polyethylene terephthalate fibers was 0.1 dtex, the thermal bonding fiber fineness was 1.1 dtex, and the basis weight of the sheet for front side fibers was 150 g/m$^2$. The production conditions and evaluation results for the obtained artificial leather are shown in Table 5.

[Example 2-2-6]

Suede-like artificial leather 6 was obtained in the same manner as Example 2-2-1, except that the thermal bonding fibers were changed to Fully-meltable thermal bonding fibers (MELTY 4000 by Unitika, Ltd.) having a single fiber fineness of 2.2 dtex and lengths of 5 mm, and composed of a polyethylene terephthalate copolymer with a melting point of 110°C, the basis weight of the sheet for front side fibers was 110 g/m$^2$, the basis weight of the sheet for back side fibers was 50 g/m$^2$, and the thermal annealing treatment temperature was 125°C. The production conditions and evaluation results for the obtained artificial leather are shown in Table 5.

[Example 2-2-7]

Suede-like artificial leather 7 was obtained in the same manner as Example 2-2-1, except that the fineness of the weaving yarn of the scrim layer was changed to 75 denier, the basis weight of the sheet for front side fibers was 90 g/m$^2$, and the weight ratio of the sheet for back side fibers was main fiber:thermal bonding fiber = 95:5. The production conditions and evaluation results for the obtained artificial leather are shown in Table 5.

[Example 2-2-8]

Suede-like artificial leather 8 was obtained in the same manner as Example 2-2-1, except that the fineness of the weaving yarn of the scrim layer was changed to 75 denier, the total woven density of the scrim layer was 100/2.54 cm, the basis weight of the sheet for front side fibers was 80 g/m$^2$, the weight ratio of the sheet for front side fibers was main fiber:thermal bonding fiber = 92:8, and the weight ratio of the sheet for back side fibers was main fiber:thermal bonding fiber = 95:5. The production conditions and evaluation results for the obtained artificial leather are shown in Table 5.

[Example 2-2-9]

Suede-like artificial leather 9 was obtained in the same manner as Example 2-2-1, except that the total woven density of the scrim layer was 140/2.54 cm, the weight ratio of the sheet for front side fibers was main fiber:thermal bonding fiber = 95:5, the basis weight of the sheet for front side fibers was 200 g/m$^2$, and the weight ratio of the sheet for back side fibers was main fiber:thermal bonding fiber = 97:3. The production conditions and evaluation results for the obtained artificial leather are shown in Table 5.

[Comparative Example 2-2-1]

Suede-like artificial leather 13 was obtained in the same manner as Example 2-2-1, except that a pin tenter dryer was used for heating for 5 minutes at 190°C without slackening the base fabric in the MD or CD, and thermal annealing shrinkage was not carried out. The production conditions and evaluation results for the obtained artificial leather are shown in Table 5.

[Comparative Example 2-2-2]

Suede-like artificial leather 14 was obtained in the same manner as Example 2-2-1, except that the basis weight of the sheet for front side fibers was 140 g/m$^2$, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 5.

[Comparative Example 2-2-3]

Suede-like artificial leather 15 was obtained in the same manner as Example 2-2-1, except that the lengths of the main fibers were 2 mm, the single fiber fineness of the thermal bonding fibers was 1.1 dtex, the basis weight of the sheet for front side fibers was 140 g/m$^2$, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 5.

[Comparative Example 2-2-4]

Suede-like artificial leather 16 was obtained in the same manner as Example 2-2-1, except that the basis weight of the sheet for front side fibers was 150 g/m$^2$, and 3% elongation in both the MD and CD was carried out instead of thermal annealing shrinkage. The production conditions and evaluation results for the obtained artificial leather are shown in Table 5.

[Comparative Example 2-2-5]

Suede-like artificial leather 17 was obtained in the same manner as Example 2-2-1, except that the basis weight of the sheet for front side fibers was 20 g/m$^2$, the weight ratio of the sheet for front side fibers was main fiber:thermal bonding fiber = 70:30, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 5.

[Comparative Example 2-2-6]

Suede-like artificial leather 18 was obtained in the same manner as Example 2-2-1, except that the basis weight of the sheet for front side fibers was 125 g/m$^2$, the weight ratio of the sheet for front side fibers was main fiber:thermal bonding fiber = 97.5:2.5, a pin tenter dryer was used for heating for 5 minutes at 190°C without slackening the base fabric in the MD or CD, and thermal annealing shrinkage was not carried out. The production conditions and evaluation results for the obtained artificial leather are shown in Table 5.

[Comparative Example 2-2-7]

Suede-like artificial leather 19 was obtained in the same manner as Example 2-2-1, except that the basis weight of the sheet for front side fibers was 115 g/m$^2$, the weight ratio of the sheet for back side fibers was main fiber:thermal bonding fiber = 95:5, and 3% elongation in both the MD and CD was carried out instead of thermal annealing shrinkage. The production conditions and evaluation results for the obtained artificial leather are shown in Table 5.

[Comparative Example 2-2-8]

Suede-like artificial leather 20 was obtained in the same manner as Example 2-2-1, except that the thermal bonding fibers used were sheath-core thermal bonding fibers (MELTY 4080 by Unitika, Ltd.) having a single fiber fineness of 2.2 dtex and lengths of 5 mm, and composed of a polyethylene terephthalate copolymer with a melting point of 110°C, the basis weight of the sheet for front side fibers was 139 g/m$^2$, and heat treatment was carried out with 3% elongation in both the MD and CD at a temperature of 125°C, instead of thermal annealing shrinkage. The production conditions and evaluation results for the obtained artificial leather are shown in Table 5.

[Comparative Example 2-2-9]

Suede-like artificial leather 21 was obtained in the same manner as Example 2-2-1, except that the fineness of the weaving yarn of the scrim layer was 75 denier, the sum of the woven density of the scrim layer was 75/2.54 cm, the basis weight of the sheet for front side fibers was 100 g/m$^2$, the weight ratio of the sheet for back side fibers was main fiber:thermal bonding fiber = 95:5, and 3% elongation in both the MD and CD was carried out instead of thermal annealing shrinkage. The production conditions and evaluation results for the obtained artificial leather are shown in Table 5.

[Comparative Example 2-2-10]

Suede-like artificial leather 22 was obtained in the same manner as Example 2-2-1, except that the fineness of the weaving yarn of the scrim layer was 100 denier, the sum of the woven density of the scrim layer was 120/2.54 cm, the fineness of the thermal bonding fibers was 1.1 dtex, the basis weight of the sheet for front side fibers was 220 g/m$^2$, the weight ratio of the sheet for front side fibers was main fiber:thermal bonding fiber = 80:20, and a pin tenter dryer was used for heating for 5 minutes at 130°C without slackening the base fabric in the MD or CD. No molten resin formation was found. The production conditions and evaluation results for the obtained artificial leather are shown in Table 5.

[Comparative Example 2-2-11]

Suede-like artificial leather 22 was obtained in the same manner as Example 2-2-1, except that the thermal bonding fibers used were sheath-core thermal bonding fibers having a single fiber fineness of 2.2 dtex and lengths of 5 mm (MELTY 4080 by Unitika, Ltd.), and composed of a polyethylene terephthalate copolymer with a melting point of 110°C, the fineness of the weaving yarn of the scrim layer was 75 denier, the sum of the woven density of the scrim layer was 120/2.54 cm, the basis weight of the sheet for front side fibers was 180 g/m$^2$, the weight ratio of the sheet for front side fibers was main fiber:thermal bonding fiber = 85:15, the weight ratio of the sheet for back side fibers was main fiber:thermal bonding fiber = 96:4, and a pin tenter dryer was used for heating for 5 minutes at 80°C without slackening the base fabric in the MD or CD. No molten resin formation was found. The production conditions and evaluation results for the obtained artificial leather are shown in Table 5.

[Table 5]

| Units | Main fibers Fine-ness (dTex) | Fiber length (mm) | Basis weight of artifi-cial leather (g/m²) | Elonga-tion in MD under 500 g constant load (%) | Elonga-tion in CD under 500 g constant load (%) | Sum of elonga-tions under 500 g constant load (A) (%) | Sum of woven densities (B) (No./inch) | Scrim fine-ness (C) (Denier) | (A)x(B) x(C) | Thermoplastic resin (thermal bonding fibers) (Product No.) (Composition) | Type | Melt-ing point | Fine-ness (dtex) | Mixing ratio Front side fiber layer (%) | Back side fiber layer (%) | Thermal anneal-ing tempera-ture (°C) | Number average volume (μm³) | Volume number density of thermo-plastic resin ($10^{12}$/m³) | Mass size ($\times 10^{-9}$ m²) | Thermal anneal-ing shrink-age (-) | Front fiber layer/scrim entangling strength (N/cm) | Texture (KES bending) | Abra-sion resist-ance (MD) | Ceiling material stretch suitability (Organo-leptic evaluation) |
|---|---|---|---|---|---|---|---|---|---|---|---|---|---|---|---|---|---|---|---|---|---|---|---|---|
| Example 2-2-1 | 0.15 | 5 | 280 | 5.3 | 8.9 | 14.2 | 120 | 150 | 255,600 | CASVEN 8000 | Fully-meltable | 178 | 0.7 | 10% | 3% | 190°C | 6200 | 2.2 | 0.82 | + | G | G | VG | G |
| Example 2-2-2 | 0.15 | 5 | 280 | 4.8 | 12.4 | 17.2 | 120 | 150 | 309,600 | CASVEN 8000 | Fully-meltable | 178 | 0.7 | 10% | 5% | 190°C | 5800 | 2.3 | 0.71 | + | G | G | VG | G |
| Example 2-2-3 | 0.15 | 5 | 260 | 5.4 | 16.7 | 22.1 | 120 | 150 | 397,800 | CASVEN 8000 | Fully-meltable | 178 | 0.7 | 10% | 3% | 190°C | 6100 | 2.2 | 0.74 | + | G | G | VG | G |
| Example 2-2-4 | 0.15 | 5 | 280 | 5.4 | 9.9 | 15.3 | 120 | 150 | 275,400 | CASVEN 8000 | Fully-meltable | 178 | 0.7 | 20% | 4% | 190°C | 6800 | 4.1 | 0.66 | + | G | G | VG | G |
| Example 2-2-5 | 0.1 | 5 | 300 | 5.2 | 10.9 | 16.1 | 120 | 150 | 289,800 | CASVEN 8000 | Fully-meltable | 178 | 1.1 | 10% | 3% | 190°C | 11400 | 1.7 | 1.25 | + | G | G | VG | G |
| Example 2-2-6 | 0.15 | 5 | 260 | 3.6 | 12.0 | 15.6 | 120 | 150 | 280,800 | MELTY 4000 | Fully-meltable | 110 | 2.2 | 10% | 3% | 125°C | 13800 | 1.2 | 2.40 | + | G | G | VG | G |
| Example 2-2-7 | 0.15 | 5 | 240 | 5.2 | 22.3 | 27.5 | 120 | 75 | 247,500 | CASVEN 8000 | Fully-meltable | 178 | 0.7 | 10% | 5% | 190°C | 6600 | 2.1 | 0.69 | + | G | G | VG | G |
| Example 2-2-8 | 0.15 | 5 | 230 | 5.8 | 27.4 | 33.2 | 100 | 75 | 249,000 | CASVEN 8000 | Fully-meltable | 178 | 0.7 | 8% | 5% | 190°C | 6300 | 1.8 | 0.71 | + | G | G | VG | G |
| Example 2-2-9 | 0.15 | 5 | 350 | 5.6 | 8.3 | 13.9 | 140 | 150 | 291,900 | CASVEN 8000 | Fully-meltable | 178 | 0.7 | 5% | 3% | 190°C | 6000 | 1.1 | 0.73 | + | G | G | VG | G |
| Comp. Ex. 2-2-1 | 0.15 | 5 | 280 | 3.6 | 5.9 | 9.5 | 120 | 150 | 171,000 | CASVEN 8000 | Fully-meltable | 178 | 0.7 | 10% | 3% | 190°C | 14300 | 0.9 | 0.65 | 0 | G | G | G | P |
| Comp. Ex. 2-2-2 | 0.15 | 5 | 290 | 3.2 | 5.1 | 8.3 | 120 | 150 | 149,400 | CASVEN 8000 | Fully-meltable | 178 | 0.7 | 5% | 5% | 190°C | 14400 | 0.4 | 0.74 | 0 | G | G | P | P |
| Comp. Ex. 2-2-3 | 0.15 | 2 | 260 | 4.2 | 6.9 | 11.1 | 120 | 150 | 199,800 | CASVEN 8000 | Fully-meltable | 178 | 1.1 | 10% | 3% | 190°C | 19800 | 0.7 | 1.18 | 0 | P | P | G | P |
| Comp. Ex. 2-2-4 | 0.15 | 5 | 300 | 3.2 | 4.4 | 7.6 | 120 | 150 | 136,800 | CASVEN 8000 | Fully-meltable | 178 | 0.7 | 10% | 3% | 190°C | 15200 | 0.8 | 0.72 | - | G | P | F | P |
| Comp. Ex. 2-2-5 | 0.15 | 5 | 170 | 4.5 | 5.7 | 10.2 | 120 | 150 | 183,600 | CASVEN 8000 | Fully-meltable | 178 | 0.7 | 30% | 3% | 190°C | 15400 | 2.9 | >10 | 0 | G | P | P | P |
| Comp. Ex. 2-2-6 | 0.15 | 5 | 275 | 3.8 | 7.5 | 11.3 | 120 | 150 | 203,400 | CASVEN 8000 | Fully-meltable | 178 | 0.7 | 2.5% | 3% | 190°C | 14200 | 0.3 | 0.65 | 0 | G | P | P | P |
| Comp. Ex. 2-2-7 | 0.15 | 5 | 260 | 2.8 | 3.5 | 6.3 | 120 | 150 | 113,400 | CASVEN 8000 | Fully-meltable | 178 | 0.7 | 10% | 5% | 190°C | 15300 | 0.8 | 0.58 | - | G | P | P | P |
| Comp. Ex. 2-2-8 | 0.15 | 5 | 289 | 2.6 | 4.9 | 7.5 | 120 | 150 | 135,000 | MELTY 4080 | Sheath-core | 110 | 2.2 | 10% | 3% | 125°C | 24600 | 0.3 | 2.35 | - | G | P | F | P |
| Comp. Ex. 2-2-9 | 0.15 | 5 | 250 | 7.1 | 27.4 | 34.5 | 75 | 75 | 194,063 | CASVEN 8000 | Fully-meltable | 178 | 0.7 | 10% | 5% | 190°C | 15300 | 0.8 | 0.64 | - | G | G | P | G |
| Comp. Ex. 2-2-10 | 0.15 | 5 | 370 | 5.2 | 10.0 | 15.2 | 120 | 100 | 182,400 | CASVEN 8000 | Fully-meltable | 178 | 1.1 | 20% | 3% | 130°C | - | - | No fusible resin | 0 | G | G | PP | P |
| Comp. Ex. 2-2-11 | 0.15 | 5 | 330 | 5.3 | 12.0 | 17.3 | 120 | 75 | 155,700 | MELTY 4080 | Sheath-core | 110 | 2.2 | 15% | 4% | 80°C | - | - | No fusible resin | 0 | G | G | PP | P |

INDUSTRIAL APPLICABILITY

(Aspect 1)

The artificial leather of the invention has thermoplastic resins adhering between main fibers with single fiber fineness of 0.01 dtex to 0.5 dtex, the deformation of the thermoplastic resin being 20 nm to 200 nm in force curve measurement using an atomic force microscope, and the sizes of the thermoplastic resin masses being 3500 $\mu m^3$ to 24,000 $\mu m^3$ as the number average volume of the thermoplastic resin in the first surface fiber layer as measured by X-ray CT, and it exhibits excellent hand quality and high abrasion resistance without addition of a polyurethane resin. The artificial leather of the invention can therefore be utilized not only as a car interior material but also as a seat skin material or interior material for railway vehicles, aircraft and ships, as well as in the fields of clothing, shoes, bags, smartphone cases, interiors and furniture.

(Aspect 2)

In the artificial leather of the invention, since the thermoplastic resin adheres between main fibers having single fiber fineness of 0.01 dtex to 0.5 dtex, with the entangled structure in a relaxed state, while the sizes of the masses of the thermoplastic resin are 5000 $\mu m^3$ to 14,000 $\mu m^3$ as the number average volume of the thermoplastic resin in the first surface fiber layer as measured by X-ray CT, and the volume number density is $1.1 \times 10^{12}/m^3$ to $3.0 \times 10^{12}/m^3$, the hand quality is excellent and the abrasion resistance is high. Furthermore, if the artificial leather of the invention uses polyester fibers as the main fibers and the thermoplastic fibers, without including an elastic polymer such as water-dispersed polyurethane, then its recycling properties are excellent as well. The artificial leather of the invention has high stretchability and especially excellent stretch suitability as a vehicle ceiling material, and it is therefore suitable for use as a car interior material. The artificial leather of the invention can also be utilized not only as a car interior material but also as a seat skin material or interior material for railway vehicles, aircraft and ships, as well as in the fields of clothing, shoes, bags, smartphone cases, interiors and furniture.

REFERENCE SIGNS LIST

    1 Main fiber
    2 Resin mass
    3 Unmelted resin (thermal bonding fiber)
    4 Sheath-core fiber

(Aspect 2)

    11 Fiber sheet
    12 Scrim layer
    13 Front side fiber layer (A)
    14 Fiber layer (B)
    21 Front layer
    22 Scrim layer
    23 Back layer

**Claims**

1.    Artificial leather that includes at least a front side fiber layer constituting a first surface, and having the following features:

    (1) the front side fiber layer includes main fibers and thermoplastic resins;
    (2) the main fibers have a fineness of 0.01 dtex to 0.5 dtex;
    (3) at least part of the thermoplastic resin adheres between the main fibers;
    (4) when the probe is pressed into the thermoplastic resin in the surface fiber layer using a cantilever with a load of 200 nN in atomic force microscopy, the distance at which the probe is pressed between the contact start point and the maximum load is between 20 nm and 200 nm, and

(5) the number average volume of the thermoplastic resin in the front side fiber layer is 3500 $\mu m^3$ to 24,000 $\mu m^3$.

2. The artificial leather according to claim 1, wherein
the thermoplastic resin satisfies the following (i), (ii) and (iii) in measurement by Time-Of-Flight Secondary Ion Mass Spectrometry(TOF-SIMS):

(i) the ion intensity ratio (71/27) of the positive ion for m/z = 71 (fragment ion: $C_4H_7O$) with respect to m/z = 27 (fragment ion: $C_2H_3$) is 0.2 to 2.3;
(ii) the ion intensity ratio (42/25) for m/z = 42 (fragment ion: CNO) with respect to m/z = 25 (fragment ion: $C_2H$) of the negative ion is 0 to 0.5; and
(iii) the ion intensity ratio (104/27) of the positive ion for m/z = 104 (fragment ion: $C_7H_4O$) with respect to m/z = 27 (fragment ion: $C_2H_3$) is 0.2 to 1.0.

3. The artificial leather according to claim 1 or 2, wherein the thermoplastic resin is copolymer of a polyester and an aliphatic polyether.

4. The artificial leather according to claim 3, wherein the thermoplastic resin is copolymer of polybutylene phthalate and an aliphatic polyether.

5. The artificial leather according to claim 4, wherein the thermoplastic resin is a copolymer of polybutylene phthalate and polytetramethylene ether glycol.

6. The artificial leather according to claim 1 or 2, wherein the main fibers are polyester fibers.

7. The artificial leather according to claim 1 or 2, wherein the volume number density of the thermoplastic resin per unit volume in the front side fiber layer is $0.5 \times 10^{12}/m^3$ to $5.0 \times 10^{12}/m^3$.

8. The artificial leather according to claim 1 or 2, wherein the average quotient of the long axis divided by the short axis is 100 or lower, where the thermoplastic resin in the front side fiber layer is approximated as elliptical.

9. The artificial leather according to claim 1 or 2, wherein the front side fiber layer is entangled with a woven scrim layer.

10. The artificial leather according to claim 9, wherein the scrim layer is composed of polyester resin fibers.

11. The artificial leather according to claim 1 or 2, wherein the scrim layer is not exposed after less than 50,000 abrasion cycles, with abrasion of the surface under a pressing load of 12 kPa according to JIS-L-1096 E (Martindale method).

12. The artificial leather according to claim 1 or 2, wherein the abrasion loss is 21 mg or less after 50,000 abrasion cycles, with abrasion of the surface under a pressing load of 12 kPa according to JIS-L-1096 E (Martindale method).

13. A method for producing artificial leather according to claim 1 or 2, wherein the method includes the following steps:

(1) a step in which the main fibers are mixed with thermal bonding fibers produced using the thermoplastic resin for which the distance from the point of contact initiation to the maximum load is between 20 nm and 200 nm at which the probe is pressed into the thermoplastic resin with a cantilever under a load of 200 nN in measurements using an atomic force microscope, so that the weight ratio of the thermal bonding fibers is 3% to 25% with respect to the front side fiber layer, and the fibers are entangled by wet method (papermaking method)method (papermaking method), after which a front side fiber web is formed by hydroentangling or needle punching, and
(2) a step of forming a front side fiber layer by heat annealing the entangled structure of the front side fiber web obtained in step (1) at a temperature above the melting point of the heatbonded fibers but below the melting point of the main fibers; and wherein in step (2), the front side fiber web has a shrinkage factor in the MD of 0.5 to 5% and a shrinkage factor in the CD of 1 to 8%.

14. The method according to claim 13, wherein the fiber lengths of the thermal bonding fibers composed of the thermoplastic resin are 2 mm to 90 mm.

15. The method according to claim 13, wherein:

the single fiber fineness of the thermal bonding fibers produced using the thermoplastic resin are 0.5 dtex to 2.2 dtex, and
the single fiber fineness of the main fibers are 0.01 dtex to 0.5 dtex.

16. The method according to claim 13, wherein the thermal bonding fibers composed of the thermoplastic resin are produced by melt spinning.

17. The method according to claim 13, wherein at least 95 wt% of the resin in the thermal bonding fibers is a copolymer of a polyester and an aliphatic polyether.

18. The method according to claim 13, wherein the melting point of the thermoplastic resin is 130°C or higher, and at least 20°C lower than the melting point of the main fibers.

19. Artificial leather that includes at least a front side fiber layer constituting a first surface, and having the following features:

(1) the front side fiber layer includes at least one type of main fiber and thermoplastic resins having a melting point of 20°C to 170°C lower than the melting point of the main fibers;
(2) the main fibers have single fiber fineness of 0.01 dtex to 0.5 dtex;
(3) at least part of the thermoplastic resin adheres between the main fibers;
(4) the number average volume of the thermoplastic resin in the first surface fiber layer is 5000 $\mu$m$^3$ to 14,000 $\mu$m$^3$ as measured by X-ray CT; and
(5) the volume number density of the thermoplastic resin in the first surface fiber layer is $1.1 \times 10^{12}$/m$^3$ to $3.0 \times 10^{12}$/m$^3$.

20. The artificial leather according to claim 19, which has the following features:

(6) at least part of the thermoplastic resin is exposed on the surface of the front side fiber layer in the form of resin masses, at a projected area mean value of $0.66 \times 10^{-9}$ m$^2$ to $5.0 \times 10^{-9}$ m$^2$ per resin mass;
(7) the front side fiber layer is entangled with the woven scrim layer; and
(8) the artificial leather satisfies the following inequality:

$$220,000 \leq (A) \times (B) \times (C) \leq 600,000$$

where (A) is the sum of the elongation under 500 gf/cm constant load in the MD (%) and the elongation under 500 gf/cm constant load in the CD (%), (B) is the sum of the scrim layer woven density in the MD (number/2.54 cm) and the woven density in the CD (number/2.54 cm), and (C) is the single fiber fineness (denier) of the weaving yarn forming the scrim layer, and which has an elongation of 3% or greater under 500 gf/cm constant load in the MD.

21. The artificial leather according to claim 19 or 20, wherein the main fibers are polyester fibers.

22. The artificial leather according to claim 19 or 20, wherein the thermoplastic resin is polyester resin.

23. The artificial leather according to claim 19 or 20, wherein the scrim layer is composed of polyester resin fibers.

24. The artificial leather according to claim 19 or 20, wherein the scrim layer is not exposed after less than 50,000 abrasion cycles, with abrasion of the surface under a pressing load of 12 kPa according to JIS-L-1096 E (Martindale method).

25. The artificial leather according to claim 19 or 20, wherein the abrasion loss is 21 mg or less after 50,000 abrasion cycles, with abrasion of the surface under a pressing load of 12 kPa according to JIS-L-1096 E (Martindale method).

26. The artificial leather according to claim 19 or 20, wherein the flexural rigidity per unit width in KES pure bending measurement is 1.0 gfcm$^2$/cm or lower.

**27.** A method for producing artificial leather according to claim 19 or 20, wherein the method has the following steps:

(1) a step in which the main fibers are mixed with thermal bonding fibers composed of the thermoplastic resin having a melting point of 20°C to 170°C the melting point of the main fibers, so that the weight ratio of the thermal bonding fibers is 3% to 25% with respect to the front side fiber layer, and then the fibers are entangled by wet method (papermaking method) on a scrim layer, after which hydroentangling or needle punching is carried out to form a front side fiber web entangled with the scrim layer, and

(2) a step of forming a front side fiber layer by heat annealing the entangled structure of the front side fiber web obtained in step (1) at a temperature above the melting point of the heat-bonded fibers but below the melting point of the main fibers and wherein in step (2), the front side fiber web has a shrinkage factor in the MD of 0.5 to 5% and a shrinkage factor in the CD of 1 to 8%.

and wherein in step (2), the front side fiber web has a shrinkage factor in the MD of 0.5 to 5% and a shrinkage factor in the CD of 1 to 8%.

**28.** The method according to claim 27, wherein the lengths of the main fibers in step (1) are 2.5 mm to 90 mm.

**29.** The method according to claim 27, wherein the single fiber fineness of the thermal bonding fibers in step (1) is 0.5 dtex to 2.2 dtex and the single fiber fineness of the main fibers is 0.01 dtex to 0.5 dtex.

# FIG. 1

# FIG. 2

# FIG. 3

# FIG. 4

# FIG. 5

# FIG. 6

# FIG. 7

# FIG. 8

EP 4 343 054 A1

# FIG. 9

# FIG. 10

## FIG. 11

# FIG. 12

C$_4$H$_7$O (m/z=71)
max 30

C$_7$H$_4$O (m/z=104)
max 27

CNO (m/z=42)
max 6

# FIG. 13

Main fiber group

Adhesion point (resin mass) ○

Polyester-based

Fineness 1.7~2.2 dtex

Large molten masses, insufficient number, non-uniform

⟹ Molten mass sizes

Fineness 0.01~0.5 dtex

Nonwoven fabric properties
• Rough surface quality, poor smoothness
• Insufficient abrasion resistance

Dry heat shrinkage

• Good surface quality, insufficient flexibility
• Improved abrasion resistance

Final product

Shrinkage in MD/CD directions during melting step further increases abrasion resistance by relaxation of entangled fibers, also producing flexibility and stretchability.

EP 4 343 054 A1

FIG. 14

FIG. 15

FIG. 16

FIG. 17

FIG. 18

FIG. 19

Fiber diameters: small            large

FIG. 20

11

13
12
14

FIG. 21

11

13
12

FIG. 22

## INTERNATIONAL SEARCH REPORT

| International application No. |
|---|
| **PCT/JP2022/021013** |

### A. CLASSIFICATION OF SUBJECT MATTER

**D06N 3/00**(2006.01)i
FI: D06N3/00

According to International Patent Classification (IPC) or to both national classification and IPC

### B. FIELDS SEARCHED

Minimum documentation searched (classification system followed by classification symbols)

D04H1/00-18/04, D06N1/00-7/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2021-070904 A (ASAHI KASEI CORP) 06 May 2021 (2021-05-06) | 1-29 |
| A | JP 2021-070881 A (ASAHI KASEI CORP) 06 May 2021 (2021-05-06) | 1-29 |
| A | JP 2014-227637 A (ASAHI KASEI FIBERS CORP) 08 December 2014 (2014-12-08) | 1-29 |
| A | JP 2001-226881 A (KURARAY CO LTD) 21 August 2001 (2001-08-21) | 1-29 |
| A | JP 2006-089863 A (TEIJIN CORDLEY LTD) 06 April 2006 (2006-04-06) | 1-29 |

☐ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **01 July 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/021013**

| Patent document cited in search report | | | Publication date (day/month/year) | Patent family member(s) | | | Publication date (day/month/year) |
|---|---|---|---|---|---|---|---|
| JP | 2021-070904 | A | 06 May 2021 | EP | 3816343 | A1 | |
| JP | 2021-070881 | A | 06 May 2021 | EP | 3816342 | A1 | |
| JP | 2014-227637 | A | 08 December 2014 | (Family: none) | | | |
| JP | 2001-226881 | A | 21 August 2001 | (Family: none) | | | |
| JP | 2006-089863 | A | 06 April 2006 | US | 2008/0102245 | A1 | |
| | | | | EP | 1793031 | A1 | |
| | | | | CN | 101031685 | A | |
| | | | | KR | 10-2007-0057928 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5685003 B
- JP 6118174 B
- JP 470494 A
- JP 5919627 B
- JP HEINO07216756 A
- JP HEINO03016427 B
- JP 4835181 B

**Non-patent literature cited in the description**

- **T. HILDEBRAND ; P. RUEGSEGGER.** A new method for the model-independent assessment of thickness in three-dimensional images. *J. of Microscopy,* 1996, vol. 185, 67-75